(19) Europäisches Patentamt
European Patent Office
Office européen des brevets

(11) Publication number: **0 281 822 B1**

# (12) EUROPEAN PATENT SPECIFICATION

(45) Date of publication of patent specification: **20.07.94**

(51) Int. Cl.⁵: **C12N 15/85**, C12P 21/02

(21) Application number: **88102491.3**

(22) Date of filing: **20.02.88**

The file contains technical information submitted after the application was filed and not included in this specification

## (54) Basic fibroblast growth factor mutein, DNA and its use.

(30) Priority: **24.02.87 JP 42218/87**
**25.02.87 JP 43444/87**
**02.04.87 JP 81977/87**
**12.06.87 JP 147511/87**
**11.08.87 JP 201510/87**
**17.11.87 JP 290283/87**
**26.01.88 JP 16260/88**

(43) Date of publication of application:
**14.09.88 Bulletin 88/37**

(45) Publication of the grant of the patent:
**20.07.94 Bulletin 94/29**

(84) Designated Contracting States:
**AT BE CH DE ES FR GB GR IT LI LU NL SE**

(56) References cited:
**EP-A- 109 748**
**WO-A-86/07595**
**WO-A-86/07595**
**WO-A-87/01728**

**J. Biol. Chem., vol.267, pp. 2269-2271 (1992)**

**Proc. Natl. Acad. Sci. USA, pp. 2324 (1988)**

**J. Biol. Chem., vol.188, pp. 239-245 (1990)**

**Advances in Biotechnology Series, vol.7, p.373 (1990)**

(73) Proprietor: **Takeda Chemical Industries, Ltd.**
**1-1, Doshomachi 4-chome**
**Chuo-ku, OSAKA(JP)**

(72) Inventor: **Senoo, Masaharu**
**1B-212, 744 Nishi-izumigaoka 2-chome**
**Toyonaka Osaka 560(JP)**
Inventor: **Krokawa, Tsutomu**
**1-50, Suimeidai 1-chome**
**Kawanishi Hyogo 666-01(JP)**
Inventor: **Igarashi, Koichi**
**66-3, Shimogamo-miyazakicho**
**Sakyo-ku Kyoto 606(JP)**
Inventor: **Sasada, Reiko**
**C1-405, 2 Takenodai**
**Nagaokakyo Kyoto 560(JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to muteins of basic fibroblast growth factors (hereinafter also briefly referred to as bFGF), a recombinant DNA having a base sequence which codes for a mutein of bFGF and its use.

A basic polypeptide hormone having a molecular weight of about 17,000 secreted mainly by the pituitary gland, bFGF was first separated as a factor exhibiting strong growth promoting action on fibroblasts such as BALB/c3T3 [D. Gospodarowicz; Nature, *249*, 123 (1974)]. Thereafter, however, it was proven that it exhibits growth promoting action on almost all cells deriving from mesoblast [D. Gospodarowicz et al.; National Cancer Institute Monograph, *48*, 109 (1978)]. Specifically, the angiogenic action of bFGF, together with its cell growth promoting action, is suggestive of a potential for the application thereof as a therapeutic drug for traumas and as a preventive and therapeutic drug for thrombosis, arteriosclerosis, etc.

Bovine bFGF was first described in the Proceedings of the National Academy of Sciences of the United States of America, Vol. 82, pp. 6507 (1985). In Science, *233*, 545 (1986), there were disclosed the bovine bFGF-constituent amino acids deduced from the clone which was produced by cloning cDNA of human bFGF.

As for human bFGF, it is reported in Biochemical and Biophysical Research Communications, Vol. 135, p. 541 (1986) that human bFGF was extracted from human brain.

In European Molecular Biology Organization (EMBO) Journal, Vol 5, p. 2523 (1986) and PCT International Publication No. WO/01728, it is shown that the human bFGF-constituent amino acids were deductively specified on the basis of the clone which was produced by cloning cDNA of human bFGF using bovine bFGF as a probe.

In addition, in FEBS Letters, *213,* 189 (1987), is described the production of human bFGF by cultivation of a transformant obtained by cloning cDNA of human bFGF.

Comparing human bFGF and bovine bFGF, the human one has *Thr* for the 112-position, while the bovine one, *Ser*; the human one *Ser* for the 128-position, while the bovine one has *Pro*. (In this case, the N-terminal Pro is determined as the lst amino acid.).

The present inventors presumed that, by modifying the amino acid sequence, bFGF was improved in stability, productivity in a cell and cell proliferating activity per molecule, and, in addition, its unidentified bioactivities were potentiated.

In addition, biologically active proteins microbiologically produced by DNA recombination technique contain cysteine residues, which are not essential to their activities but which may form undesirable intermolecular or intramolecular linkages.

In the course of a production of bFGF by recombinant DNA technique, heterogeneous conformations were found in *Escherichia coli* extract containing a high concentration of bFGF. These conformations are thought to have been produced due to random intra-or inter-molecular disulfide bridging, and are causative of difficulties in bFGF purification and reduction in recovery.

Taking note of this fact, the present inventors intended to so modify microbiologically produced bioactive proteins such as recombinant bFGF that, while their activities are not affected adversely, their capabilities of forming such intermolecular bridges and intramolecular linkages as will result in the formation of undesirable tertiary structures (e.g., conformations which lower the protein activity) are reduced or eliminated.

The present inventors constructed modified bFGF muteins by DNA recombination and site-directed mutagenesis, and made investigations to improve their stability, upgrade their intracellular productivities and activities, and describe changes in their bioactivities; as a result, the present inventors found a mutein which serves these purposes. The present inventors made further investigations based on this finding, and completed the present invention.

The present invention involves:

(1) a mutein of a basic fibroblast growth factor (bFGF), which is a replacement type mutein or a combination of a replacement type and a deletion type mutein, wherein

a replacement type mutein is a mutein in which at least one of the four cystein residues in the constituent amino acid sequence of the natural type bFGF is replaced with one amino acid other than cysteine, and

a deletion type mutein is a mutein in which one to 41 amino acids are lacking from the amino-terminal of the constituent amino acid sequence of bFGF and/or one to 61 amino acids are lacking from the carboxyl-terminal of the constitutent amino acid sequence of bFGF,

(2) a recombinant DNA having a base sequence which codes for the above-mentioned mutein (1),

(3) a transformant harboring a vector containing a recombinant DNA having the base sequence which codes for the above-mentioned mutein (1), and

(4) a method for producing the above-mentioned mutein (1) which comprises cultivating a transformant harboring a vector containing a recombinant DNA having the base sequence which codes for the said mutein in a culture medium to produce and accumulate in the culture broth.

For bFGF, any bFGF can be listed, as long as it derives from a warm-blooded mammal.

As typical examples thereof, mention may be made of human, bovine and murine bFGFs.

Especially, polypeptides which includes the amino acid sequence represented as follows:

**Phe–Phe–Leu–Arg–Ile–His–Pro–Asp–Gly–Arg–Val–Asp–Gly–Val–Arg–Glu–Lys–Ser–Asp–Pro** [I]

are preferable.

More Specifically, polypeptide represented by the formula:

**Pro–Ala–Leu–Pro–Glu–Asp–Gly–Gly–Ser–Gly–Ala–Phe–Pro–Pro–Gly–His–Phe–Lys–Asp–Pro–Lys–Arg–Leu–Tyr–Cys–Lys–Asn–Gly–Gly–Phe–Phe–Leu–Arg–Ile–His–Pro–Asp–Gly–Arg–Val–Asp–Gly–Val–Arg–Glu–Lys–Ser–Asp–Pro–His–Ile–Lys–Leu–Gln–Leu–Gln–Ala–Glu–Glu–Arg–Gly–Val–Val–Ser–Ile–Lys–Gly–Val–Cys–Ala–Asn–Arg–Tyr–Leu–Ala–Met–Lys–Glu–Asp–Gly–Arg–Leu–Leu–Ala–Ser–Lys–Cys–Val–Thr–Asp–Glu–Cys–Phe–Phe–Phe–Glu–Arg–Leu–Glu–Ser–Asn–Asn–Tyr–Asn–Thr–Tyr–Arg–Ser–Arg–Lys–Tyr–X–Ser–Trp–Tyr–Val–Ala–Leu–Lys–Arg–Thr–Gly–Gln–Tyr–Lys–Leu–Gly–Y–Lys–Thr–Gly–Pro–Gly–Gln–Lys–Ala–Ile–Leu–Phe–Leu–Pro–Met–Ser–Ala–Lys–Ser,** [II]

in which X represents *Thr* or *Ser*; when X is *Thr*, Y represents *Ser*, and when X is *Ser*, Y represents *Pro*, is preferable.

More specifically, polypeptide containing the amino acid sequence:

**Pro–Ala–Leu–Pro–Glu–Asp–Gly–Gly–Gly–Ala–Phe–Pro–Pro–Gly–His–Phe–Lys–Asp–Pro–Lys–Arg–Leu–Tyr–Cys–Lys–Asn–Gly–Gly–Phe–Phe–Leu–Arg–Ile–His–Pro–Asp–Gly–Arg–Val–Asp–Gly–Val–Arg–Glu–Lys–Ser–Asp–Pro–His–Val–Lys–Leu–Gln–Leu–Gln–Ala–Glu–Glu–Arg–Gly–Val–Val–Ser–Ile–Lys–Gly–Val–Cys–Ala–Asn–Arg–Tyr–Leu–Ala–Met–Lys–Glu–Asp–Gly–Arg–Leu–Leu–Ala–Ser–Lys–Cys–Val–Thr–Glu–Glu–Cys–Phe–Phe–Phe–Glu–Arg–Leu–Glu–Ser–Asn–Asn–Tyr–Asn–Thr–Tyr–Arg–Ser–Arg–Lys–Tyr–Ser–Ser–Trp–Tyr–Val–Ala–Leu–Lys–Arg–Thr–Gly–Gln–Tyr–Lys–Leu–Gly–Ser–Lys–Thr–Gly–Pro–Gly–Gln–Lys–Ala–Ile–Leu–Phe–Leu–Pro–Met–Ser–Ala–Lys–Ser**

is preferable.

The mutein of the present invention essentially has the amino acid sequence of the original peptide or protein; as such variations, there may be mentioned an addition of amino acid(s), deletion of constituent amino acid(s) and substitution of constituent amino acid(s) by other amino acid(s).

Such addition of amino acid includes addition of at least one amino acid.

Such deletion of constituent amino acid includes deletion of at least one bFGF-constituent amino acid.

Such substitution of constituent amino acid by other amino acids includes substitution of at least one bFGF-constituent amino acid by other amino acid.

The at least one amino acid in the mutein which has at least one amino acid added to bFGF excludes methionine deriving from initiation codon used for peptide expression and signal peptide.

The number of added amino acids is at least 1, but it may be any one, as long as bFGF characteristics are not lost. Preferable amino acids should include some or all of the amino acid sequences of proteins which have homology with bFGF and which exhibit activities similar to those of bFGF.

As examples of such added amino acids, mention may be made of a part or all of the amino acid sequences of proteins such as acidic fibroblast growth factors (aFGF), interleukin 1-α (IL1-α), interleukin 1-β(IL1-β), and a protein which is coded by int-2 in oncogenes.

Such aFGFs include those deriving from humans and those deriving from bovines. Such IL1-αs and IL1-βs include those deriving from humans.

Amino acid sequences of such bovine aFGFs include:

$NH_2$-Phe-Asn-Leu-Pro-Leu-Gly-Asn-Tyr-Lys-Lys-Pro-Lys-Leu-Leu-
Tyr-Cys-Ser-Asn-Gly-Gly-Tyr-Phe-Leu-Arg-Ile-Leu-Pro-Asp-Gly-Thr-
Val-Asp-Gly-Thr-Lys-Asp-Arg-Ser-Asp-Gln-His-Ile-Gln-Leu-Gln-Leu-
Cys-Ala-Glu-Ser-Ile-Gly-Glu-Val-Tyr-Ile-Lys-Ser-Thr-Glu-Thr-Gly-
Gln-Phe-Leu-Ala-Met-Asp-Thr-Asp-Gly-Leu-Leu-Tyr-Gly-Ser-Gln-Thr-
Pro-Asn-Glu-Glu-Cys-Leu-Phe-Leu-Glu-Arg-Leu-Glu-Glu-Asn-His-
Tyr-Asn-Thr-Tyr-Ile-Ser-Lys-Lys-His-Ala-Glu-Lys-His-Trp-Phe-Val-
Gly-Leu-Lys-Lys-Asn-Gly-Arg-Ser-Lys-Leu-Gly-Pro-Arg-Thr-His-Phe-
Gly-Gln-Lys-Ala-Ile-Leu-Phe-Leu-Pro-Leu-Pro-Val-Ser-Ser-Asp-COOH

Amino acid sequences of human IL1-α include:

$NH_2$–Met–Ala–Lys–Val–Pro–Asp–Met–Phe–Glu–Asp–Leu–Lys–Asn–Cys–
Tyr–Ser–Glu–Asn–Glu–Glu–Asp–Ser–Ser–Ser–Ile–Asp–His–Leu–Ser–Leu–
Asn–Gln–Lys–Ser–Phe–Tyr–His–Val–Ser–Tyr–Gly–Pro–Leu–His–Glu–Gly–
Cys–Met–Asp–Gln–Ser–Val–Ser–Leu–Ser–Ile–Ser–Glu–Thr–Ser–Lys–Thr–
Ser–Lys–Leu–Thr–Phe–Lys–Glu–Ser–Met–Val–Val–Val–Ala–Thr–Asn–Gly–
Lys–Val–Leu–Lys–Lys–Arg–Arg–Leu–Ser–Leu–Ser–Gln–Ser–Ile–Thr–Asp–
Asp–Asp–Leu–Glu–Ala–Ile–Ala–Asn–Asp–Ser–Glu–Glu–Glu–Ile–Ile–|Lys|–
|Asn|
Pro–Arg–Ser–Ala–Pro–Phe–Ser–Phe–Leu–Ser–Asn–Val–Lys–Tyr–Asn–Phe–
Met–Arg–Ile–Ile–Lys–Tyr–Glu–Phe–Ile–Leu–Asn–Asp–Ala–Leu–Asn–Gln–
Ser–Ile–Ile–Arg–Ala–Asn–Asp–Gln–Tyr–Leu–Thr–Ala–Ala–Ala–Leu–His–
Asn–Leu–Asp–Glu–Ala–Val–Lys–Phe–Asp–Met–Gly–Ala–Tyr–Lys–Ser–Ser–
Lys–Asp–Asp–Ala–Lys–Ile–Thr–Val–Ile–Leu–Arg–Ile–Ser–Lys–Thr–Gln–
Leu–Tyr–Val–Thr–Ala–Gln–Asp–Glu–Asp–Gln–Pro–Val–Leu–Leu–Lys–Glu–
Met–Pro–Glu–Ile–Pro–Lys–Thr–Ile–Thr–Gly–Ser–Glu–Thr–Asn–Leu–Leu–
Phe–Phe–Trp–Glu–Thr–His–Gly–Thr–Lys–Asn–Tyr–Phe–Thr–Ser–Val–Ala–
His–Pro–Asn–Leu–Phe–Ile–Ala–Thr–Lys–Gln–Asp–Tyr–Trp–Val–Cys–Leu–
Ala–Gly–Gly–Pro–Pro–Ser–Ile–Thr–Asp–Phe–Gln–Ile–Leu–Glu–Asn–Gln–
Ala–COOH

Amino acid sequences of human IL1-β include:

$NH_2$–Met–Ala–Glu–Val–Pro–Glu–Leu–Ala–Ser–Glu–Met–Met–Ala–Tyr–
Tyr–Ser–Gly–Asn–Glu–Asp–Asp–Leu–Phe–Phe–Glu–Ala–Asp–Gly–Pro–Lys–
Gln–Met–Lys–Cys–Ser–Phe–Gln–Asp–Leu–Asp–Leu–Cys–Pro–Leu–Asp–
Gly–Gly–Ile–Gln–Leu–Arg–Ile–Ser–Asp–His–His–Tyr–Ser–Lys–Gly–Phe–
Arg–Gln–Ala–Ala–Ser–Val–Val–Val–Ala–Met–Asp–Lys–Leu–Arg–Lys–Met–
Leu–Val–Pro–Cys–Pro–Gln–Thr–Phe–Gln–Glu–Asn–Asp–Leu–Ser–Thr–Phe–
Phe–Pro–Phe–Ile–Phe–Glu–Glu–Glu–Pro–Ile–Phe–Phe–Asp–Thr–Trp–Asp–
Asn–Glu–Ala–Tyr–Val–His–Asp–Ala–Pro–Val–Arg–Ser–Leu–Asn–Cys–Thr–
Leu–Arg–Asp–Ser–Gln–Gln–Lys–Ser–Leu–Val–Met–Ser–Gly–Pro–Tyr–Glu–
Leu–Lys–Ala–Leu–His–Leu–Gln–Gly–Gln–Asp–Met–Glu–Gln–Gln–Val–Val–
Phe–Ser–Met–Ser–Phe–Val–Gln–Gly–Glu–Glu–Ser–Asn–Asp–Lys–Ile–Pro–
Val–Ala–Leu–Gly–Leu–Lys–Glu–Lys–Asn–Leu–Tyr–Leu–Ser–Cys–Val–Leu–
Lys–Asp–Asp–Lys–Pro–Thr–Leu–Gln–Leu–Glu–Ser–Val–Asp–Pro–Lys–Asn–
Tyr–Pro–Lys–Lys–Lys–Met–Glu–Lys–Arg–Phe–Val–Phe–Asn–Lys–Ile–Glu–
Ile–Asn–Asn–Lys–Leu–Glu–Phe–Glu–Ser–Ala–Gln–Phe–Pro–Asn–Trp–Tyr–

Ile–Ser–Thr–Ser–Gln–Ala–Glu–Asn–Met–Pro–Val–Phe–Leu–Gly–Gly–Thr–Lys–Gly–Gly–Gln–Asp–Ile–Thr–Asp–Phe–Thr–Met–Gln–Phe–Val–Ser–Ser–COOH

Amino acid sequences encoded to int-2 include:

$NH_2$–Met–Gly–Leu–Ile–Trp–Leu–Leu–Leu–Leu–Ser–Leu–Leu–Glu–Pro–Ser–Trp–Pro–Thr–Thr–Gly–Pro–Gly–Thr–Arg–Leu–Arg–Arg–Asp–Ala–Gly–Gly–Arg–Gly–Gly–Val–Tyr–Glu–His–Leu–Gly–Gly–Ala–Pro–Arg–Arg–Arg–Lys–Leu–Tyr–Cys–Ala–Thr–Lys–Tyr–His–Leu–Gln–Leu–His–Pro–Ser–Gly–Arg–Val–Asn–Gly–Ser–Leu–Glu–Asn–Ser–Ala–Tyr–Ser–Ile–Leu–Glu–Ile–Thr–Ala–Val–Glu–Val–Gly–Val–Val–Ala–Ile–Lys–Gly–Leu–Phe–Ser–Gly–Arg–Tyr–Leu–Ala–Met–Asn–Lys–Arg–Gly–Arg–Leu–Tyr–Ala–Ser–Asp–His–Tyr–Asn–Ala–Glu–Cys–Glu–Phe–Val–Glu–Arg–Ile–His–Glu–Leu–Gly–Tyr–Asn–Thr–Tyr–Ala–Ser–Arg–Leu–Tyr–Arg–Thr–Gly–Ser–Ser–Gly–Pro–Gly–Ala–Gln–Arg–Gln–Pro–Gly–Ala–Gln–Arg–Pro–Trp–Tyr–Val–Ser–Val–Asn–Gly–Lys–Gly–Arg–Pro–Arg–Arg–Gly–Phe–Lys–Thr–Arg–Arg–Thr–Gln–Lys–Ser–Ser–Leu–Phe–Leu–Pro–Arg–Val–Leu–Gly–His–Lys–Asp–His–Glu–Met–Val–Arg–Leu–Leu–Gln–Ser–Ser–Gln–Pro–Arg–Ala–Pro–Gly–Glu–Gly–Ser–Gln–Pro–Arg–Gln–Arg–Arg–Gln–Lys–Lys–Gln–Ser–Pro–Gly–Asp–His–Gly–Lys–Met–Glu–Thr–Leu–Ser–Thr–Arg–Ala–Thr–Pro–Ser–Thr–Gln–Leu–His–Thr–Gly–Gly–Leu–Ala–Val–Ala–COOH

As for the number of deleted bFGF-constituent amino acids in the present mutein which lacks at least 1 bFGF-constituent amino acid, it may be any one, as long as any characteristic of bFGF is not lost.

As examples of such deleted constituent amino acids, mention may be made of:
the deletion of amino acids from amino terminal or carboxyl terminal;
the 10 residues in the amino terminal of human bFGF:
Met-Pro-Ala-Leu-Pro-Glu-Asp-Gly-Gly-Ser
the 14 residues in the amino terminal of human bFGF:
Met-Pro-Ala-Leu-Pro-Glu-Asp-Gly-Gly-Ser-Gly-Ala-Phe-Pro
the 41 residues in the amino terminal of human bFGF:

| 1 | 2 | 3 | 4 | | 41 |
|---|---|---|---|---|---|
| Met– | Pro– | Ala– | Leu– | .... | –Val |

or the 61 residues in the carboxyl terminal of human bFGF:

| 87 | 88 | | 146 | 147 |
|---|---|---|---|---|
| Lys– | Cys– | ..... | –Lys– | Ser |

is deleted.

As for the number of at least 1 bFGF-constituent amino acids before substitution in mutein which has at least 1 bFGF-constituent amino acid substituted by other amino acids, it may be any one, as long as any characteristic of bFGF is not lost.

As examples of constituent amino acids before substitution, mention may be made of cysteine and amino acids other than cysteine. Cysteine is preferable. As the constituent amino acid other than cysteine before substitution, there may be mentioned aspartic acid, arginine, glycine, serine, valine and so forth.

When the constituent amino acid before substitution is cysteine, for substituted amino acids are preferable, for example, neutral amino acids. As specific examples of such neutral amino acids, mention may be made of glycine, valine, alanine, leucine, isoleucine, tyrosine, phenylalanine, histidine, tryptophan, serine, threonine and methionine. Specifically, serine and threonine are preferable.

When the constituent amino acid before substitution is any one other than cysteine, for other substituted amino acids are selected amino acids which are different from the amino acid before substitution in a property such as hydrophilicity, hydrophobicity or electric charge.

When the constituent amino acid before substitution is aspartic acid, substituted amino acids include asparagine, threonine, valine, phenylalanine and arginine; asparagine and arginine are preferable.

When the constituent amino acid before substitution is arginine, substituted amino acids include glutamine, threonine, leucine, phenylalanine and aspartic acid; glutamine is preferable.

When the constituent amino acid before substitution is glycine, substituted amino acids include threonine, leucine, phenilalanine, serine, glutamic acid, and arginine; threonine is preferable.

When the constituent amino acid before substitution is serine, substituted amino acids include methionine, alanine, leucine, eysteine, glutamine, arginine and aspartic acid; methionine is preferable.

When the constituent amino acid before substitution is valine, substituted amino acids include serine, leucine, proline, glycine, lysine, and aspartic acid; serine is preferable.

As the constituent amino acid before substitution, aspartic acid, arginine, glycine, serine and valine are preferred.

As the substituted amino acid, asparagine, glutamine, arginine, threonine, methionine, serine, and leucine are preferred.

As the preferred embodiment on the substitution in the mutein, a substitution of serine for cysteine (i.e. cysteine is replaced by serine) is most preferred.

In said substitution, not less than 2 substitutions may be occurred, and two or three substitutions are preferred.

The mutein of the present invention may be a combination of 2 or 3 of the above-mentioned additions, deletions and substitutions.

In addition to conventional DNA recombination technique , site-directed mutagenesis is employed to produce the mutein of the present invention. The said technique is well-known, and it is described in Genetic Engineering, Lather, R. F. and Lecoq, J. P., Academic Press, pp. 31 to 50 (1983). Mutagenesis directed to oligonucleotide is described in Genetic Engineering: Principles and Methods, Smith, M. and Gillam, S., Plenum Press, Vol. 3, pp. 1 to 32 (1981).

The production of the structural gene which encodes the mutein of the present invention is, for example, carried out by:

(a) hybridizing with a mutagenic oligonucleotide primer a single-stranded DNA comprising 1 strand of the structural gene of bFGF,

(b) elongating the primer using DNA polymerase to form a mutational heteroduplex, and

(c) replicating this mutational heteroduplex.

The size of oligonucleotide primer depends upon conditions essential to stable hybridization of the primer to the gene region to which mutation is to be introduced, and upon limitations in currently available methods of oligonucleotide synthesis. The factors to be considered in designing the oligonucleotide intended for the use for mutagenesis directed by the oligonucleotide (e.g., the overall size of the nucleotide and the size of the mismatching portion at the mutation site) are described by Smith, M. and Gillam, S. in the above-mentioned literature. In general, the overall length of the oligonucleotide is adjusted to such length that stable and unique hybridization at the mutation site is optimized, and the extensions between the mutation site and the 5'- and 3'-terminals are provided with sufficient sizes to prevent mutation editing due to the exonuclease activity of DNA polymerase.

The oligonucleotides used for mutagenesis in accordance with the present invention normally contain some 12 to 24 bases, preferably 14 to 20 bases, and more preferably 14 to 18 bases. These normally contain at least about 3 base 3'-terminal of the codons to be changed.

For the purpose of obtaining, for example, a mutein having an added amino acid, a mutagenic bFGF gene is produced by synthesizing the gene which encodes the amino acid sequence to be added, and, directly or after fragmentation by digestion with restriction enzyme, inserting or adding it into an appropriate site in the bFGF gene using DNA ligase. When any suitable restriction enzyme recognition site is not present in the bFGF gene, restriction enzyme recognition sites may be produced by the above-mentioned site-directed mutagenesis.

For the purpose of obtaining, for example, a mutein lacking bFGF-constituent amino acids, a mutagenic bFGF gene is produced in 3 different ways. In one of the 3 cases, the amino terminal of bFGF is deleted; in another case, a central region of bFGF is deleted; and in the remaining one case, the carboxyl terminal is deleted.

In the case of deletion of the amino terminal, a codon of the gene which encodes the carboxyl terminal of the amino acid sequence to be deleted is changed to the *Met*-encoding codon ATG by site-directed mutagenesis, and the codon has an appropriate restriction enzyme recognition site produced in the 5'-terminal side thereof to facilitate its ligation to the promoter.

In the case of deletion a central region of the amino acid sequence, a unique restriction enzyme recognition site is produced, by site-directed mutagenesis, in each of the 5'-terminal and 3'-terminal sides of the gene which encodes the sequence to be deleted, and the relevant gene is cleaved off from the gene by enzymatic digestion, and this is followed by re-ligation of the remaining 2 gene fragments to construct the desired gene which encodes bFGF lacking the specified amino acids. It is of course necessary not to shift the reading frame due to the digestion with the restriction enzymes.

In the case of deletion of an amino acid sequence in the carboxyl terminal side, a codon of the gene which encodes amino-terminal amino acids of the sequence to be deleted is changed to a stop codon by site-directed mutagenesis.

For the purpose of obtaining a mutein which has had the constituent amino acid cysteine substituted, a mutagenic bFGF gene is produced by eliminating, for example, the *Cys*-expression codon or inducing site-directed mutagenesis in the *Cys*-expression codon TGC or TGT using a synthetic nucleotide primer which so changes the codon that it encodes a different amino acid. A primer is hybridized with a sense chain of the FGF gene, for example, to change cysteine (26-position) of human bFGF to serine. As an appropriate nucleotide primer, there may be mentioned, for example, 5'-CGTTCTT<u>GCT</u>GTAGAGCCGCT-3', in which the underlined triplet represents the changed codon.

As an appropriate primer in changing cysteine (70-position) to serine, there may be mentioned 5'-AACGATTAGC<u>GCT</u>CACTC-3', in which the underlined triplet represents the changed codon.

As an appropriate primer for changing cysteine (88-position) to serine, there may be mentioned 5'-GTAAC<u>AGA</u>CTTAGAAGCTAGT-3', in which the underlined triplet represents the changed codon.

As an appropriate primer for changing cysteine (93-position) to serine, there may be mentioned 5'-TCGAAGAAGAA<u>AGA</u>CTCATCC-3', in which the underlined triplet represents the changed codon.

In the case of *Cys* (26-position), cysteine changes to serine via T→A transpostition of the 1st base; in the case of *Cys* (70-position), cysteine changes to serine via T→A transposition of the 1st base and T→C transposition of the 2nd base; and in the case of *Cys* (88-position, 93-position), cysteine changes to serine via G→C transposition of the 2nd base.

It should be noted that, in producing bFGF mutein protein by site-directed mutagenesis, 2 or more variations may be induced in the DNA sequence, that is, the DNA codons corresponding to the amino acids have degenerated.

For the purpose of obtaining a mutein which has had a constituent amino acid other than cysteine substituted by another amino acid, a mutagenic bFGF gene is produced by changing a codon using an oligonucleotide primer in the same manner as with cysteine.

The design of oligonucleotide primer of course varies with which amino acid is to be changed.

The primer is hybridized to a single-stranded phage resulting from cloning of a single strand of the bFGF gene, such as M13 [Yanish-Perror, C. Vieira, and J. Messing, Gene, *33*, 103-119 (1985); Messing, J., Methods in Enzymology, *101*, 20-78 (1983)], fd [R. Herrman et al., Molecular and General Genetics, *177*, 231 (1980)] or Φ × 174 [M. Smith and S. Gillam, Genetic Engineering, Plenum Press, Vol. 3, pp. 1-32-(1981)]. It is noted that the phage is capable of carrying either the sense chain or antisense chain of the gene. When the phage carries an antisense chain, in addition to discrepancies from the relevant codon determining a triplet which has encoded another amino acid, the primer may have discrepancies due to codon degeneracy from the sense chain region containing the codon to which mutation is to be induced. Similarly, when the phage carries a sense chain, the primer may not be complementary to the sense chain region containing the codon to which mutation is to be induced, as well as appropriate discrepancies from the triplet which pairs to the codon to be deleted. The conditions used for hybridization are described by M. Smith and S. Gillam in the above-mentioned literature. Temperature is normally between about 0°C and 70°C, more generally, between about 10°C and 50°C. After hybridization, the primer is elongated on the phage DNA by reaction with *Escherichia coli* DNA polymerasa I, T4 DNA polymerase, reverse transcriptase or other appropriate DNA polymerase. The resulting dsDNA is converted to a closed circular dsDNA by treatment with DNA ligase such as T4 DNA ligase. The DNA molecules containing single-strand regions can be decomposed by S1 endonuclease treatment.

The resulting mutational heteroduplex is used to transform an infectable host organism or cell. In the replication of the heteroduplex by the host, progenies are produced from both chains. Following the replication, the mutant gene is isolated from a progeny of the mutant's chain, and is inserted in an appropriate vector, which is then used to transform an appropriate host organism or cell.

The phage DNA carrying the mutational gene is then isolated, and incorporated in a plasmid.

As examples of plasmids in which DNA is incorporated, mention may be made of plasmids deriving from *Escherichia coli* such as pBR322 [Gene, *2*, 95 (1977)], pBR325 [Gene, *4*, 121 (1978)], pUC12 [Gene, *19,* 259 (1982)] and pUC13 [Gene, *19,* 259 (1982)]; and plasmids deriving from *Bacillus subtilis* such as pUB110 [Biochemical and Biophysical Research Communication, *112,* 678 (1983)]; but any other plasmid can also be used, as long as it is replicated and held in the host.

As methods of incorporation in plasmid, there may be mentioned, for example, the method described in Molecular Cloning, T. Maniatis et al., Cold Spring Harbor Laboratory, p. 239 (1982), etc.

The cloned gene is ligated to the downstream of a promoter in a suitable vehicle (vector) for expression to obtain an expression vector.

Vectors include the above-mentioned plasmids deriving from *Escherichia coli* (e.g., pBR322, pBR325, pUC12 and pUC13), plasmids deriving from *Bacillus subtilis* (e.g., pUB110, pTP5 and pC194), plasmids deriving from yeast (e.g., pSH19 and pSH15), bacteriophages such as λ phage, and animal viruses such as retrovirus and vaccinia virus.

The said gene may have ATG as a translation initiation codon at its 5'-terminal, and may also have TAA, TGA or TAG as a translation termination codon at its 3'-terminal. For the purpose of expression of the said gene, a promoter is ligated to the upstream thereof. Any promoter can be used for the present invention, as long as it appropriately corresponds to the host used for the gene expression.

When the host for transformation is a bacterium of the genus *Escherichia*, *trp* promoter, *lac* promoter, *rec* A promoter, $\lambda P_L$ promoter, *lpp* promoter, etc. are appropriate; when the host is a *bacterium* of the genus Bacillus, SP01 promoter, SP02 promoter, *penP* promoter etc. are appropriate; when the host is a yeast, PH05 promoter, PGK promoter, GAP promoter, ADH promoter, etc. are appropriate. Specifically, it is preferable that the host be a bacterium of the genus *Escherichia*, and the promoter be *trp* promoter or $\lambda P_L$ promoter.

When the host is an animal cell, appropriate promoters include promoters deriving from SV40 and retrovirus promoters; specifically, promoters deriving from SV40 are preferable.

Using the vector thus constructed, which contains a recombinant DNA having a mutein-encoding base sequence, a transformant is produced.

As hosts, there may be mentioned, for example, bacteria of the genus *Escherichia*, bacteria of the genus *Bacillus*, yeasts, animal cells, etc.

As examples of said bacteria of the genus *Escherichia*, mention may be made of *Escherichia coli* K12DH1 [Proc. Natl. Acad. Sci. USA, *60,* 160 (1968)], JM103 [Nucleic acids Research, *9*, 309 (1981)], JA221 [Journal of Molecular Biology, *120,* 517 (1978)], HB101 [Journal of Molecular Biology, *41*, 459 (1969)], C600 [Genetics, *39*, 440 (1954)] and MM294 [Proc. Natl. Acad. Sci. USA, *73*, 4174 (1976)].

As examples of said bacteria of the genus *Bacillus*, mention may be made of *Bacillus subtilis* MI114 [Gene, *24,* 255 (1983)] and 207-21 [Journal of Biochemistry, *95*, 87 (1984)].

As examples of said yeasts, mention may be made of *Saccharomyces cerevisiae* AH22R$^-$, NA 87-11A and DKD-5D.

As examples of animal cells, mention may be made of simian cell COS-7, Vero, Chinese hamster ovary cell CHO, mouse L-cell and human FL-cell.

The transformation of the above-mentioned bacteria of the genus *Escherichia* is carried out in accordance with, for example, the methods described in Proc. Natl. Acad. Sci. USA, *69,* 2110 (1972), Gene, *17,* 107 (1982), etc.

The transformation of bacteria of the genus *Bacillus* is carried out in accordance with, for example, the methods described in Molecular and General Genetics, *168*, 111 (1979) etc.

The transformation of yeasts is carried out in accordance with, for example, the method described in Proc. Natl. Acad. Sci. USA, *75*, 1929 (1978).

The transformation of animal cells is carried out in accordance with, for example, the method described in Virology, *52,* 456 (1973).

A transformant harboring a vector containing a recombinant DNA having a mutein-encoding base sequence is thus obtained.

The said transformant is cultured in a medium to allow it to produce mutein.

In culturing a transformant whose host is a bacterium of the genus *Escherichia* or *Bacillus*, liquid medium is appropriate for the cultivation, in which carbon sources, nitrogen sources, minerals, and other substances essential to the growth of the said transformant are contained. As carbon sources, there may be mentioned, for example, glucose, dextrin, soluble starch, sucrose, etc.; as nitrogen sources, there may be mentioned, for example, inorganic or organic substances such as ammonium salts, nitrates, corn steep liquor, peptone, casein, meat extract, soybean cake and potato extract; as minerals, there may be mentioned, for example, calcium chloride, sodium dihydrogenphosphate and magnesium chloride. Yeasts extracts, vitamins, growth accelerators, etc. may also be added.

It is desirable that the pH of medium be about 6 to 8.

As an example of appropriate medium for the cultivation of bacteria of the genus *Escherichia*, mention may be made of M9 medium containing glucose and casamino acid [Miller, Journal of Experiments in Molecular Genetics, 431-433, Cold Spring Harbor Laboratory, New York (1972)]. Chemicals such as 3$\beta$-indolylacrylic acid may be added thereto, when it is necessary to increase promoter efficiency.

When the host is a bacterium of the genus *Escherichia*, cultivation is normally carried out at about 15 to 43 °C for about 3 to 24 hours, and, where necessary, aeration and/or agitation are added.

When the host is a bacterium of the genus *Bacillus*, cultivation is normally carried out at about 30 to 40 °C for about 6 to 24 hours, and, where necessary, aeration and/or agitation are added.

As a medium for the cultivation of transformants whose host is a yeast, there may be mentioned, for example, Burkholder's minimum medium [Bostian, K. L. et al., Proc. Natl. Acad. Sci. USA, *77*, 4505 (1980)]. It is preferable that the pH of the medium be adjusted to about 5 to 8. Cultivation is normally carried out at about 20 to 35 °C for about 24 to 72 hours, and, where necessary, aeration and/or agitation are added.

As media for the cultivation of transformants whose host is an animal cell, there may be mentioned, for example, MEM media containing about 5 to 20% fetal bovine serum [Science, ***122,*** 501 (1952)], DMEM medium [Virology, ***8,*** 396 (1959)], BPMI1640 medium [Journal of the American Medical Association, ***199,*** 519 (1967)] and 199 medium [Proceeding of the Society for the Biological Medicine, *73,* 1 (1950)]. It is preferable that pH be about 6 to 8. Cultivation is normally carried out at about 30 to 40 °C for about 15 to 60 hours, and, where necessary, aeration and/or agitation are added.

The separation and purification of mutein from the above-mentioned culture can be carried out by, for example, the following methods.

In extracting mutein from cultured bacterial, fungal or animal cells, there can be employed as appropriate, for example, the method in which bacterial, fungal or animal cells, after cultivation, are collected by a known method, and suspended in a buffer solution containing a protein denaturant such as guanidine hydrochloride to elute the desired protein out of the cells, and the method in which bacterial, fungal or animal cells, after disintegration by French press, ultrasonication, lysozyme treatment and/or freezing-thawing, are subjected to centrifugation to obtain mutein. Specifically, the method using in combination lysozyme treatment and ultrasonication is preferable.

The purification of mutein from the supernatant thus obtained can be carried out by well-known methods of separation and purification in appropriate combination. As examples of such well-known methods of separation and purification, mention may be made of methods based on solubility such as salting-out and solvent precipitation; methods based mainly on the difference in molecular weight such as dialysis, ultrafiltration, gel filtration and SDS-polyacrylamide gel electrophoresis; methods based on the difference in electric charge such as ion-exchange chromatography; methods based on specific affinity such as affinity chromatography; methods based on the difference in hydrophobicity such as reverse phase high performance liquid chromatography; and methods based on the difference in isoelectric point such as isoelectric electrophoresis.

More specifically, by subjecting the above-mentioned supernatant to ion-exchange chromatography using DEAE cellulose etc. as carriers, impurities such as nucleic acids and acid protein can be removed. For example, it is efficient to pass the supernatant through a DEAE cellulose column equilibrated with a nearly neutral buffer solution such as Tris, and collect the fraction not adsorbed to the column. By further subjecting the collected fraction to ion-exchange chromatography using as a carrier CM cellulose or the like, it is possible to adsorb mutein to the carrier, and elute the mutein with a salt solution. These eluates may be lyophilized after dialysis.

Affinity chromatography using heparin-Sepharose can be suitably applied to purifying bFGF mutein in extracts. Thus, for instance, the bFGF mutein protein can be purified by applying the above eluate to a heparin-Sepharose column equilibrated with an almost neutral buffer (e.g. Tris or phosphate buffer), washing the column thoroughly and performing elution by linear gradient constructed with NaCl or the like.

Heparin columns (e.g. Shodex AF-pak HR-894, available from Showa Denko, Japan) developed for high performance liquid chromatography are particularly efficient.

In this case, bFGF mutein can be recovered as homogeneous product in the same manner as in the case of the heparin-Sepharose column mentioned above, namely by applying the sample to a heparin column with an about neutral buffer, washing the column thoroughly and conducting elution on a linear gradient constructed with NaCl, for instance.

The thus-obtained product can be made up into a dry powder form by dialysis and lyophilization. To preserve the product with an added carrier (e.g. serum albumin) is desirable since the adsorption of the product on the vessel wall can be prevented thereby.

Furthermore, it is preferable to add a slight amount of a reducing agent in the course of purification or preservation, in order to prevent an oxidation of the product.

As examples of the reducing agent, there are mentioned β-mercaptoethanol, dithiothreitol, glutathione, and so forth.

In this way, substantially pure bFGF mutein protein can be obtained. The substantially pure bFGF mutein protein according to this invention includes products whose bFGF mutein protein content is not less than 95% (w/w) and, more preferably, products whose bFGF mutein content is not less than 98% (w/w).

The mutein thus obtained possesses fibroblast growth promoting activity, growth stimulating activity of capillary endothelial cells and angiogenic acitivity, has high stability and is low in toxicity; therefore, it can be used as a healing accelerator for burns, wounds, postoperative tissues, etc., or as a therapeutic drug based on its angiogenic action for thrombosis, arteriosclerosis, etc. It can also be used as a reagent for acceleration of cell cultivation.

Especially, a mutein wherein at least one constituent cysteine is replaced by serine is preferred, because the mutein is remarkably stable.

The mutein according to the present invention, when used as a pharmaceutical, can be safely administered parenterally or orally to warm-blooded animals (e.g., humans, mice, rats, hamsters, rabbits, dogs and cats) directly in a powder form or after preparing as a pharmaceutical composition (e.g., injection, tablets, capsules, solutions and ointments) with other pharmacologically allowable carriers, excipients or diluents.

The preparation of injection is carried out in accordance with a routine method using, for example, a physiological saline solution or an aqueous solution containing glucose and other auxiliary agents. Pharmaceutical compositions such as tablets and capsules can also be prepared in accordance with routine methods.

The mutein according to the present invention, when used as any of the above-mentioned pharmaceuticals, is, for example, administered to the above-mentioned warm-blooded animals in an appropriate amount selected in the range of from about 1 ng/kg to 100 μg/kg daily, taking note of the route of administration, symptoms, etc.

The mutein according to the present invention, when used as a reagent for accelerating cell cultivation, is preferably added to the medium so that it is contained therein in an amount of about 0.01 to 10 μg per 1 liter of medium, more preferably about 0.1 to 10 μg per 1 liter of medium.

The abbreviations for bases, amino acids, etc., used in the present specification and the drawings, are based on the abbreviations specified by the IUPAC-IUB Commission on Biochemical Nomenclature or those used commonly in relevant fields, and instances thereof are listed below. When there is a possibility of the presence of an optical isomer in amino acids, the isomer is an L-body unless otherwise specified.

| | |
|---|---|
| DNA | : Deoxyribonucleic acid |
| cDNA | : Complementary deoxyribonucleic acid |
| A | : Adenine |
| T | : Thymine |
| G | : Guanine |
| C | : Cytosine |
| RNA | : Ribonucleic acid |
| dATP | : Deoxyadenosine triphosphate |
| dTTP | : Deoxythymidine triphosphate |
| dGTP | : Deoxyguanosine triphosphate |
| dCTP | : Deoxycytidine triphosphate |
| ATP | : Adenosine triphosphate |
| Tdr | : Thymidine |
| EDTA | : Ethylenediaminetetraacetic acid |

| | |
|---|---|
| SDS | : Sodium dodecyl sulfate |
| Gly | : Glycine |
| Ala | : Alanine |
| Val | : Valine |
| Leu | : Leucine |
| Ile | : Isoleucine |
| Ser | : Serine |
| Thr | : Threonine |
| Cys | : Cysteine |
| Met | : Methionine |
| Glu | : Glutamic acid |
| Asp | : Aspartic acid |
| Lys | : Lysine |
| Arg | : Arginine |
| His | : Histidine |
| Phe | : Phenylalanine |
| Tyr | : Tyrosine |
| Trp | : Tryptophan |
| Pro | : Proline |
| Asn | : Asparagine |
| Gln | : Glutamine |

In the present specification and the drawings, in numbering the human bFGF-constituent amino acids, the *Met* deriving from the translation initiation codon is determined as the 1st amino acid, unless otherwise noted.

The following transformants which were produced in the Reference Examples or Examples mentioned below were deposited at the Institute for Fermentation, Osaka (IFO), Japan and at the Fermentation Research Institute, Agency of Industrial Science and Technology, Ministry of International Trade and Industry (FRI), Japan under the accession numbers on the deposit dates shown in Table 1 (The deposit dates are indicated in parentheses.). As to the deposit number of FRI, FERM BP number denotes the number of deposit under the Budapest Treaty; and in case both FERM P number and FERM BP number are described, it shows that the deposit under the number of FERM P has been converted to the deposit under Budapest Treaty and the transformants have been stored at FRI under the number of FERM BP.

Table 1

| Transformants | IFO | FRI | |
|---|---|---|---|
| E. coli K12 DHI/pTB 627 [Reference Example 1] | IFO 14494 (March 13, 1986) | FERM P-8726 (April 2, 1986) | FERM BP-1280 |
| E. coli K12 MM294/pTB 669 [Reference Example 2] | IFO 14532 (August 11, 1986) | FERM P-8918 (August 21, 1986) | FERM BP-1281 |
| E. coli DH1/pTB 739 [Example 2] | IFO 14575 (February 18, 1987) | FERM P-9216 (February 25, 1987) | FERM BP-1641 |
| E. coli DH1/pTB 742 [Example 3] | IFO 14584 (March 24, 1987) | FERM P-9307 (March 30, 1987) | FERM BP-1642 |
| E. coli DH1/pTB 743 [Example 4] | IFO 14585 (March 24, 1987) | FERM P-9308 (March 30, 1987) | FERM BP-1643 |
| E. coli DH1/pTB 744 [Example 5] | IFO 14586 (March 24, 1987) | FERM P-9309 (March 30, 1987) | FERM BP-1644 |

| Transformants | IFO | FRI | |
|---|---|---|---|
| E. coli MM294/pTB 762 [Example 7] | IFO 14613 (May 27, 1987) | FERM P-9409 (June 11, 1987) | FERM BP-1645 |
| E. coli MM294/pTB 764 [Example 9] | IFO 14614 (May 27, 1987) | FERM P-9410 (June 11, 1987) | FERM BP-1646 |
| E. coli MM294/pTB 765 [Example 11] | IFO 14615 (May 27, 1987) | FERM P-9411 (June 11, 1987) | FERM BP-1647 |
| E. coli MM294/pTB 795 [Example 34] | IFO 14700 (January 14, 1988) | | FERM BP-1660 (January 20, 1988) |
| E. coli MM294/pTB 796 [Example 35] | IFO 14701 (January 14, 1988) | | FERM BP-1661 (January 20, 1988) |
| E. coli MM294/pTB 797 [Example 36] | IFO 14702 (January 14, 1988) | | FERM BP-1662 (January 20, 1988) |

The following hybridomas which were produced in the Example 35(3)(d) mentioned below were deposited at IFO since August 17, 1987 under the accession numbers indicated below.

Mouse HbF 52 cell: IFO 50143

Mouse HbF 78 cell: IFO 50144

Brief Explanation of the Drawings

Figure 1 shows the base sequence which encodes human bFGF and the amino acid sequence deduced therefrom.

Figure 2 shows the synthetic oligomers used as primers to mutate *Cys*-encoding codons to *Ser*-encoding codons.

Figure 3 shows the base sequence which encodes the human bFGF mutein CS1, carried by the plasmid pTB739 obtained in Example 2, and the amino acid sequence of the human bFGF mutein CS1, encoded thereby. The mutated bases are underlined, and the region containing the converted amino acid is surrounded by the square.

Figure 4 shows the base sequence which encodes the human bFGF mutein CS2, carried by the plasmid pTB742 obtained in Example 3, and the amino acid sequence of the human bFGF mutein CS2, encoded thereby. The mutated bases are underlined, and the region containing the converted amino acid is surrounded by the square.

Figure 5 shows the base sequence which encodes the human bFGF mutein CS3, carried by the plasmid pTB743 obtained in Example 4, and the amino acid sequence of the human bFGF mutein CS3, encoded thereby. The mutated bases are underlined, and the region containing the converted amino acid is surrounded by the square.

Figure 6 shows the base sequence which encodes the human bFGF mutein CS4, carried by the plasmid pTB744 obtained in Example 5, and the amino acid sequence of the human bFGF mutein CS4, encoded thereby. The mutated bases are underlined, and the region containing the converted amino acid is surrounded by the square.

Figure 7 shows the construction scheme of the plasmid pTB739 in Example 2 (1).

Figure 8 shows the construction scheme of the plasmid pTB742 in Example 3 (1).

Figure 9 shows the construction scheme of the plasmid pTB743 in Example 4 (1).

Figure 10 shows the construction scheme of the plasmid pTB744 in Example 5 (1).

Figure 11 shows the base sequence which encodes the human bFGF mutein CS23, carried by the plasmid pTB762 obtained in Example 7, and the amino acid sequence of the human bFGF mutein CS23, encoded thereby. The mutated bases are underlined, and the regions containing either of the converted amino acids are surrounded by the squares.

Figure 12 shows the construction chart of the plasmid pTB762 in Example 7 (1).

Figure 13 shows the base sequence which encodes the human bFGF mutein CS123, carried by the plasmid pTB764 obtained in Example 9, and the amino acid sequence of the human bFGF mutein CS123 encoded thereby. The mutated bases are underlined, and the regions containing any of the converted amino acids are surrounded by the squares.

Figure 14 shows the construction scheme of the plasmid pTB764 in Example 9 (1).

Figure 15 shows the base sequence which encodes the human bFGF mutein CS1234, carried by the plasmid pTB765 obtained in Example 11 and the amino acid sequence of the human bFGF mutein CS1234, coded thereby. The mutated bases are underlined, and the regions containing any of the converted amino acids are surrounded by the squares.

Figure 16 shows the construction scheme of the plasmid pTB765 in Example 11 (1).

Figure 17 shows the base sequence which encodes the human bFGF mutein CS12, carried by the plasmid pTB776 obtained in Example 15, and the amino acid sequence of the human bFGF mutein CS12, encoded thereby. The mutated bases are underlined, and the regions containing either of the converted amino acids are surrounded by the squares.

Figure 18 shows the construction scheme of the plasmid pTB776 in Example 15 (1).

Figure 19 shows the base sequence which encodes the human bFGF mutein CS24, carried by the plasmid pTB778 obtained in Example 16, and the amino acid sequence of the human bFGF mutein CS24, encoded thereby. The mutated bases are underlined, and the regions containing either of the converted amino acids are surrounded by the squares.

Figure 20 shows the construction scheme of the plasmid pTB778 in Example 16 (1).

Figure 21 shows the base sequence which encodes the human bFGF mutein CS13, carried by the plasmid pTB779 obtained in Example 17, and the amino acid sequence of the human bFGF mutein CS13, encoded thereby. The mutated bases are underlined, and the regions containing either of the converted amino acids are surrounded by the squares.

Figure 22 shows the construction scheme of the plasmid pTB779 in Example 17 (1).

Figure 23 shows the base sequence which encodes the human bFGF mutein CS14, carried by the plasmid pTB763 obtained in Example 18, and the amino acid sequence of the human bFGF mutein CS14, encoded thereby. The mutated bases are underlined, and the regions containing either of the converted amino acids are surrounded by the squares.

Figure 24 shows the construction scheme of the plasmid pTB763 in Example 18 (1).

Figure 25 shows the base sequence which encodes the human bFGF mutein CS34, carried by the plasmid pTB777 obtained in Example 19, and the amino acid sequence of the human bFGF mutein CS34, encoded thereby. The mutated bases are underlined, and the regions containing either of the converted amino acids are surrounded by the squares.

Figure 26 shows the construction scheme of the plasmid pTB777 in Example 19 (1).

Figure 27 shows the base sequence which encodes the human bFGF mutein CS234, carried by the plasmid pTB782 obtained in Example 20, and the amino acid sequence of the human bFGF mutein CS234, encoded thereby. The mutated bases are underlined, and the regions containing any of the converted amino acids are surrounded by the squares.

Figure 28 shows the construction scheme of the plasmid pTB782 in Example 16 (1).

Figure 29 shows the base sequence which encodes the human bFGF mutein CS124, carried by the plasmid pTB780 obtained in Example 21, and the amino acid sequence of the human bFGF mutein CS124, encoded thereby. The mutated bases are underlined, and the regions containing any of the converted amino acids are surrounded by the squares.

Figure 30 shows the construction scheme of the plasmid pTB780 in Example 21 (1).

Figure 31 shows the base sequence which encodes the human bFGF mutein CS134, carried by the plasmid pTB781 obtained in Example 22, and the amino acid sequence of the human bFGF mutein CS134, encoded thereby. The mutated bases are underlined, and the regions containing any of the converted amino acids are surrounded by the squares.

Figure 32 shows the construction scheme of the plasmid pTB781 in Example 22 (1).

Figures 33 to 35 show the high performance liquid chromatography elution patterns of the muteins obtained in Example 24.

Figure 36 shows the high performance liquid chromatography elution patterns of the mutein oxides obtained in Example 25.

Figure 37 shows the high performance liquid chromatography elution patterns of the mutein reduction products obtained in Example 26.

Figure 38 shows the synthetic oligomers used as primers to prepare mutein-encoding DNAs.

Figure 39 shows the base sequence of the phage M13-PN10 obtained in Example 27 and the amino acid sequence of mutein TM910 encoded thereby, the mutein TM910 being obtained in Example 32.

Figure 40 shows the base sequence of the phage M13-PN14 obtained in Example 27 and the amino acid sequence of mutein N14 encoded thereby, the mutein N14 being obtained in Example 34.

Figure 41 shows the base sequence of the phage M13-PC86 obtained in Example 27 and the amino acid sequence of mutein C86 encoded thereby, the mutein C86 being obtained in Example 35.

Figure 42 shows the base sequence of the phage M13-PDN42 obtained in Example 27 and the amino acid sequence of mutein DN42 encoded thereby, the mutein DN42 being obtained in Example 36.

Figure 43 shows the base sequence of the phage M13-PRQ45 obtained in Example 27 and the amino acid sequence of mutein RQ45 encoded thereby, the mutein RQ45 being obtained in Example 37.

Figure 44 shows the base sequence of the phage M13-PNR42 obtained in Example 28 and the amino acid sequence of mutein NR42 encoded thereby, the mutein NR42 being obtained in Example 38.

Figure 45 shows the stability of the human bFGF muteins CS2, CS3 and CS23, obtained in Example 29.

Figure 46 shows the construction scheme of the plasmid pTB854 in Example 32.

Figure 47 shows the construction scheme of the plasmid pTB852 in Example 33.

Figure 48 shows the base sequence which encodes the human bFGF mutein N10, carried by the plasmid pTB852 obtained in Example 33, and the amino acid sequence of the mutein N10 encoded thereby, the mutein N10 being ontained in Example 33. The mutated bases are underlined.

Figure 49 shows the construction scheme of the plasmid pTB795 in Example 34.

Figure 50 shows the construction scheme of the plasmid pTB796 in Example 35.

Figure 51 shows the construction scheme of the plasmid pTB797 in Example 36.

Figure 52 shows the construction scheme of the plasmid pTB799 in Example 37.

Figure 53 shows the construction scheme of the plasmid pTB798 in Example 38.

Figure 54 shows the base sequence which encodes the human bFGF mutein FINT, carried by the plasmid pTB853 obtained in Example 39, and the amino acid sequence of the mutein FINT encoded thereby, the mutein FINT being obtained in Example 39. The mutated bases are underlined.

Figure 55 shows the construction scheme of the plasmid pTB853 in Example 39.

Figure 56 shows the construction scheme of the plasmid pTB855 in Example 40.

Figure 57 shows the base sequence which encodes the human bFGF mutein N41 carried by the plasmid pTB855 obtained in Example 40, and the amino acid sequence of the mutein N41 encoded thereby, the mutein N41 being obtained in Example 40. The mutated bases are underlined.

Figure 58 shows the construction scheme of the plasmid pTB856 in Example 41.

Figure 59 shows the base sequence which encodes the human bFGF mutein C129 carried by the plasmid pTB856 obtained in Example 41, and the amino acid sequence of the mutein C129 encoded thereby, the mutein C129 being obtained in Example 41. The mutated bases are underlined.

Figure 60 shows the construction scheme of the plasmid pTB831 in Example 42.

## Reference Example 1

(Construction of plasmid containing gene encoding human bFGF)

(1) Isolation of cDNA-containing plasmid:

A cDNA whose host is *Escherichia coli* x1776 and which was prepared by incorporating a primary culture cell mRNA deriving from human prepuce in pCD vector [refer to Okayama et al., Molecular Cell Biology, *3,* 280 (1983)] was supplied by Dr. Okayama at the National Institute of Child Health and Human Development, Bethesda, USA. From this DNA was extracted a plasmid DNA by the alkali method [Birnboim, H. C. and Doly, J., Nucleic Acids Research, *1*, 1513 (1979)], and this DNA was infected with *Escherichia coli* DH1 to produce about 2 × $10^6$ clones of a cDNA library whose host is *Escherichia coli* DH1.

Said cDNA library using *Escherichia coli* DH1 was spread over 10 nitrocellulose filters (Millipore Inc., U.S.A. HATF filters) in an amount of about 5 × $10^4$ clone/filter, whereafter a total of 20 replica filters in pairs were prepared using the above 10 filters as the master filters. *Escherichia coli* cells on the replica filters were lysed with a 0.5N NaOH solution, and the exposed denatured plasmid DNA was immobilized to the filters [Grunstein, M. and Hogness, D. S., Proc. Natl. Acad. Sci. USA, *72,* 3961 (1975)].

Separately, based on the amino acid Nos. 13 to 20 (Pro-Pro-Gly-His-Phe-Lys-Asp-Pro) and amino acid Nos. 89 to 96 (Thr-Asp-Glu-Cys-Phe-Phe-Phe-Glu) on the amino acid sequence of bovine basic fibroblast growth factor as reported by F. Esch et al. [Proc. Natl. Acad. Sci. USA, 82, 6507 (1985)], the base sequences corresponding to these amino acid sequences were chemically synthesized. (For some codons, the 3rd letter was fixed arbitrarily. 5'GG A/G TC T/C TT A/G AA A/G TGGCCAGGAGG and 5'TC A/G AA A/G AA A/G AA A/G CA T/C TCGTCGGT, respectively. The underlined letters represent the fixed bases). For each of these oligonucleotides, reaction was carried out at 37 °C for 1 hour in 50 μℓ of a reaction liquid using T4 polynucleotide kinase (produced by Takara Shuzo Co., Ltd., Japan) [oligonucleotide 0.1 μg, 50mM Tris-HCl pH 8.0, 0.10mM $MgCl_2$, 10mM mercaptoethanol, 50 μCi $\gamma$-$^{32}$P ATP (>5000Ci/mmole), 3 units of T4 polynucleotide kinase] to label the 5'-terminal of the oligonucleotides with $^{32}$P.

Using as probes the 2 oligonucleotides labeled in the above method, these were independently associated to a replica filter to which the DNA had been immobilized. Association reaction was carried out at 35 °C for 16 hours in a solution of 5 × SSPE [180mM NaCl, 10mM $NaH_2PO_4$, 1mM EDTA (pH 7.4)], 5 × Denhardt's solution, 0.1% SDS and 100 μg/mℓ denatured salmon sperm DNA, containing 10 μCi of the probe. After the reaction, the filter was washed with a 0.1% SDS solution of 5 × SSC [0.15M NaCl, 0.015M sodium citrate] at room temperature for 30 minutes 3 times, and then at 45 °C for 30 minutes 2 times [T. Maniatis et al., Molecular Cloning, Cold Spring Harbor Laboratory, p. 309 (1982)].

Radioautograms were taken from the washed filters, and strains responding to both probes were searched for by overlapping the 2 radioautograms from each pair of replica filters. By this method, 1 strain responding to both probes [*Escherichia coli* K12 DH1/pTB627 (IFO 14494, FERM BP-1280)] was obtained from 5 × $10^5$ clones.

(2) From the strain obtained in (1) above [*Escherichia coli* K12 DH1/pTB627 (IFO 14494, FERM BP-1280)] was extracted and purified a plasmid DNA (pTB627) by the alkali method [Nucleic Acids Research, *1*, 1513 (1979)].

Reference Example 2

(Expression in *Escherichia coli* of gene which encodes human bFGF)

(1) Construction of the plasmid pTB669 for human bFGF expression:

The plasmid pTB627 containing a human bFGF cDNA obtained in Reference Example 1 (2) above was cleaved using the restriction enzymes Ava I and Bal I to obtain a 0.44 kb DNA fragment containing the region coding for human bFGF. To the Bal I cleavage site (blunt end) of this DNA fragment was ligated the Bgl II linker pCAGATCTG using T4 DNA ligase, and a 0.44 kb Ava I-Bgl II DNA fragment was separated. To this 0.44 kb Ava I-Bgl II DNA fragment T4 DNA ligase was reacted to ligate together the Bgl II cleavage sites, and this was followed by DNA polymerase (Klenow fragment) reaction in the presence of dXTP to blunt the Ava I cleavage site. To this DNA fragment were ligated the synthetic oligonucleotides $^{5'}$AATTCTATGCCAGCATTGC$^{3'}$ and $^{5'}$GCAATGCTGGCATAG$^{3'}$ after phosphorylation, using T4 DNA ligase, and this was followed by cleavage using EcoR I-Bgl II to prepare an about 0.46 kb DNA fragment. Separately, the DNA of plasmid p*trp*781 [Kurokawa, T. et al., Nucleic Acids Research, *11*, 3077-3085 (1983)], which has *trp* promoter, was cleaved using Pst I, and was blunted by T4 DNA polymerase reaction. Alter ligation of the Bgl II linker pCAGATCTG to the blunt end by T4 DNA ligase reaction, cleavage using EcoR I-Bgl II was carried out to separate an about 3.2 kb DNA fragment containing *trp* promoter, the tetracycline resistance gene and a plasmid replication initiation site. The above-mentioned 0.46 kb EcoR I-Bgl II DNA fragment containing the gene region encoding human bFGF and this 3.2 kb DNA fragment were ligated together by T4 DNA ligase reaction to construct the plasmid pTB669 for human bFGF expression.

Using this plasmid pTB669, *Escherichia coli* DH1 was transformed, whereby *Escherichia coli* DH1/pTB669, which harbors the plasmid pTB669, was obtained.

In addition, using pTB669, *Escherichia coli* K12 MM294 or C600 was transformed in the same manner as above, whereby *Escherichia coli* K12 MM294/pTB669 (IFO 14532, FERM BP-1281) and *E. coli* C600/pTB669 were respectively obtained.

(2) Preparation of bacterial cell extracts:

Each of the above-mentioned transformants were cultured in an M9 medium containing 1% glucose, 0.4% casamino acid and 8 μg/mℓ tetracycline, and, when Klett value became about 200, 3β-indolylacrylic acid was added to a concentration of 25 μg/mℓ, and this was followed by 4 more hours of cultivation. After cultivation, bacterial cells were collected, and were suspended in a 10% sucrose solution containing a 1/20 amount of 20mM Tris-HCl (pH 7.6). To this suspension were added phenylmethylsulfonyl fluoride (PMSF) to 1mM, EDTA to 10mM, NaCl to 0.1M, spermidine hydrochloride to 10mM and lysozyme to 100 μg/mℓ - (every figure shows the final concentration), and the mixture was left at 0°C for 45 minutes, after which it was subjected to ultrasonication for 30 seconds. This solution was centrifuged at 18000 rpm (Sorval centrifuge, SS34 rotor) for 30 minutes to give a supernatant, which was used as a bacterial cell extract.

(3) Human bFGF activity of bacterial cell extracts:

Human bFGF activities are indicated by the weights of the standard sample of purified bovine brain FGF (produced by Takara Shuzo Co., Ltd.) in amounts equivalent to those of bacterial cell extracts in activity in growth promoting action on BALB/c3T3 cells.

Mouse BALB/c3T3 cells, in an amount of $2 \times 10^3$ cells per well, were inoculated to a DMEM medium containing 5% calf serum on a Nunc 96-well microtiter plate (flat base) with each well containing 0.2 mℓ of the medium, and were cultured. Next day the medium was replaced with a DMEM medium containing 0.5% calf serum. Alter 3 days of cultivation, 10 μℓ of a bacterial cell extract, previously serially diluted in 5-fold steps with a DME medium containing 0.5% BSA, was added to each well, and was cultured. 20 hours later, 2 μℓ of $^3$H-Tdr (5 Ci/mmol, 0.5 mCi/mℓ RCC Amersham, UK) was added to each well. 6 hours later, cells were stripped by treatment with a phosphate-buffered solution (PBS) containing 0.2% trypsin-0.02% EDTA, and the cells were harvested onto a glass filter by means of a Titertech cell harvester, whereafter the amount of $^3$H-Tdr taken in the cells was determined using a scintillation counter. Using the same procedure, determinations were made of the activities of bovine brain FGF samples (produced by Takara Shuzo) of known weight. From the working curve thus obtained, calculations were made of the amounts of human bFGF in the bacterial cell extract samples. The results are shown in Table 2.

For control, the human bFGF productivity of the transformant *E. coli* DH1/p*trp*781 obtained by transformation of *Escherichia coli* DH1 using the plasmid p*trp*781 was determined.

Table 2

| Human bFGF Productivities | |
|---|---|
| Transformant | Human bFGF Productivity (per liter of culture medium) |
| E. coli DH1/pTB669 | 2.95 mg |
| E. coli MM294/pTB669 | 23.15 |
| E. coli C600/pTB669 | 8.15 |
| E. coli DH1/ptrp781 | <0.0005 |

Example 1

(Production of recombinant DNAs containing mutein-encoding base sequence)

(1) Cloning of M13 vector of human bFGF gene:

The plasmid pTB669 obtained in Reference Example 2 was digested with the restriction enzymes EcoR I and BamH I. The phage vector M13mp8 [J. Messing, Methods, in Enzymology, *101*, 20-78 (1983)] replicative-form (RF) DNA was digested with the restriction enzymes EcoR I and BamH I, and was mixed with the human bFGF DNA fragment deriving from the digested pTB669. The mixture was then ligated together by means of T4 DNA ligase; the ligated DNA was transformed into an infectable cell of the strain *Escherichia coli* JM105; the resulting transformant was inoculated onto a plate using Xga1 as an indicator [J. Messing et al., Nucleic Acids Research, *9,* 309-321 (1981)]; the plaque containing the recombinant phage (white plaque) was picked up; the base sequence of the recombinated region was determined by the dideoxynucleotide synthetic chain termination method [J. Messing et al., Nucleic Acids Research, *9,* 309 (1981)], whereby it was confirmed that human bFGF DNA had been accurately inserted.

From this M13-PO clone was purified a single-stranded phage DNA, which was then used as a template for site-directed mutagenesis using a synthetic oligonucleotide.

(2) Site-specific mutagenesis:

In the presence of 0.1mM adenosine triphosphate (ATP), 50mM Tris (hydroxymethyl)aminomethane hydrochloride (Tris-HCl, pH 8.0), 10mM $MgCl_2$, 5mM dithiothreitol (DTT) and 9 units of T4 kinase, in a total amount of 50 $\mu\ell$, 40 picomoles of the synthetic oligonucleotide:
5' > CGT TCT TGC TGT AGA GCC GCT < 3'
[primer for changing *Cys* 26 to *Ser* (the recognition sequence for the restriction enzyme Rsa I disappears.) (see Figure 2)] was treated with T4 kinase at 37°C for 1 hour. This kinase-treated primer (12 picomoles) was heated at 67°C for 5 minutes, and at 42°C for 25 minutes, in 50 $\mu\ell$ of a mixture containing 50mM NaCl, 1.0mM Tris-HCl (pH 8.0), 10mM $MgCl_2$ and 10mM $\beta$-mercaptoethanol, whereby the primer was hybridized to 5 $\mu$g of the single-stranded (ss) M13-PO DNA. The annealed mixture was then cooled on ice, and it was added to 50 $\mu\ell$ of a reaction mixture containing 0.5mM deoxynucleotide triphosphate (dNTP), 80mM Tris-HCl (pH 7.4), 8mM $MgCl_2$, 100mM NaCl, 9 units of DNA plymerase I Klenow fragment, 0.5mM ATP and 2 units of T4 DNA ligase, and reaction was carried out at 37°C for 3 hours, and at 25°C for 2 hours, whereafter the reaction was stopped by adding 2 $\mu\ell$ of 0.2mM EDTA. The reaction product was used to transform infectable JM 105 cells; the transformant cells thus obtained were allowed to grow overnight, whereafter an ssDNA was isolated from the culture medium supernatant. Using this ssDNA as a template for the 2nd cycle of primer elongation, gel-purified RF-type DNA was transformed into infectable JM 105 cells; the resulting transformant cells were spread over an agar plate, and were cultured overnight to obtain phage plaques.

(3) Site-directed mutagenesis:

The procedure of the above term(2) was repeated but the used synthetic oligonucleotide primer was:
5' > AAC GAT TAG CGC TCA CTC C < 3'
which so changes the cysteine-70-encoding codon that the codon encodes serine. (A recognition sequence of the restriction enzyme Hae II was produced.) (See Figure 2.)

(4) Site-directed mutagenesis:

The procedure of the above term (2) was repeated but the used synthetic oligonucleotide primer was:
5' > GTA ACA GAC TTA GAA GCT AGT < 3'
which so changes the cysteine-88-encoding codon that the codon encodes serine. (A recognition sequence for the restriction enzyme Alu I was produced.) (See Figure 2.)

(5) Site-directed mutagenesis:

The procedure of the above term (2) was repeated but the used synthetic oligonucleotide primer was:
5' > TCG AAG AAG AAA GAC TCA TCC < 3'
which so changes the cysteine-93-encoding codon that the codon encodes serine. (A recognition sequence for the restriction enzyme Hinf I was produced.) (See Figure 2.)

(6) Screening and identification of plaques made mutagenic:

Plates containing mutated M13-PO plaques [above term (1)] and 2 plates containing unmutated M13-PO phage plaques were cooled to 4°C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl having a pH-value of 7.5 and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridization at 55°C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 μg/mℓ denatured salmon sperm DNA. Hybridization was carried out at 42°C for 24 hours with $10^5$ cpm/mℓ of an oligonucleotide primer. The filters were each washed in a buffer solution for washing containing 0.1% SDS and a reduced amount of SSC at 50°C for 30 minutes. The filters were then first washed with a buffer solution containing 2 × SSC; the control filters, which contained unmutated M13-PO plaques, were examined for radioactivity using a Geiger counter. While stepwise reducing SSC concentration, the control filters were washed until no detectable radioactivity remained on the filters. The minimum of the used SSC concentrations was 0.1 × SSC. The filters were allowed to dry in air, and autoradiographs were taken by 2 to 3 days of exposure at - 70°C. Screening was carried out of 10,000 mutated M13-PO plaques and 100 unmutated control plaques using a kinase-treated oligonucleotide probe. None of the control plaques hybridized to the probe, while 3 to 10 of the mutated M13-PO plaques hybridized to the probe.

One of the mutated M13-PO plaques was taken, and was inoculated to a JM105 culture medium. From the supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGC (*Cys*-26) codon had been changed to a TCT (*Ser*) codon; the TGT (*Cy*s-70) codon, to an AGC (*Ser*) codon; the TGT (*Cys*-88) codon, to a TCT (*Ser*) codon; and the TGT (*Cys*-93) codon, to a TCT (*Ser*) codon.

Of the mutated M13-PO phages, the phage in which the codon *Cys*-26 had become a *Ser*-encoding codon was named M13-P1; the phage in which the codon *Cys*-70 had become a *Ser*-encoding codon, M13-P2; the phage in which the codon *Cys*-88, M13-P3; and the phage in which the codon *Cys*-93 had become a *Ser*-encoding codon, M13-P4.

Example 2

(Expression in *Escherichia coli* of gene which encodes human bFGF mutein)

(1) Construction of the plasmid pTB739 for human bFGF mutein expression:

The M13-P1 replicative form (RF) obtained in Example 1 above was cleaved using the restriction enzymes EcoR I and Pst I to obtain about 0.5 kb DNA fragment containing the region encoding human bFGF mutein.

Separately, the plasmid p*trp*781 [Kurokawa, T. et al., Nucleic Acids Research, *11*, 3077-3085 (1983)] DNA containing a *trp* promoter was cleaved using EcoR I-Pst I to separate an about 3.2 kb DNA fragment containing a *trp* promoter, a tetracycline resistance gene and a plasmid replication initiation site. The above-mentioned 0.5 kb EcoR I-Pst I DNA fragment containing the gene region encoding human bFGF mutein and this 3.2 kb DNA fragment were ligated together by T4 DNA ligase reaction to construct the plasmid pTB739 for human bFGF mutein expression (Figure 7).

Using this plasmid pTB739, *Escherichia coli* DH1 was transformed, whereby the strain *Escherichia coli* DH1/pTB739 (IFO 14575, FERM BP-1641) was obtained, which harbors the plasmid pTB739 containing the mutein-encoding gene shown in Figure 3.

(2) Preparation of bacterial cell extract:

The above-mentioned transformant was cultured in an M9 medium containing 1% glucose, 0.4% casamino acid and 8 $\mu$g/mℓ tetracycline, and, when Klett value became about 200, 3$\beta$-indolylacrylic acid was added to a concentration of 25 $\mu$g/mℓ, and this was followed by cultivation for 4 more hours. After cultivation, bacterial cells were collected, and were suspended in a 10% sucrose solution containing a 1/20 amount of 20mM Tris-HCl (pH 7.6). To this suspension were added phenylmethylsulfonyl fluoride (PMSF) to 1mM, EDTA to 10mM, NaCl to 0.1M, spermidine hydrochloride to 10mM and lysozyme to 100 $\mu$g/mℓ - (every figure shows the final concentration), and the mixture was left at 0°C for 45 minutes, after which it was subjected to ultrasonication for 30 seconds. This solution was centrifuged at 18,000 rpm (Sorval® centrifuge, SS34 rotor) for 30 minutes to give a supernatant, which was then used as a bacterial cell extract.

(3) Human bFGF activity of the bacterial cell extract:

Mouse BALB/c3T3 cells, in an amount of $2 \times 10^3$ cells per well, were inoculated to a DMEM medium containing 5% calf serum on Nunc 96-well microtiter plate (flat base) with each well containing 0.2 mℓ of the medium, and were cultured. Next day the medium was replaced with a DMEM medium containing 0.5% calf serum. Alter 3 days of cultivation, 10 $\mu$ℓ of the bacterial cell extract, previously serially diluted in 5-fold steps with a DME medium containing 0.5% BSA, was added to each well, and was cultured. 20 hours later, 2 $\mu$ℓ of $^3$H-Tdr (5 Ci/mmol, 0.5 mCi/mℓ RCC Amersham) was added to each well. 6 hours later, cells were stripped by treatment with a phosphate-buffered solution (PBS) containing 0.2% trypsin-0.02% EDTA, and the cells were harvested onto a glass filter by means of a Titertech cell harvester, whereafter the amout of $^3$H-Tdr taken in the cells was determined using a scintillation counter.

As a result, the bacterial cell extract from *E. coli* DH1/pTB739 exhibited FGF activity.

The mutein CS1, in which *Cys* at the 26-position of human bFGF had been replaced by *Ser*, was thus obtained.

Example 3

(Expression in *Escherichia coli* of gene encoding human bFGF mutein)

(1) Construction of the plasmid pTB742 for human bFGF mutein expression:

The M13-P2 replicative form (RF) obtained in Example 1 above was cleaved using the restriction enzymes EcoR I and Pst I to obtain an about 0.5 kb DNA fragment containing a region which encodes a human bFGF mutein.

Separately, a plasmid p*trp*781 DNA containing a *trp* promoter was cleaved using EcoR I-Pst I to separate an about 3.2 kb DNA fragment containing a *trp* promoter, a tetracycline resistance gene and a plasmid replication initiation site. This 3.2 kb DNA fragment and the above-mentioned 0.5 kb EcoR I-Pst I DNA fragment containing a gene region encoding a human bFGF mutein were ligated together by T4 DNA ligase reaction to construct the plasmid pTB742 for the expression of a human bFGF mutein (Figure 8).

Using this plasmid pTB742, *Escherichia coli* DH1 was transformed, whereby the strain *Escherichia coli* DH1/pTB742 (IFO 14584, FERM BP-1642) was obtained, which harbors the plasmid pTB742 containing the mutein-encoding gene shown in Figure 4.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity on the bacterial cell extract obtained in (2) above, by the method described in Example 2 (3).

As a result, the bacterial cell extract of *E. coli* DH1/pTB742 exhibited FGF activity.

The mutein CS2, in which *Cys* at the 70-position of human bFGF had been replaced by *Ser*, was thus obtained.

Example 4

(Expression in *Escherichia coli* of gene encoding human bFGF mutein)

(1) Construction of the plasmid pTB743 for human bFGF mutein expression:

The M13-P3 replicative form (RF) obtained in Example 1 above was cleaved using the restriction enzymes EcoR I and Pst I to obtain an about 0.5 kb DNA fragment containing a region which encodes a human bFGF mutein.

Separately, a plasmid p*trp*781 DNA containing a *trp* promoter was cleaved using EcoR I-Pst I to separate an about 3.2 kb DNA fragment containing a *trp* promoter, a tetracycline resistance gene and a plasmid replication initiation site. This 3.2 kb DNA fragment and the above-mentioned 0.5 kb EcoR I-Pst I DNA fragment containing a gene region encoding a human bFGF mutein were ligated together by T4 DNA ligase reaction to construct the plasmid pTB743 for the expression of human bFGF mutein (Figure 9).

Using this plasmid pTB743, *Escherichia coli* DH1 was transformed, whereby the strain *Escherichia coli* DH1/pTB743 (IFO 14585, FERM BP-1643) was obtained, which harbors the plasmid pTB743 containing the mutein-encoding gene shown in Figure 5.

(2) Preparation of bacterial cell extract:

The above-mentioned transformant was cultured in the manner described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* DH1/pTB743 exhibited FGF activity.

The mutein CS3, in which *Cys* at the 88-position of human bFGF had been replaced by *Ser*, was thus obtained.

Example 5

(Expression in *Escherichia coli* of gene which encodes human bFGF mutein)

(1) Construction of the plasmid pTB744 for human bFGF mutein expression:

The M13-P4 replicative form (RF) obtained in Example 1 above was cleaved using the restriction enzymes EcoR I and Pst I to obtain an about 0.5 kb DNA fragment containing a region which encodes a human bFGF mutein.

Separately, a plasmid p*trp*781 DNA containing a *trp* promoter was cleaved using EcoR I-Pst I to separate an about 3.2 kb DNA fragment containing a *trp* promoter, a tetracycline resistance gene and a plasmid replication initiation site. This 3.2 kb DNA fragment and the above-mentioned 0.5 kb EcoR I-Pst I DNA fragment containing a gene region encoding a human bFGF mutein were ligated together by T4 DNA ligase reaction to construct the plasmid pTB744 for the expression of a human bFGF mutein (Figure 10).

Using this plasmid pTB744, *Escherichia coli* DH1 was transformed, whereby the strain *Escherichia coli* DH1/pTB744 (IFO 14586, FERM BP-1644) was obtained, which harbors the plasmid pTB744 containing the mutein-encoding gene shown in Figure 6.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from *E. coli* DH1/pTB744 exhibited FGF activity.

The mutein CS4, in which *Cys* at the 93-position in human bFGF had been replaced by *Ser* was thus obtained.

Example 6

(Screening and identification of plaques which were made mutagenic)

Plates containing mutated M13-P2 phage plaques obtained in Example 1 and 2 plates containing unmutated M13-P2 phage plaques obtained in Example 1 were cooled to 4°C, and the plaque from each plate was transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridization at 55°C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 μg/mℓ denatured salmon sperm DNA. Hybridization was carried out at 42°C for 24 hours with $10^5$ cpm/mℓ of an oligonucleotide primer. The filters were each washed in a buffer solution for washing containing 0.1% SDS and a reduced amount of SSC at 50°C for 30 minutes. The filters were then first washed with a buffer solution containing 2 × SSC; the control filters, which contained unmutated M13-P2 plaques, were examined for radioactivity using a Geiger counter. While stepwise reducing SSC concentration, the control filters were washed until no detectable radioactivity remained on the filters. The minimum of the used SSC concentrations was 0.1 × SSC. The filters were allowed to dry in air, and radioautographs were taken by 2 to 3 days of exposure at - 70°C. Screening was carried out of 10,000 mutated M13-P2 plaques and 100 unmutated control plaques using a kinase-treated oligonucleotide probe. None of the control plaques hybridized to the probe, while 3 to 10 of the mutated M13-P2 plaques hybridized to the probe.

One of the mutated M13-P2 plaques was taken, and was inoculated to a JM105 culture medium. From the resulting supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGC (*Cys*-26) codon had been changed to a TCT (*Ser*) codon; the TGT (*Cys*-88) codon, to a TCT (*Ser*) codon; and the TGT (*Cys*-93) codon, to a TCT (*Ser*) codon.

Of the mutated M13-P2 phages, the phage in which the codons *Cys*-26 and -70 had become *Ser*-encoding codons was named M13-P12; the phage in which the codons *Cys*-70 and -88 had become *Ser*-encoding codons, M13-P23; and the phage in which the codons *Cys*-70 and -93 had become *Ser*-encoding codons, M13-P24.

Example 7

(Expression in *Escherichia coli* of gene encoding human bFGF mutein)

(1) Construction of the plasmid pTB762 for human bFGF mutein expression:

The M13-P23 replicative form (RF) obtained in Example 6 above was treated in the manner described in Example 2 (1) to construct the plasmid pTB762 for human bFGF mutein expression (Figure 12).

Using this plasmid pTB762, *Escherichia coli* MM294 was transformed, whereby the strain *Escherichia coli* MM294/pTB762 (IFO 14613, FERM BP-1645) was obtained, which harbors the plasmid pTB762 containing the mutein-encoding gene shown in Figure 11.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from *E. coli* MM294/pTB762 exhibited FGF activity.

The mutein CS23, in which *Cys* at the 70-position and at the 88-position had been replaced by *Ser*, was thus obtained.

Example 8

(Screening and identification of plaques made mutagenic)

Plates containing mutated M13-P23 phage plaques obtained in Example 7 and 2 plates containing unmutated M13-P23 phage plaques obtained in Example 7 were cooled to 4 °C, and the plaque from each plate was transferred to 2 round nitrocellulose filters by keeping a dry filter placed on the agar plate for 5 minutes in the case of te 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80 °C for 2 hours in a vacuum oven. The overlapped filters were subjected to prehybridization at 55 °C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 µg/mℓ denatured salmon sperm DNA. Hybridization was carried out at 42 °C for 24 hours with $10^5$ cpm/mℓ of an oligonucleotide primer. Each filter was washed in a buffer solution for washing containing 0.1% SDS and a reduced amount of SSC at 50 °C for 30 minutes. The filters were then first washed with a buffer solution containing 2 × SSC; the control filters, which contained unmutated M13-P23 plaques, were examined for radioactivity using a Geiger counter. While stepwise reducing SSC concentration, the control filters, which contained unmutated M13-P23 plaques, were washed until no detectable radioactivity remained on the filters. The minimum of the used SSC concentrations was 0.1 × SSC. The filters were allowed to dry in air, and autoradiographs were taken by 2 to 3 days of exposure at - 70 °C. Screening was carried out of 10,000 mutated M13-P23 plaques and 100 unmutated control plaques by means of a kinase-treated oligonucleotide probe. None of the control plaques hibridized to the probe, while 3 to 10 of the mutated M13-P23 plaques hybridized to the probe.

One of the mutated M13-P23 plaques was taken, and was inoculated to a JM105 culture medium. From the resulting supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGC (*Cys*-26) codon had been changed to a TCT (*Ser*) codon; and the TGT (*Cys*-93) codon, to a TCT (Ser) codon.

Of the mutated M13-P23 phages, the phage in which the codons *Cys*-26, -70 and -88 had become *Ser*-encoding codons was named M13-P123; and the phage in which the codons *Cys*-70, -88 and -93 had become *Ser*-encoding codons, M13-P234.

Example 9

(Expression in *Escherichia coli* of gene encoding human bFGF mutein)

(1) Construction of the plasmid pTB764 for human bFGF mutein expression:

The M13-P123 replicative form (RF) obtained in Example 8 above was treated in the manner described in Example 2 (1) to construct the plasmid pTB764 for human bFGF mutein expression (Figure 14).

Using this plasmid pTB764, *Escherichia coli* MM294 was transformed, whereby the strain *Escherichia coli* MM294/pTB764 (IFO 14614, FERM BP-1646) was obtained, which harbors the plasmid pTB764 containing the mutein-encoding gene shown in Figure 13.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF acitivity of the bacterial cell extract obtained in (2) above, by the method described in Example 2 (3).

As a result, the bacterial cell extract from *E. coli* MM294/pTB764 exhibited FGF activity.

The mutein CS123, in which *Cys* at the 26-, 70- and 88-positions in human bFGF had been replaced by *Ser*, was thus obtained.

Example 10

(Screening and identification of plaques which were made mutagenic)

Plates containing mutated M13-P123 phage plaques obtained in Example 8 and 2 plates containing unmutated M13-P123 phage plaques obtained in Example 8 were cooled to 4 °C, and the plaque from each plate was transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80 °C for 2 hours in a vacuum oven. The overlapped filters were subjected to prehybridization at 55 °C for 4 hours with 10 mℓ/filter of a DNA hybridization buffer solution (5 × SSC) having a pH-value of 7.0 containing 4 × Denhardt's solution (polyvinyl-pyrrolidone, Ficoll® and bovine serum albumin, 1 × = 0.02%), 0.1% sodium dodecyl sulfate (SDS), 50mM sodium phosphate-buffered solution having a pH-value of 7.0 and 100μg/mℓ denatured salmon sperm DNA. Hybridization was carried out at 42 °C for 24 hours with $10^5$ cpm/mℓ of an oligonucleotide primer. The filters were each washed in a buffer solution for washing containing 0.1% SDS and a reduced amount of SSC at 50 °C for 30 minutes. The filters were then first washed with a buffer solution containing 2 × SSC; the control filters, which contained unmutated M13-P123 plaques, were examined for radioactivity using a Geiger counter. While stepwise reducing SSC concentration, the control filters, which contained unmutated M13-P123 plaques, were washed until no detectable radioactivity remained on the filters. The minimum of the used SSC concentrations was 0.1 × SSC. The filters were allowed to dry in air, and autoradiographs were taken by 2 to 3 days of exposure at -70 °C. Screening was carried out of 10,000 mutated M13-P123 plaques and 100 unmutated control plaques by means of a kinase-treated oligonucleotide probe. None of the control plaques hybridized to the probe, while 3 to 10 of the mutated M13-P123 plaques hybridized to the probe.

One of the mutated M13-P123 plaques was taken, and was inoculated to a JM105 culture medium. From the resulting supernatant an ssDNA was prepared, and from the bacterial cell pelletes a double-stranded (ds) DNA was prepared. Analyses were made of the base sequence using an appropriate oligonucleotide primer and ssDNA.

As a result, it was confirmed that the TGT (*Cys*-93) codon had been changed to a TCT (*Ser*) codon.

Of the mutated M13-P123 phages, the phage in which the codons *Cys*-26, -70, -88 and -93 had become *Ser*-encoding codons was named M13-P1234.

Example 11

(Expression in *Escherichia coli* of gene encoding human bFGF mutein)

(1) Construction of the plasmid pTB765 for human bFGF mutein expression:

The M13-P1234 replicative form (RF) obtained in Example 10 above was treated in the manner described in Example 2 (1) to construct the plasmid pTB765 for the expression of a human bFGF mutein (Figure 16).

Using this plasmid pTB765, *Escherichia coli* MM294 was transformed, whreby the strain *Escherichia coli* MM294/pTB765 (IFO 14615, FERM BP-1647) was obtained, which harbors the plasmid pTB765 containing the mutein-encoding gene shown in Figure 15.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, in the manner described in Example 2 (3).

As a result, the bacterial cell extract from *E. coli* MM294/pTB765 exhibited FGF activity.

The mutein CS1234, in which *Cys* at the 26-, 70-, 88- and 93-positions in human bFGF had been replaced by *Ser*, was thus obtained.

Example 12

(Screening and identification of plaques which were made mutagenic)

Plates containing M13-P1 phages mutated by the method of Example 1 using a synthetic oligomer [Figure 2 (3) or (4)] and 2 plates containing unmutated M13-P1 phage plagues obtained in Example 1 were cooled to 4 °C, and the plaque from each plate was transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80 °C for 2 hours in a vacuum oven. The overlapped filters were subjected to prehybridization at 55 °C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 µg/mℓ denatured salmon sperm DNA. Hybridization was carried out at 42 °C for 24 hours with $10^5$ cpm/mℓ of an oligonucleotide primer. The filters were each washed in a buffer solution for washing containing 0.1% SDS and a reduced amount of SSC at 50 °C for 30 minutes. The filters were then first washed with a buffer solution containing 2 × SSC; the control filters, which contained unmutated M13-P1 plaques, were examined for radioactivity using a Geiger counter. While reducing SSC concentration stepwise, the control filters, which contained unmutated M13-P1 plaques, were washed until no detectable radioactivity remained on the filters. The minimum of the used SSC concentrations was 0.1 × SSC. The filters were allowed to dry in air, and radioautographs were taken by 2 to 3 days of exposure at -70 °C. Screening was carried out of 10,000 mutated M13-P1 plaques and 100 unmutated control plaques using an oligonucleotide probe treated with kinase in the presence of $\gamma$-$^{32}$P-ATP. None of the control plaques hybridized to the probe, while 3 to 10 of the mutated M13-P1 plaques hybridized to the probe.

One of the mutated M13-P1 plaques was taken, and was inoculated to a JM105 culture medium. From the resulting supernatant an ssDNA was prepared, and from the bacterial cell pellets a double-stranded (ds)DNA was prepared. Analyses were made of the base sequences using appropriate oligonucleotide primers and ssDNAs.

As a result, it was respectively confirmed that the TGT (*Cys*-88) codon had been changed to a TCT (*Ser*) codon; and the TGT (*Cys*-93) codon, to a TCT (*Ser*) codon.

Of the mutated M13-P1 phages, the phage in which the codons *Cys*-26 and -88 had become *Ser*-encoding codons was named M13-P13; and the phage in which the codons *Cys*-26 and -93 had become *Ser*-encoding codons, M13-P14.

Example 13

(Screening and identification of plaques made mutagenic)

Plates containing M13-P14 phage plaques mutated by the method of Example 1 using a synthetic oligomer [Figure 2 (2) or (3)] and 2 plates containing unmutated M13-P14 phage plaques were cooled to 4 °C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80 °C for 2 hours in a vacuum oven. The overlapped filters were subjected to prehybridization at 55 °C for 4 hours with 10mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 µg/mℓ denatured salmon sperm DNA. Hybridization was carried out at 42 °C for 24 hours with $10^5$ cpm/mℓ of an oligonucleotide primer. Each filter was washed in a buffer solution for washing containing 0.1% SDS and a reduced amount of SSC at 50 °C for 30 minutes. The filters were then first washed with a buffer solution containing 2 × SSC; the control filters, which contained unmutated M13-P14 plaques, were examined for radioactivity using a Geiger counter. While reducing SSC concentration stepwise, the control filters, which contained unmutated M13-P14 plaques, were washed until no detectable radioactivity remained on the filters. The minimum of the used SSC concentrations was 0.1 × SSC. The filters were allowed to dry in air, and autoradiographs were taken by 2 to 3 days of exposure at -70 °C. Screening was carried out of 10,000 mutated M13-P14 plaques and 100 unmutated control plaques by means of an oligonucleotide probe treated with kinase in the presence of $\gamma$-$^{32}$P-ATP. None of the control plaques hybridized to the probe, while 3 to 10 of the mutated M13-P14 plaques hybridized to the probe.

One of the mutated M13-P14 plaques was taken, and was inoculated to a JM105 culture medium. From the resulting supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGT (*Cys*-70) codon had been changed to a TCT (*Ser*) codon; and the TGT (*Cys*-93) codon, to a TCT (*Ser*) codon.

Of the mutated M13-P14 phages, the phage in which the codons *Cys*-26, -70 and -93 had become *Ser*-encoding codons was named M13-P124; and the phage in which the codons *Cys*-26, -88 and -93 had become *Ser*-encoding codons, M13-P134.

Example 14

(Screening and identification of plaques made mutagenic)

Plates containing M13-P3 phage plaques mutated by the method of Example 1 using a synthetic oligomer [Figure 2 (4)] and 2 plates containing unmutated M13-P3 phage plaques obtained in Example 1 were cooled to 4°C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them for 5 more minutes on filter papers immersed in 0.5M Tris-HCl having a pH-value of 7.5 and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridization at 55°C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50mM sodium phosphate-buffered solution having a pH-value of 7.0 and 100 µg/mℓ denatured salmon sperm DNA. Hybridization was carried out at 42°C for 24 hours with $10^5$ cpm/mℓ of an oligonucleotide primer. The filters were each washed in a buffer solution for washing containing 0.1% SDS and a reduced amount of SSC at 50°C for 30 minutes. The filters were then first washed with a buffer solution containing 2 × SSC; the control filters, which contained unmutated M13-P3 plaques, were examined for radioactivity using a Geiger counter. While stepwise reducing SSC concentration, the control filters, which contained unmutated M13-P3 plaques, were washed until no detectable radioactivity remained on the filters. The minimum of the used SSC concentrations was 0.1 × SSC. The filters were allowed to dry in air, and autoradiographs were taken by 2 to 3 days of exposure at -70°C. Screening was carried out of 10,000 mutated M13-P3 plaques and 100 unmutated control plaques by means of an oligonucleotide primer treated with kinase in the presence of $\gamma$-$^{32}$P-ATP. None of the control plaques hybridized to the probe, while 3 to 10 of the mutated M13-P3 plaques hybridized to the probe.

One of the mutated M13-P3 plaques was taken, and was inoculated to a JM105 culture medium. From the resulting supernatant an ssDNA was prepared, and from the bacterial cell pellets a double-stranded (ds) DNA was prepared. Analyses were made of the base sequences using an appropriate oligonucleotide primer and ssDNA.

As a result, it was confirmed that the TGT (*Cys*-93) codon had been changed to a TCT (*Ser*) codon.

Of the mutated M13-P3 phages, the phage in which the codons *Cys*-88 and -93 had become *Ser*-encoding codons was named M13-P34.

Example 15

(Expression in *Escherichia coli* of gene which encodes human bFGF mutein)

(1) Construction of the plasmid pTB776 for human bFGF mutein expression:

The M13-P12 replicative form (RF) obtained in Example 6 above was treated in the manner described in Example 2 (1) to construct the plasmid pTB776 for human bFGF mutein (Figure 18).

Using this plasmid pTB776, Escherichia coli MM294 was transformed, whereby the strain Escherichia coli MM294/pTB776 was obtained, which harbors the plasmid pTB776 containing the mutein-encoding gene shown in Figure 17.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB776 exhibited FGF activity.

The mutein CS12, in which Cys at the 26- and 70-positions of human bFGF had been replaced by Ser, was thus obtained.

Example 16

(Expression in Escherichia coli of gene which encodes human bFGF mutein)

(1) Construction of the plasmid pTB778 for human bFGF mutein expression:

The M13-P24 replicative form (RF) obtained in Example 6 above was treated in the manner described in Example 2 (1) to construct the plasmid pTB778 for human bFGF mutein expression (Figure 20).

Using this plasmid pTB778, *Escherichia coli* MM294 was transformed, whereby the strain *Escherichia coli* MM294/pTB778 was obtained, which contains the plasmid pTB778 containing the mutein-encoding gene shown in Figure 19.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB778 exhibited FGF activity.

The mutein CS24, in which *Cys* at the 70- and 93-positions of human bFGF had been replaced by *Ser*, was thus obtained.

Example 17

(Expression in *Escherichia coli* of gene which encodes human bFGF mutein)

(1) Construction of the plasmid pTB779 for human bFGF mutein expression:

The M13-P13 replicative form (RF) obtained in Example 12 above was treated in the manner described in Example 2 (1) to construct the plasmid pTB779 for human bFGF mutein expression (Figure 22).

Using this plasmid pTB779, *Escherichia coli* MM294 was transformed, whereby the strain *Escherichia coli* MM294/pTB779 was obtained, which harbors the plasmid pTB779 containing the mutein-encoding gene shown in Figure 21.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB779 exhibited FGF activity.

The mutein CS13, in which *Cys* at the 26- and 88-positions of human bFGF had been replaced by *Ser*, was thus obtained.

Example 18

(Expression in *Escherichia coli* of gene which encodes human bFGF mutein)

(1) Construction of the plasmid pTB763 for human bFGF mutein expression:

The M13-P14 replicative form (RF) obtained in Example 12 above was treated in the manner described in Example 2 (1) to construct the plasmid pTB763 for human bFGF mutein expression (Figure 24).

Using this plasmid pTB763, *Escherichia coli* MM294 was transformed, whereby the strain *Escherichia coli* MM294/pTB763 was obtained, which harbors the plasmid pTB763 containing the mutein-encoding gene shown in Figure 23.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB763 exhibited FGF activity.

The mutein CS14, in which *Cys* at the 26- and 93-positions of human bFGF had been replaced by *Ser*, was thus obtained.

Example 19

(Expression in *Escherichia coli* of gene which encodes human bFGF mutein)

(1) Construction of the plasmid pTB777 for human bFGF mutein expression:

The M13-P34 replicative form (RF) obtained in Example 14 above was treated in the manner described in Example 2 (1) to construct the plasmid pTB777 for human bFGF mutein expression (Figure 26).

Using this plasmid pTB777, *Escherichia coli* MM294 was transformed, whereby the strain *Escherichia coli* MM294/pTB777 was obtained, which harbors the plasmid pTB777 containing the mutein-encoding gene shown in Figure 25.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB777 exhibited FGF activity.

The mutein CS34, in which *Cys* at the 88- and 93-positions of human bFGF had been replaced by *Ser*, was thus obtained.

Example 20

(Expression in *Escherichia coli* of gene which encodes human bFGF mutein)

(1) Construction of the plasmid pTB782 for human bFGF mutein expression:

The M13-P234 replicative form (RF) obtained in Example 8 above was treated in the manner described in Example 2 (1) to construct the plasmid pTB782 for human bFGF mutein expression (Figure 28).

Using this plasmid pTB782, *Escherichia coli* MM294 was transformed, whereby the strain *Escherichia coli* MM294/pTB782 was obtained, which harbors the plasmid pTB782 containing the mutein-encoding gene shown in Figure 27.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB782 exhibited FGF activity.

The mutein CS234, in which *Cys* at the 70-, 88- and 93-positions of human bFGF had been replaced by *Ser*, was thus obtained.

Example 21

(Expression in *Escherichia coli* of gene which encodes human bFGF mutein)

(1) Construction of the plasmid pTB780 for human bFGF mutein expression:

The M13-P124 replicative form (RF) obtained in Example 13 above was treated in the manner described in Example 2 (1) to construct the plasmid pTB780 for human bFGF mutein expression (Figure 30).

Using this plasmid pTB780, *Escherichia coli* MM294 was transformed, whereby the strain *Escherichia coli* MM294/pTB780 was obtained, which harbors the plasmid pTB780 containing the mutein-encoding gene shown in Figure 29.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB780 exhibited FGF activity.

The mutein CS124, in which *Cys* at the 26-, 70- and 93-positions of human bFGF had been replaced by *Ser*, was thus obtained.

Example 22

(Expression in *Escherichia coli* of gene which encodes human bFGF)

(1) Construction of the plasmid pTB781 for human bFGF mutein expression:

The M13-P134 replicative form (RF) obtained Example 13 above was treated in the manner described in Example 2 (1) to construct the plasmid pTB781 for human bFGF mutein expression (Figure 31).

Using this plasmid pTB781, *Escherichia coli* MM294 was transformed, whereby the strain *Escherichia coli* MM294/pTB781 was obtained, which harbors the plasmid pTB781 containing the mutein-encoding gene shown in Figure 32.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract of *E. coli* MM294/pTB781 exhibited FGF activity.

The mutein CS134, in which *Cys* at the 26-, 88- and 93-positions of human bFGF had been replaced by *Ser*, was thus obtained.

Example 23

(Mutein productivities)

Determinations were made of the FGF activities of bacterial cell extracts containing respective muteins in accordance with the method of Reference example 2 (2), whereby the mutein productivities of respective transformants were calculated. The values thus obtained are shown in Table 3.

Table 3

| Plasmid | Mutein | Productivity (mg-ptFGF/1 culture) |
|---|---|---|
| pTB739 | CS1 | 0.3 |
| pTB742 | CS2 | 10.6 |
| pTB743 | CS3 | 13.3 |
| pTB744 | CS4 | 0.8 |
| pTB776 | CS12 | 0.2 |
| pTB779 | CS13 | 0.1 |
| pTB763 | CS14 | 0.5 |
| pTB762 | CS23 | 25.8 |
| pTB778 | CS24 | 0.3 |
| pTB777 | CS34 | 0.5 |
| pTB764 | CS123 | 0.3 |
| pTB780 | CS124 | 0.4 |
| pTB781 | CS134 | 0.2 |
| pTB782 | CS234 | 1.4 |
| pTB765 | CS1234 | 0.1 |
| pTB669 | rhbFGF | 6.0 |
| ptrp781 | - | 0.0 |

Example 24

(Purification of hbFGF mutein)

Transformants producing respective muteins were cultured by the method described in Reference example 2, whereby bacterial cell extracts were prepared. 25 mℓ of each extract (prepared from 500 mℓ of the culture medium) was passed through a column (ϕ2 × 10 cm) of DEAE cellulose (DE52, Wattman®, Inc., United Kingdom) equilibrated with a solution containing 20mM Tris-HCl having a pH-value of 7.4 and 0.2M NaCl, whereby the nucleic acid constituents in the extract were removed. The effluent from the column and the washings resulting from the washing of the column with a solution containing 20mM Tris-HCl having a pH-value of 7.4 and 0.2M NaCl were combined and collected (DEAE effluent fraction, 60 mℓ).

This fraction was subjected to high performance liquid chromatography using a high performance liquid chromatograph (Gilson, Inc., France) loaded with a Shodex AF-pak HR-894® heparin column (8 mm ID × 5 cm, produced by Showa Denko, Japan). After washing the column with a 20mM Tris-HCl solution having a pH-value of 7.4, and then with a solution containing 20mM Tris-HCl having a pH-value of 7.4 and 0.5M NaCl, linear gradient elution was carried out with the NaCl gradient of from 0.5M to 2M (60 mℓ volume, 1.0 mℓ/min. flow rate) in a buffer containing 20mM Tris-HCl having a pH-value of 7.4.

The elution patterns of respective muteins are shown in Figures 33 to 35. In these figures, the ordinates represent absorptions for $OD_{280}$ and NaCl concentrations in the gradient. The abscissas represent times, gradient elution being initiated on the time-0 point. Peak fractions were collected, and their FGF activities were investigated. The post-purification specific activities thereof are shown in Table 4.

Table 4

| Mutein | Specific Activity (mg-ptFGF/mg protein) |
|---|---|
| CS1 | 0.4 |
| CS2 | 0.9 |
| CS3 | 1.0 |
| CS4 | 1.0 |
| CS12 | 0.5 |
| CS13 | 0.5 |
| CS14 | 0.3 |
| CS23 | 1.1 |
| CS24 | 0.8 |
| CS34 | 0.5 |
| CS123 | 0.4 |
| CS124 | 0.1 |
| CS134 | 0.5 |
| CS234 | 0.9 |
| CS1234 | 0.1 |
| rhbFGF | 1.0 |

In Table 4, the specific activities are shown on the basis of the FGF activity of bovine brain-derived FGF (purity, not less than 95%) produced by Takara Shuzo Co., Ltd.

In all muteins, the peak corresponding to Peak I of bFGF was eluted at an elution time between 15 and 25 minutes. This can also be detected as a single band at the position of about 17,000 molecular weight in 17.25% SDS polyacrylamide gel electrophoresis.

Example 25

(Oxidation of FGF muteins)

Each mutein eluted from a heparin HPLC column and bFGF were separately twice dialyzed at 4°C for 3 hours to a 1,000-fold amount by volume of distilled water, and this was followed by lyophilization. 200 µg (protein basis) of each dry standard sample was dissolved in 200 µℓ of an oxidation reaction liquid [50mM Tris-HCl (pH 8.5)/0.2M NaCl/1 mg/mℓ BSA/20mM $H_2O_2$], and oxidation was carried out at 37°C for 30 minutes.

The solution was then passed through a heparin HPLC column, and the pattern which appeared in elution with the salt gradient between 0.6M and 2M was examined. The obtained elution patterns are shown in Figure 36. In bFGF, the peaks were generally low; while in CS2, CS3 and CS23, the peak corresponding to Peak I was high; the peak rise was noticeable specifically in CS3 and CS23.

This suggests that CS3 and CS23 are stable to oxidation. In addition, in all cases of CS2, CS3 and CS23, the protein eluted as the peak exhibited FGF activity.

Example 26

(Reduction of FGF muteins)

200 µg (protein basis) of each lyophilized standard sample obtained in the same manner as in Example 25 was dissolved in 200 µℓ of a reduction reaction liquid [50mM Tris-HCl (pH 8.5)/0.2M NaCl/1 mg/mℓ BSA/20mM dithiothreitol (DTT)], and reduction was carried out at 37 °C for 30 minutes.

The solution was then passed through a heparin HPLC column, and the pattern which appeared in elution with the salt gradient between 0.6M and 2M was examined. The obtained elution patterns are shown in Figure 37. In bFGF and CS2, the peak corresponding to Peak I was noticeable, and it was suggested that the other peaks (Peaks II to IV) are attributable to oxidation. It was also suggested that, in CS3 and CS23, the peak is not affected by oxidation. That is, this supports the stability referred to in Example 25. The bFGF and muteins eluted as these peaks were found to possess FGF activity.

Example 27

(Production of recombinant DNAs having mutein-encoding base sequence)

(1) Cloning of M13 vector for human bFGF gene:

The plasmid pTB669 obtained in Reference example 2 was digested with the restriction enzymes EcoR I and BamH I. Phage vector M13mp8 [J. Messing, Methods in Enzymology, *101*, 20 ~ 78 (1983)] replicative form (RF) DNA was mixed with a human bFGF DNA fragment derived from pTB669, previously digested with EcoR I and BamH I. The mixture was then subjected to ligation using T4 DNA ligase. The resulting ligated DNA was transformed into infectable cells of the strain *Escherichia coli* JM105; the transformant cells were spread over a plate whose indicator species was Xgal [J. Messing et al., Nucleic Acids Research, *9*, 309-321 (1981)]; the plaque containing the recombinant phage (white plaque) was picked up; the base sequence of the recombinated region was determined by the dideoxynucleotide synthesis chain termination method [J. Messing et al., Nucleic Acids Research, *9*, 309 (1981)], whereby it was confirmed that the human bFGF DNA had been accurately inserted.

From this M13-PO clone was purified a single-stranded phage DNA, which was used as a template for site-directed mutagenesis using a synthetic oligonucleotide.

(2) Site-specific mutagenesis:

In the presence of 0.1 mM adenosine triphosphate (ATP), 50 mM hydroxymethylaminomethane hydrochloride (Tris-HCl) having a pH-value of 8.0, 10 mM $MgCl_2$, 5 mM dithiothreitol (DTT) and 9 units of T4 kinase, in a total amount of 50 µℓ, 40 picomoles of the synthetic oligonucleotide:
5'-CGGGCATGAATTCGCCGCT-3'
[primer for producing in the base sequence a recognition site for the restriction enzyme EcoR I, and subsequently changing *Pro*-14 to *Met* (see Figure 38 (2))] was treated with T4 kinase at 37 °C for 1 hour. This kinase-treated primer (12 picomoles) was heated at 67 °C for 5 minutes, and at 42 °C for 25 minutes, in 50 µℓ of a mixture containing 50 mM NaCl, 1.0 mM Tris-HCl having a pH-value of 8.0, 10 mM $MgCl_2$ and 10 mM β-mercaptoethanol, whereby it was hybridized to 5 µg of the single-stranded (ss) M13-PO DNA. The annealed mixture was then cooled on ice, and was added to 50 µℓ of a reaction mixture containing 0.5 mM deoxynucleotide triphosphate (dNTP), 80 mM Tris-HCl having a pH-value of 7.4, 8 mM $MgCl_2$, 100 mM NaCl, 9 units of DNA polymerase I Klenow fragment, 0.5 mM ATP and 2 units of T4 DNA ligase, and reaction was carried out at 37 °C for 3 hours, and at 25 °C for 2 hours, whereafter the reaction was stopped by adding 2 µℓ of 0.2 mM EDTA. The reaction product was used to transform infectable JM 105 cells; the transformant cells were allowed to grow overnight, whereafter an ssDNA was isolated from the culture medium supernatant. Using this ssDNA as a template for the 2nd cycle of primer elongation, gel-purified RF-type DNA was transformed into infectable JM 105 cells; the resulting transformant cells were spread over an agar plate, and were cultured overnight to obtain phage plaques.

(3) Site-directed mutagenesis:

The procedure of the above term (2) was repeated but the used synthetic oligonucleotide primer was: 5'-CGCCCATGGTGCCATCCTC-3' which produces in the base sequence a recognition site for the restriction enzyme Nco I, and concurrently changes *Gly*-9 to *Thr* and *Ser*-10 to *Met*, *respectively*. [See Figure 38 (1).]

(4) Site-directed mutagenesis:

The procedure of the above term (2) was repeated but the used synthetic oligonucleotide primer was: 5'-TAACACCTTAAGAAGCCAG-3' which produces in the base sequence a recognition site for the restriction enzyme Af1 II, and concurrently changes the *Lys*-87-encoding codon to a termination codon. [See Figure 38 (3).]

(5) Site-directed mutagenesis:

The procedure of the above term (2) was repeated but the used synthetic oligonucleotide primer was: 5'-CCGGACTCCGTTAACTCGG-3' which produces in the base sequence a recognition site for the restriction enzyme Hpa I, and concurrently changes *Asp*-42 to *Asn*. [See Figure 38 (4).]

(6) Site-directed mutagenesis:

The procedure of the above term (2) was repeated but the used synthetic oligonucleotide primer was: 5'-CTTCTCCTGGACTCCGTCAAC-3' which deletes the recognition site for the restriction enzyme Hpa II in the base sequence, and concurrently changes *Arg*-45 to *Gln*. [See Figure 38 (5).]

(7) Screening and identification of plaques which were mutagenic:

Plates containing mutated M13-PO plaques [above term (1)] and 2 plates containing unmutated M13-PO phage plaques were cooled to 4 ° C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl having a pH-value of 7.5 and 1.5M NaCl. The filters were twice washed on filters immersed in 2 × SSC (Standard Sodium Citrate) in the same manner, and were allowed to dry, and this was followed by drying at 80 ° C for 2 hours in a vacuum oven. The overlapped filters were subjected to prehybridization at 55 ° C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50 mM sodium phosphate-buffered solution having a pH-value of 7.0 and 100 μg/mℓ denatured salmon sperm DNA. Hybridization was carried out at 42 ° C for 24 hours with $10^5$ cpm/mℓ of an oligonucleotide primer. Each filter was washed at 50 ° C for 30 minutes in a buffer solution for washing containing 0.1% SDS and a reduced amount of SSC. The filters were then first washed with a buffer solution containing 2 × SSC; the control filters, which contained unmutated M13-PO plaques, were examined for radioactivity using a Geiger counter. While stepwise reducing SSC concentration, the control filters, which contained unmutated M13-PO plaques, were washed until no detectable radioactivity remained on the filters. The minimum of the used SSC concentrations was 0.1 × SSC. The filters were allowed to dry in air, and autoradiographs were taken by 2 to 3 days of exposure at -70 ° C. Screening was carried out of 10,000 mutated M13-PO plaques and 100 unmutated control plaques by means of an oligonucleotide probe treated with $^{32}$P-γ-ATP. None of the control plaques hybridized to the probe, while 3 to 10 of the mutated M13-PO plaques hybridized to the probe.

One of the mutated M13-PO plaques was taken, and was inoculated to a JM105 culture medium. From the resulting supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the GGC (*Gly*-9) codon had been changed to an ACC (*Thr*) codon and the AGC (*Ser*-10) codon had been changed to an ATG (*Met*) codon; the CCG (*Pro*-14) codon, to an ATG (*Met*) codon; the AAA (*Lys*-87) codon, to a TAA (termination) codon; the GAC (*Asp*-42) codon, to an AAC (*Asn*-42) codon; and the CGG (*Arg*-45) codon, to a CAG (*Gln*-45) codon.

Of the mutated M13-PO phages, the phage in which *Gly*-9 codon had become a *Thr*-encoding codon and the *Ser*-10 codon had become a *Met*−encoding codon was named M13-PN10 [Its base sequence and the amino acid sequence encoded thereby are shown in Figure 39.];
the phage in which the *Pro*-14 codon had become a *Met*-encoding codon, M13-PN14 [Its base sequence and the amino acid sequence encoded thereby are shown in Figure 40];
the phage in which the *Lys*-87 codon had become a termination codon, M13-PC86 [Its base sequence and the amino acid sequence encoded thereby are shown in Figure 41.]:
the phage in which the *Asp*-42 codon had become an *Asn*-encoding codon, M13-PDN42 [Its base sequence and the amino acid sequence encoded thereby are shown in Figure 42.]; and,
the phage in which the *Arg*-45 codon had become a *Gln*-encoding codon, M13-PRQ45 [Its base sequence and the amino acid sequence encoded thereby are shown in Figure 43.].

Example 28

(Production of recombinant DNAs having mutein-encoding base sequence)

(1) Site-specific mutagenesis:

In the presence of 0.1 mM adenosine triphosphate (ATP), 50 mM hydorxymethylaminomethane hydrochloride (Tris-HCl) having a pH-value of 8.0, 10 mM $MgCl_2$, 5 mM dithiothreitol (DTT) and 9 units of T4 kinase, in a total amount of 50 μℓ, 40 picomoles of the synthetic oligonucleotide:
5'-CGGACTCCTCTAACTCGGC-3'
[primer for deleting the recognition site for the restriction enzyme Hpa I in the base sequence, and subsequently changing *Asn*-42 to *Arg* (see Figure 38 (6)] was treated with T4 kinase at 37°C for 1 hour. This kinase-treated primer was heated at 67°C for 5 minutes, and at 42 °C for 25 minutes, in 50 μℓ of a mixutre containing 50 mM NaCl, 1.0 mM Tris-HCl having a pH-value of 8.0, 10 mM $MgCl_2$ and 10 mM β-mercaptoethanol, whereby it was hybridized to 5 μg of the single-stranded (ss) M3-PDN 42 DNA. The annealed mixture was then cooled on ice, and was added to 50 μℓ of a reaction mixture containing 0.5 mM deoxynucleotide triphosphate (dNTP), 80 mM Tris-HCl having a pH-value of 7.4, 8 mM $MgCl_2$, 100 mM NaCl, 9 units of DNA polymerase I Klenow fragment, 0.5 mM ATP and 2 units of T4 DNA ligase, and reaction was carried out at 37°C for 3 hours, and at 25°C for 2 hours, whereafter the reaction was stopped by adding 2 μℓ of EDTA. The reaction product was used to transform infectable JM 105 cells; the transformant cells were allowed to grow overnight, whereafter an ssDNA was isolated from the culture medium supernatant. Using this ssDNA as a template for the 2nd cycle of primer elongation, gel-purified RF-type DNA was transformed into infectable JM 105 cells; the resulting transformant cells were spread over an agar plate, and were cultured overnight to obtain phage plaques.

(2) Screening and identification of plaques made mutagenic:

Plates containing M13-PDN42 phage plaques mutated by the method of Example 26 using a synthetic oligomer [Figure 38 (6)] and 2 plates containing unmutated M13-PDN42 phage plaques obtained in Example 26 were cooled to 4°C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl having a pH-value of 7.5 and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridisation at 55°C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50 mM sodium phosphate-buffered solution having a pH-value of 7.0 and 100 μg/mℓ denatured salmon sperm DNA. Hybridization was carried out at 42°C for 24 hours with $10^5$ cpm/mℓ of an oligonucleotide primer. Each filter was washed in a buffer solution containing 0.1% SDS and a reduced amount of SSC at 50°C for 30 minutes. The filters were then first washed with a buffer solution containing 2 × SSC; the control filters, which contained unmutated M13-PDN42 plaques were examined for radioactivity using a Geiger counter. While stepwise reducing SSC concentration, the control filters, which contained unmutated M13-PDN42 plaques, were washed until no detectable radioactivity remained on the filters. The minimum of the used SSC concentrations was 0.1 × SSC. The filters were allowed to dry in air, and autoradiographs were taken by 2 to 3 days of exposure at -70°C. Screening was carried out of 10,000 mutated M13-PDN42 plaques and 100 unmutated control plaques by means of an oligonucleotide probe treated with kinase in the presence of $^{32}P$-γ-ATP. None of the control plaques hybridized to the probe, while 3 to 10 of the mutated M13-PDN42 plaques hybridized to the probe.

One of the mutated M13-PDN42 plaques was taken, and was inoculated to a JM105 culture medium. From the resulting supernatant an ssDNA was prepared, and from the bacterial cell pellets a double-stranded (ds)DNA was prepared. Analyses were made of the base sequence using an appropriate oligonucleotide primer and ssDNA.

As a result, it was confirmed that the AAC (*Asn*-42) codon had been changed to an AGA (*Arg*) codon.

Of the mutated M13-PDN42 phages, the phage in which the *Asn*-42 codon had become an *Arg*-encoding codon was named M13-PNR42. Its base sequence and the amino acid sequence encoded thereby are shown in Figure 44.

Example 29

(Stabilities of the Muteins CS2, CS3 and CS23)

Examinations were made of the stabilities under acid conditions of human bFGF purified by heparin HPLC and the muteins CS2, CS3 and CS23 (those obtained in Example 24) as follows: The muteins and human bFGF, in solution in 50mM Na-acetate (pH 4.0) adjusted to 1 µg/mℓ concentration, were separately incubated at 37°C. Incubation time was varied between 0 and 1 hour at intervals of 10 minutes, and activities were determined for respective incubation times using the mouse BALB/c3T3 cell system (mentioned above). Activities for respective incubation times were calculated in percent ratio based on the activities of non-incubated standard samples, taken as 100%, and were plotted to obtain the graphs shown in Figure 45. In Figure 45, -●-, -▲-, -■- and -○ - respectively indicate the results from human bFGF, the mutein CS2, the mutein CS3 and the mutein CS23. Human bFGF almost completely inactivated itself within 10 minutes, while the muteins remained active for longer periods in the ascending order of CS2, CS3 and CS23. The mutein CS23 maintained about 50% activity even 20 minutes later. As a result, these muteins proved to have greater stability than that of human bFGF.

Example 30

(Human umbilical cord vascular endothelial cell proliferation promoting activities of muteins)

Determinations were made of the vascular endothelial cell proliferation promoting activities of human bFGF purified by heparin HPLC and the muteins CS2, CS3 and CS23 (those obtained in Example 24). The procedure used for the determinations is as follows: Human vascular endothelial cells were obtained from human umbilical cord vein by perfusion using a trypsin solution, and were maintained by subculture using liquid medium prepared by adding 2.5% fetal bovine serum and 2.0 ng/mℓ human bFGF to GIT medium (produced by Daigo Eiyo Kagaku K.K., Japan). To a 96-well cultivation plate (Nunc, 1-67008) 100 µℓ of a suspension of 2 × $10^3$ human vascular endothelial cells was seeded, and this was followed by cultivation in a constant temperature chamber filled with carbon dioxide. Next day, FGF in such amount that final concentration was 2 ng/mℓ and 100 µℓ of a medium containing samples at various concentrations were added. After 3 days of cultivation, the medium containing the sample was aspirated, and 100 µℓ of a 1 mg/1 mℓ MTT solution (prepared by dissolving 3-(4,5-dimethyl-2-thiazolyl)-2,5-diphenyl-2H-tetrazolium bromide in the medium) was added, and this was followed by incubation for 4 hours. 100 µℓ of a 10% SDS solution (aqueous solution of sodium dodecyl sulfate) was then added, and incubation was carried out for 5 to 6 hours to solubilize the cells and MTT pigment, whereafter $OD_{590}$ value was determined by means of a spectrophotometer. Calculations were made of the cell proliferation rates for respective sample concentrations in percent ratio based on the maximum proliferation rate obtained with 8.0 ng/mℓ human bFGF, taken as 100%, and determinations were made of the specific activities by comparing the concentrations which resulted in 50% proliferation rate with the concentration of standard FGF sample. The specific activities thus determined are shown in Table 5.

Table 5

| FGF | Specific Activity |
|---|---|
| CS2 | 1.6 |
| CS3 | 3.0 |
| CS23 | 2.5 |
| Human bFGF | 1.0 |

Based on these results, it was recognized that the muteins are higher in specific activity than human bFGF.

Example 31

(Angioneogenesis potencies of muteins, determined by the CAM method)

Investigations were made of the angioneogenesis potencies *in vivo* of human bFGF purified by heparin HPLC and the muteins CS2, CS3 and CS23 (those obtained in Example 24). The procedure was by CAM (Chorio-allantoic membrane) assay. A slightly modified technique of Auerbach's method [Developmental Biology, *41*, 391 (1974)] was used for CAM assay. After 3 days of incubation at 37°C in an incubator, fertilized fowl eggs had their shells removed, and were further incubated at 37°C for 1 more week using a Napco carbon dioxide incubator 6300 ($CO_2$:0%, $H_2O$:saturated). An aqueous solution of each protein was spotted over polypropylene disks having a diameter of 6 mm so that each protein was present in amounts of 6.25, 12.5, 25.0 and 50.0 ng. After air-drying in clean bench, the disks were incubated at 37°C for 3 more days while kept standing on fowl chorio-allantoic membrane, and observations were made of angiogenesis states by means of a stereoscopic microscope. The obtained results are shown in Table 6. NB: A total of 18 eggs for each sample were used, and the figures in the table represent in percent ratio the numbers of eggs in which angiogenesis was noted.

Table 6

| Angiogenesis Potency (%) | | | | |
|---|---|---|---|---|
| FGF | 50.0 | 25.0 | 12.5 | 6.25 ng |
| CS2 | 77.8 | 44.4 | 22.2 | 0.0 |
| CS3 | 72.2 | 61.1 | 16.7 | 0.0 |
| CS23 | 72.2 | 55.6 | 22.2 | 0.0 |
| Human bFGF | 94.4 | 50.0 | 33.3 | 0.0 |
| Carrier | 11.1 | 5.6 | 11.1 | 0.0 |

From Table 6, it is obvious that the bFGF muteins possess angiogenesis potencies which are nearly equivalent to that of the control human bFGF.

Example 32

(Expression in *Escherichia coli* of gene which encodes human bFGF mutein)

(1) Construction of the plasmid pTB854 for human bFGF mutein expression:

The M13-PN10 replicative form (RF) obtained in Example 27 above was treated in the manner described in Example 2 (1) to construct the plasmid pTB854 for human bFGF mutein (Figure 46).

Using this plasmid pTB854, *Escherichia coli* MM294 was transformed, whereby the strain *Escherichia coli* MM294/pTB854 was obtained, which harbors the plasmid pTB854 containing the mutein-encoding gene shown in Figure 39.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB854 exhibited FGF activity.

The mutein TM910, in which Gly at the 9-position and Ser at the 10-position of human bFGF had been replaced by Thr and Met, respectively, was thus obtained.

Example 33

(Expression in *Escherichia coli* of gene which encodes human bFGF mutein)

(1) Construction of plasmid pTB852 for human bFGF mutein expression:

The DNA of the plasmid pTB854 which was obtained in the above Example 32 was cleaved with restriction enzymes EcoRI and NcoI so as to remove the DNA fragment which encodes the 10 amino acid residues of N terminus of the human bFGF. The single stranded portion of the remaining DNA fragment was sealed to blunt end by the use of *Escherichia coli* DNA polimerase I in the presence of dATP, dCTP, dGTP, dTTP, and was ligated by employing T4DNA ligation reaction to construct the plasmid pTB852 for human bFGF mutein (Figure 47).

Using this plasmid 852, *Escherichia coli* MM294 was transformed, whereby the strain Escherichia coli MM294/pTB852, which harboring the plasmid pTB852 having the mutein-encoding gene shown in Figure 48.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB852 exhibited FGF activity. The mutein N10, in which the amino acid sequence of from Pro at the 2-position to Ser at the 10-position of human bFGF had been deleted, was thus obtained.

Example 34

(Expression in *Escherichia coli* of gene which encodes human bFGF mutein)

(1) Construction of the plasmid pTB795 for human bFGF mutein expression:

The M13-PN14 replicative form (RF) obtained in Example 27 above was treated in the manner described in Example 2 (1) to construct the plasmid pTB795 for human bFGF mutein (Figure 49).

Using this plasmid pTB795, *Escherichia coli* MM294 was transformed, whereby the strain *Escherichia coli* MM294/pTB795 (IFO 14700, FERM BP-1660) was obtained, which contains the plasmid pTB795 containing the mutein-encoding gene shown in Figure 40.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB795 exhibited FGF activity. The mutein N14, in which the amino acid sequence of from Pro at the 2-position to Pro at the 14-position of human bFGF had been deleted, was thus obtained.

Example 35

(Expression in *Escherichia coli* of gene which encodes human bFGF mutein)

(1) Construction of the plasmid pTB796 for human bFGF mutein expression:

The M13-PC86 replicative form (RF) obtained in Example 27 above was treated in the manner described in Example 2 (1) to construct the plasmid pTB796 for human bFGF mutein (Figure 50).

Using this plasmid pTB796, *Escherichia coli* MM294 was transformed, whereby the strain *Escherichia coli* MM294/pTB796 (IFO 14701, FERM BP-1661) was obtained, which contains the plasmid pTB796 containing the mutein-encoding gene shown in Figure 41.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

The cell extract obtained in the above (2) was subjected to an enzyme immunoassay (EIA), sandwich method, using monoclonal antibody as shown below (a) to (j).

As a result, it is confirmed that bFGF mutein C86 is present in the cell extract. The mutein C86, in which the amino acid sequence of from Lys at the 87-position to Ser at the 147-position had been deleted, was thus obtained.

Said EIA using monoclonal antibody was carried out by the following method:

(a) Immunization:

BALB/c mice (male, 4-week old) had the antigen human bFGF (as obtained in Reference Example 2) in solution in 0.4 mℓ of Freund's complete adjuvant (Difco Laboratories, USA) injected intraperitoneally. Three weeks later, 10 µg of the antigen hbFGF in solution in 0.4 mℓ of Freund's incomplete adjuvant was intraperitoneally administered. Further 3 weeks later, the same additional immunization was carried out, and, two weeks later, 10 µg of human bFGF in solution in physiological saline was intraperitoneally inoculated.

(b) Cell fusion:

From the immunized mice mentioned in said (a), the spleen was excised 4 days after final antigen challenge to thereby obtain cells to be used for cell fusion. These cells were suspended in a medium prepared by mixing together Isokov medium and Ham F-12 medium in a ratio of 1:1 (hereinafter referred to as IH medium).

The mouse myeloma cell P3-X63-Ag 8UI was subcultured in RPMI 1640 medium containing 10% fetal bovine serum under an atmosphere of 5% carbon dioxide and 95% air.

Cell fusion was conducted in accordance with the method established by Köhler and Milstein [Köhler, G. and Milstein, C.: Nature, *256*, 495 (1975)]. $2.9 \times 10^7$ cells of the above myeloma cell line and $1.5 \times 10^8$ immunized lymphocytes obtained by the above-mentioned method were mixed together and centrifuged, and 45% polyethylene glycol 6000 (hereinafter referred to as PEG 6000) in solution in 0.3 mℓ of IH medium was dropwise added. The PEG 6000 solution was preheated to 37°C, and was gradually added. Five minutes later, the 37°C-preheated IH medium was added at a rate of 0.5 mℓ per minute to make 10 mℓ. The solution was then centrifuged at room temperature at 600 rpm for 15 minutes, and the supernatant was removed. This cell precipitate was suspended in 200 mℓ of IH medium containing 20% calf serum, and this suspension was transferred to a 24-well microplate (Linbro) in an amount of 2 mℓ per well. One day later, IH medium (containing 20% calf serum) supplemented with HAT ($1 \times 10^{-4}$ M hipoxanthine, $4 \times 10^{-7}$ M aminopterin, $1.6 \times 10^{-5}$ M thymidine) (hereinafter

referred to as HAT medium) was added to the microplate in an amount of 1 mℓ per well, and, further three days later, one half amount of the medium was replaced with HAT medium. The cells thus grown are hybrid cells.

(c) Search for antibody-producing cells:

Previously, the hybrid cell culture supernatant was added in an amount of 100 μℓ per well to a 96-well microtiter plate made of polystyrene which had had human bFGF immobilized thereto, and incubation was carried out at room temperature for 2 hours. The incubation supernatant was removed, and, after washing, the horse radish peroxidase (HRP)-labeled anti-mouse IgG goat antibody (Miles Laboratories) was added as the secondary antibody, and incubation was carried out at room temperature for 2 hours. The secondary antibody was removed, and, after thoroughly washing the wells, coloring reaction was carried out in the presence of added reaction substrate (EIA method). By this method potent valency was observed in 3 wells.

(d) Cloning of hybrid cells:

Cells in each of these three cells were sown to 0.5 cell per well to a 96-well microtiter plate which had had $10^4$ cells/well mouse thymocytes as vegetative cells sown thereon, and cloning was carried out. As a result, three clones, namely the mouse hybridoma HbF52 (IFO 50143) and the mouse hybridoma HbF78 (IFO 50144) were obtained.

(e) Ascitization of hybrid cells:

$2 \times 10^6$ cells of each of the mouse hybridoma HbF52 or the mouse hybridoma HbF78 was inoculated to mice which had had mineral oil previously administered intraperitoneally. Ten days later, ascites in an amount of 2 mℓ was collected from each mouse, whereby the monoclonal antibody MoAb52 and the monoclonal antibody MoAb78 were respectively obtained.

(f) Thus obtained monoclonal antibody MoAb 78 was subjected to purification from ascites fluid in accordance with the method of Stachelin et al. The Journal of Biological Chemistry, ***256,*** 9750 (1981). Thus obtained antibody was concentrated to more than 2 mg/mℓ, and subjected to dialysis in 0.2M phosphate buffer (pH 7.0). To thus obtained 1.4 mℓ solution of MoAb 78 (concentration 2.8 mg/mℓ), 50 μℓ of S-acetylmercaptosuccinic anhydride (Aldrich Co., U.S.A) dissolved in N,N'-dimethylformamide was added so as to be the concentration of 11.5 mg/mℓ. The air in the reaction vessel is replaced by nitrogen gas. The vessel was sealed, and subjected to stirring so as to cause the reaction of introducing SH group. The unreacted S-acetylmercaptosuccinic acid anhydride was inactivated by the treatment for 10 minutes with 130 μℓ of 0.2M Tris•HCl (pH 7.0), 13 μℓ of 0.2M EDTA and 130 μℓ of 2M hydroxyamine (pH 7.0). The MoAb 78 was recovered by gel filtration using a column packed with Sephadex G-25® (diameter 1 cm × 80 cm, Farmacia, Sweden) (flow rate: 20 mℓ/hour).

(g) 10 mg of horse radish peroxidase (HRP, Behringer Manheim, Grade I, West Germany) was dissolved in 1.4 mℓ of 0.1M phosphate buffer (pH 6.8). On the other hand, 14 mg of N-hydroxysuccinimide ester of N-(4-corboxy cyclohexyl methyl) maleimide was dissolved in 335 μℓ of DMF, and 100 μℓ of thus obtained solution was added to the HRP solution above mentioned. The air in the reaction vessel was replaced by nitrogen gas, and the vessel was sealed. After 1 hour reaction at room temperature, proteins of the portion of the monoclonal MoAb 78 introduced with SH group were recovered by gel filtration using a colomn packed with Sephadex G-25 as in the above (f).

(h) 6 mℓ of the portion of the monoclonal antibody MoAb 78 introduced with SH group obtained in the above (f) and 2 mℓ of the portion of HRP introduced with maleimide group obtained in the above (g) were mixed, and the mixture was concentrated to 1 mℓ using collodion bag (Sartorius, West Germany) under reduced pressure at 4°C for 20 hours. After the reaction, the unreacted HRP introduced with SH group was removed with the use of Ultrogel AcA44 (LKB Co., diameter 1 cm × 80 cm, Sweden) column (flow rate: 10 mℓ/hour). In the eluates, the fraction containing 2.4 HRP/antibody has the most high HRP number per antibody molecule. Thus obtained was employed in the EIA in the following item (i).

(i) The monooclonal antibody MoAb 52 obtained in the above (e) was purified by the manner described in the above (f). The monoclonal antibody MoAb 52 was diluted with PBS so as to be 10 μg/mℓ or 20 μg/mℓ, and the diluent was poured into Immunoplate (Nunc. Denmark) so as to be 100 μℓ/well. The plate was kept standing at 4°C overnight to adsorbe the monoclonal antibody MoAb 52 to the plate. After removing the antibody which is not adsorbed, the plate was washed with PBS thrice, PBS containing 0.01% merthiolate and 1% bovine serum albumin (BSA) was added to the plate at 200 μℓ/well, and the plate was kept standing at 4°C overnight.

(j) The cell extract containing bFGF mutein C86 obtained in the above (2) was diluted with PBS containing 0.1% BSA. From the plate obtained in the above (i), BSA solution was removed, the plate was washed with PBS four times, and the diluted bFGF mutein C86 was added to the plate so as to be 100 μℓ/well to adsorbe to the plate at 4°C overnight. The unreacted mutein C86 was removed, and the plate was washed with PBS four times. The monoclonal antibody conjugated with HRP obtained in the above (h) was diluted with PBS containing 0.1% BSA to 1/300, and the diluent was added to the plate so as to be 100 $\mu\ell$/well. The reaction was carried out for 4 hours at room temperature. After removing the antibody, the plate was washed with PBS for 6 times, substrate for oxidase (Bio. Rad Co. U.S.A) was added to the plate so as to be 100 $\mu\ell$/well. Quantification was accomplished by absorbance measurements at 415 nm, and it was confirmed that a small amount of the mutein C86 was produced.

Example 36

(Expression in *Escherichia coli* of gene which encodes human bFGF mutein)

(1) Construction of the plasmid pTB797 for human bFGF mutein expression:

The M13-PDN42 replicative form (RF) obtained in Example 27 above was treated in the manner described in Example 2 (1) to construct the plasmid pTB797 for human bFGF mutein (Figure 51).

Using this plasmid pTB797, *Escherichia coli* MM294 was transformed, whereby the strain *Escherichia coli* MM294/pTB797 (IFO 14702, FERM BP-1662) was obtained, which harbors the plasmid pTB797 containing the mutein-encoding gene shown in Figure 42.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB797 exhibited FGF activity. The mutein DN42, in which Asp at the 42-position of human bFGF had been replaced by Asn, was thus obtained.

Example 37

(Expression in *Escherichia coli* of gene which encodes human bFGF mutein)

(1) Construction of the plasmid pTB799 for human bFGF mutein expression:

The M13-PRQ45 replicative form (RF) obtained in Example 27 above was treated in the same manner as in Example 2 (1) to construct the plasmid pTB799 for human bFGF mutein (Figure 52).

Using this plasmid pTB799, *Escherichia coli* MM294 was transformed, whereby the strain *Escherichia coli* MM294/pTB799 was obtained, which harbors the plasmid pTB799 containing the mutein-encoding gene shown in Figure 43.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB799 exhibited FGF activity. The mutein RQ45, in which Arg at the 45-position of human bFGF had been replaced by Gln, was thus obtained.

Example 38

(Expression in *Escherichia coli* of gene which encodes human bFGF mutein)

(1) Construction of the plasmid pTB798 for human bFGF mutein expression:

The M13-PNR42 replicative form (RF) obtained in Example 28 above was treated in the manner described in Example 2 (1) to construct the plasmid pTB798 for human bFGF mutein (Figure 53).

Using this plasmid pTB798, *Escherichia coli* MM294 was transformed, whereby the strain *Escherichia coli* MM294/pTB798 was obtained, which harbors the plasmid pTB798 containing the mutein-encoding gene shown in Figure 44.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB798 exhibited FGF activity. The mutein NR42, in which Asp at the 42-position of human bFGF had been replaced by Arg, was thus obtained.

Example 39

(Production of recombinant DNAs having mutein-encoding base sequence)

(1) Site-specific mutagenesis:

In the presence of 0.1 mM adenosine triphosphate (ATP), 50 mM hydorxymethylaminomethane hydrochloride (Tris-HCl) having a pH-value of 8.0, 10 mM $MgCl_2$, 5 mM dithiothreitol (DTT) and 9 units of T4 kinase, in a total amount of 50 $\mu\ell$, 40 picomoles of the synthetic oligonucleotide:
5'-TCTTCTCCAGGGATCCGTT-3'
[primer for adding the recognition site for the restriction enzyme BamHI in the base sequence and subsequently changing Val-44 to Ser and Arg 45 to Leu (see Figure 38 (7)] was treated with T4 kinase at 37°C for 1 hour. This kinase-treated primer was heated at 67°C for 5 minutes, and at 42 °C for 25 minutes, in 50 $\mu\ell$ of a mixture containing 50 mM NaCl, 1.0 mM Tris-HCl having a pH-value of 8.0, 10 mM $MgCl_2$ and 10 mM $\beta$-mercaptoethanol, whereby it was hybridized to 5 $\mu$g of the single-stranded (ss) M3-PDN 42 DNA. The annealed mixture was then cooled on ice, and was added to 50 $\mu\ell$ of a reaction mixture containing 0.5 mM deoxynucleotide triphosphate (dNTP), 80 mM Tris-HCl having a pH-value of 7.4, 8 mM $MgCl_2$, 100 mM NaCl, 9 units of DNA polymerase I Klenow fragment, 0.5 mM ATP and 2 units of T4 DNA ligase, and reaction was carried out at 37°C for 3 hours, and at 25°C for 2 hours, whereafter the reaction was stopped by adding 2 $\mu\ell$ of EDTA. The reaction product was used to transform infectable JM 105 cells; the transformant cells were allowed to grow overnight, whereafter an ssDNA was isolated from the culture medium supernatant. Using this ssDNA as a template for the 2nd cycle of primer elongation, gel-purified RF-type DNA was transformed into infectable JM 105 cells; the resulting transformant cells were spread over an agar plate, and were cultured overnight to obtain phage plaques.

(2) Screening and identification of plaques made mutagenic:

Plates containing M13-PDN42 phage plaques mutated by the method of Example 27 using said synthetic oligomer (oligonucleotide) [Figure 38 (7)] and 2 plates containing unmutated M13-PDN42 phage plaques obtained in Example 26 were cooled to 4°C, and the plaques from each plate were transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100®, after which they were neutralized by keeping them placed for 5 more minutes on filter papers immersed in 0.5M Tris-HCl having a pH-value of 7.5 and 1.5M NaCl. The filters were twice washed on filters immersed in 2 × SSC (standard sodium citrate) in the same manner, and were allowed to dry, and this was followed by drying at 80 °C for 2 hours in a vacuum oven. The overlapped filters were subjected to prehybridization at 55 °C for 4 hours with 10 mℓ/filter of a DNA hybridization buffer solution (5 × SSC) having a pH-value of 7.0 containing 4 × Denhardt's solution (polyvinylpyrrolidone, Ficoll® and bovine serum albumin, 1 × = 0.02%), 0.1% sodium dodecyl sulfate (SDS), 50 mM sodium phosphate-buffered solution having a pH-value of 7.0 and 100 µg/mℓ denatured salmon sperm DNA. Hybridization was carried out at 42 °C for 24 hours with $10^5$ cpm/mℓ of an oligonucleotide primer. Each filter was washed in a buffer solution containing 0.1% SDS and a reduced amount of SSC at 50 °C for 30 minutes. The filters were then first washed with a buffer solution containing 2 × SSC; the control filters, which contained unmutated M13-PDN42 plaques were examined for radioactivity using a Geiger counter. While stepwise reducing SSC concentration, the control filters, which contained unmutated M13-PDN42 plaques, were washed until no detectable radioactivity remained on the filters. The minimum of the used SSC concentrations was 0.1 × SSC. The filters were allowed to dry in air, and autoradiographs were taken by 2 to 3 days of exposure at -70 °C. Screening was carried out of 10,000 mutated M13-PDN42 plaques and 100 unmutated control plaques by means of an oligonucleotide probe treated with kinase in the presence of $^{32}$P-γ-ATP. None of the control plaques hybridized to the probe, while 3 to 10 of the mutated M13-PDN42 plaques hybridized to the probe.

One of the mutated M13-PDN42 plaques was taken, and was inoculated to a JM105 culture medium. From the resulting supernatant an ssDNA was prepared, and from the bacterial cell pellets a double-stranded (ds)DNA was prepared. Analyses were made of the base sequence using an appropriate oligonucleotide primer and ssDNA.

As a result, it was confirmed that the GTC (Val-44) codon and CGG (Arg-45) codon had been changed to TCC (Ser) codon and CTG (Leu) codon, respectively.

Of the mutated M13-PDN42 phages, the phage in which Val-44 codon and Arg-45 codon had become Ser-encoding codon and Leu-encoding codon, respectively was named M13-PINT. Its base sequence and the amino acid sequence encoded thereby are shown in Figure 54.

(3) Construction of the plasmid pTB853 for human bFGF mutein expression:

The M13-PINT replicative form (RF) obtained in the above (2) was treated in the manner described in Example 2 (1) to construct the plasmid pTB853 for human bFGF mutein (Figure 55).

Using this plasmid pTB853, *Escherichia coli* MM294 was transformed, whereby the strain *Escherichia coli* MM294/pTB853 was obtained, which contains the plasmid pTB853 containing the mutein-encoding gene shown in Figure 54.

(4) Preparation of bacterial cell extract:

The above-mentioned transformant was cultured by the method described in Example 2 (2) to give a supernatant, which was then used as a bacterial cell extract.

(5) Human bFGF activity of the bacterial cell extract:

A determination was made of the human bFGF activity of the bacterial cell extract obtained in (4) above, by the method described in Example 2 (3).

As a result, the bacterial cell extract from E. coli MM294/pTB853 exhibited FGF activity. The mutein FINT, in which Asp at 42-position, Val at 44-position and Arg at 45-position or human bFGF had been replaced by Asn, Ser and Leu, respectively, was thus obtained.

Example 40

(Expression in *Escherichia coli* of gene which encodes human bFGF mutein)

(1) Construction of the plasmid pTB855 for human bFGF mutein expression:

The DNA of the plasmid pTB669 which was obtained in the above mentioned Reference Example 2 was cleaved with a restriction enzyme Hinc II, and it was ligated with EcoR I linker p(5' CATGAATTCATG 3') under T4 DNA ligase reaction. Thus obtained DNA was further cleaved with a restriction enzymes EcoR I and Pst I to recover a DNA fragment of about 0.35 kb. This DNA fragment was ligated with the about 3.2 kb DNA fragment obtained in Example 2 (1), the fragment being obtained by cleaving the plasmid ptrp781 with EcoR I-Pst I, to obtain the plasmid pTB855 for human bFGF mutein expression was constructed (Figure 56).

Using this plasmid 855, *Escherichia coli* MM294 was transformed, whereby the strain *Escherichia coli* MM294/pTB855, which contains the plasmid pTB855 having the mutein -encoding gene shown in Figure 57.

(2) Preparation of bacterial cell extract:

The above-mentioned transformant was cultured by the method described in Example 2 (2) to give a supernatant, which was then used as a bacterial cell extract.

(3) Human bFGF activity of the bacterial cell extract:

A determination was made of the human bFGF activity of the bacterial cell extract obtained in (2) above, by the method described in Example 2 (3).

As a result, the bacterial cell extract from E. coli MM294/pTB855 exhibited FGF activity. The mutein N41, in which the amino acid sequence of from Pro at the 2-position to Val at the 41-position of human bFGF had been deleted, was thus obtained.

Example 41

(Expression in *Escherichia coli* of gene which encodes human bFGF mutein)

(1) Construction of the plasmid pTB856 for human bFGF mutein expression:

The DNA of the plasmid pTB669 which was obtained in the above mentioned Reference Example 2 was partly cleaved with a restriction enzyme BamHI so as to obtain BamHI recognition site in the bFGF gene. The site was further cleaved with *Escherichia coli* DNA polymerase I in the presence of dATP, dCTP, dGTP, dTTP to give blunt end. This DNA is ligated with NheI linker p(5' CTAGCTAGCTAG 3') under T4 DNA ligase reaction. After treating with the restriction enzyme NheI and ligating the cleaved site under T4 DNA ligase reaction, the plasmid pTB856 for human bFGF mutein expression was constructed (Figure 58).

Using this plasmid pTB856, *Escherichia coli* MM294 was transformed, whereby the strain *Escherichia coli* MM294/pTB856 which contains the plasmid pTB856 having the mutein-encoding gene shown in Figure 59.

(2) Preparation of bacterial cell extract:

The above-mentioned transformant was cultured by the method as in Example 2 (2) to give a supernatant, which was then used as a bacterial cell extract.

(3) The cell extract obtained in the above (2) was subjected to an enzyme immunoassay (EIA), sandwich method, as the manner described in Example 35 (3). As a result, it is confirmed that bFGF mutein is present in the cell extract. The mutein C129, in which the amino acid sequence of from Lys at the 130-position to Ser at the 147-position had been deleted, was thus obtained.

Example 42

(Expression in animal cell of gene which encodes human bFGF mutein)

(1) Construction of the plasmids pTB831, pTB833 and pTB835 for human bFGF mutein expression:

The plasmid pTB762, which was obtained in Example 7 above mentioned, was cleaved with the restriction enzymes EcoR I-Bam HI to obtain a 0.38 kb DNA fragment containing a coding region which encodes human bFGF mutein CS23, in which Cys at the 70-position and at the 88-position had been replaced by Ser.

On the other hand, the plasmid pTB504 [Example 1 (ii) of Japanese Patent Laid-open No. 62-175182 which corresponds to European Patent Publication No. 225, 701] was cleaved with the restriction enzyme Cla I-EcoR I to obtain a 1.7 kb DNA fragment containing murine leukemia virus (MuLV) LTR region, SV40 early promoter, splice junction and human interleukin-2 (IL-2) leader sequence.

Furthermore, the plasmid pTB627, which was obtained in Reference Example 1, was cleaved with the restriction enzyme Cla I-EcoR I to give 2 kb DNA fragment, and the plasmid pTB389 was cleaved with the restriction enzyme Cla I-EcoR I to give 2.6 kb DNA fragment. Thus obtained 2 kb DNA fragment and 2.6 kb DNA fragment was ligated to give the plasmid pTB675, which encodes human bFGF. The above plasmid pTB389 was obtained by converting each of the PstI cleavage site at the 5'-terminal region and the BamHI cleavage site at the 3'-terminal region of the interleukin-2 gene region of plasmid pTB106 (disclosed in Japanese Patent Laid-open No. 61-63282 which corresponds to European Patent Publication No. 172, 619) to EcoRI, to remove the interleukin-2 gene region. Then, the plasmid pTB675 was cleaved with the restriction enzymes ClaI-BamHI to give 3.4 kb DNA fragment which contains a coding region for amino acids at C-terminus of human bFGF, a non-coding region, ampicilline resistant gene originated from plasmid pBR322, and replication origin in *Escherichia coli*.

The above three DNA fragments (i.e. 0.38 kb DNA fragment, 1.7 kb DNA fragment and 3.4 kb DNA fragment) were ligated by the use of T4 DNA ligase to give the plasmid pTB831 (Figure 60).

Similarly, the plasmid pTB763 obtained in Example 18, which encodes the human bFGF mutein CS14 (in which Cys at the 26- and 93-positions of human bFGF had been replaced by Ser.) or the plasmid pTB765 obtained in Example 11, which encodes the human bFGF mutein CS 1234 (in which Cys at the 26-, 70-, 88- and 93-positions of human bFGF had been replaced by Ser.) was used instead of the plasmid pTB762 in the above procedure, whereby the plasmid pTB833 and the plasmid pTB835, respectively, were constructed.

(2) Expression in animal cells:

Monkey COS7 cells were seeded into tissue culture dishes of 6 cm diameter with DMEM medium containing 10% fetal calf serum, and after 24 hours the culture medium was replaced by a new one. After 4 hours, 10 $\mu$g DNA of the plasmid pTB831, pTB833 or pTB835 was transfected to the COS7 cells in accordance with calcium phosphate method [Graham et al. Virology, *52*, 456 (1973)]. The next day the culture medium was replaced by DMEM medium containing 1% fetal calf serum. After 48 hours cultivation, cultured broth including cells was collected and cells were recovered. The cells were washed with PBS twice, and then suspended in PBS. The suspension was treated with ultrasonic treatment in a short time, and then subjected to centrifugation for 15 minutes at 15,000 rpm to give supernatant.

A determination of human bFGF activity was made on the cultured broth of COS7 cells infected with the plasmid and on the extract from the cells, by the method described in Example 2 (3). The results thus obtained are shown in Table 7.

Table 7

| Plasmid | Mutein | FGF Activity (ng/dish) | |
|---|---|---|---|
| | | Cultured broth | Extract from cells |
| pTB831 | CS23 | 115 | 420 |
| pTB833 | CS14 | 0.2 | 35 |
| pTB835 | CS1234 | 1.1 | 20 |
| - | | <0.02 | 1 |

## Claims

### Claims for the following Contracting States : AT, BE, CH, DE, FR, GB, GR, IT, LI, LU, NL, SE

**1.** A mutein of basic fibroblast growth factor (bFGF) which is a replacement type mutein or a combination of a replacement type and a deletion type mutein, wherein

a replacement type mutein is a mutein in which at least one of the four cystein residues in the constituent amino acid sequence of the natural type bFGF is replaced with one amino acid other than cysteine, and

a deletion type mutein is a mutein in which one to 41 amino acids are lacking from the amino-terminal of the constituent amino acid sequence of bFGF and/or one to 61 amino acids are lacking from the carboxyl-terminal of the constitutent amino acid sequence of bFGF.

2. A mutein as claimed in Claim 1, wherein the natural type bFGF includes the amino acid sequence:

Phe-Phe-Leu-Arg-Ile-His-Pro-Asp-Gly-Arg-Val-Asp-
Gly-Val-Arg-Glu-Lys-Ser-Asp-Pro

in its molecule.

3. A mutein as claimed in Claims 1 and 2, wherein the newly introduced amino acid is a neutral amino acid.

4. A mutein as claimed in Claim 3, wherein the neutral amino acid is selected from the group consisting of glycine, valine, alanine, leucine, isoleucine, tyrosine, phenylalanine, histidine, tryptophan, serine, threonine and methionine.

5. A mutein as claimed in Claim 4, wherein the neutral amino acid is serine or threonine.

6. A mutein as claimed in Claim 5, wherein the neutral amino acid is serine.

7. A mutein as claimed in claim 6 wherein the cystein residue at position 70 of human bFGF is replaced by serin.

8. A mutein as claimed in claim 6 wherein the cystein residue at position 88 of human bFGF is replaced by serin.

9. A mutein as claimed in claim 6 wherein the cystein residues at position 70 and at position 88 of human bFGF are replaced by serin.

10. A mutein as claimed in Claim 1, wherein at least one amino acid lacks from the amino terminal of the constituent amino acids of bFGF.

11. A mutein as claimed in Claim 1, wherein at least one amino acid lacks from the carboxyl terminal of the constituent amino acids of bFGF.

12. A recombinant DNA having a base sequence which codes for the mutein of basic fibroblast growth factor (bFGF) as defined in any of claims 1-11.

13. A recombinant DNA as claimed in claim 12, wherein the newly introduced amino acid is a neutral amino acid selected from the group consisting of glycine, valine, alanine, leucine, isoleucine, tyrosine, phenylalanine, histidine, tryptophane, serine, threonine and methionine.

14. A recombinant DNA as claimed in Claim 13, wherein the newly introduced amino acid is serine.

15. A recombinant DNA as claimed in claim 12, wherein at least one amino acid lacks from the amino terminal or the carboxyl terminal of the constituent amino acid of bFGF.

16. A transformant harbouring a vector containing a recombinant DNA having a base sequence which codes for the mutein of basic fibroblast growth factor (bFGF) as defined in any of claims 1-11.

17. A transformant as claimed in claim 16, wherein the newly introduced amino acid is a neutral amino acid selected from the group consisting of glycine, valine, alanine, leucine, isoleucine, tyrosine, phenylalanine, histidine, tryptophane, serine, threonine and methionine.

18. A transformant as claimed in Claim 17 wherein the newly introduced amino acid is serine.

19. A transformant as claimed in claim 16, wherein at least one amino acid lacks from the amino terminal or the carboxyl terminal of the constituent amino acid of bFGF.

**20.** A method for producing a mutein of basic fibroblast growth factor (bFGF), which comprises cultivating a transformant harbouring a vector containing a recombinant DNA having a base sequence which codes for the mutein of bFGF as defined in any of claims 1-11 in a culture medium to produce and accumulate the mutein in the culture broth.

**21.** A method as claimed in claim 20, wherein the newly introduced amino acid is a neutral amino acid selected from the group consisting of glycine, valine, alanine, leucine, isoleucine, tyrosine, phenylalanine, histidine, tryptophane, serine, threonine and methionine.

**22.** A method as claimed in Claim 21, wherein the newly introduced amino acid is serine.

**23.** A method as claimed in claim 20, wherein at least one amino acid lacks from the amino terminal or the carboxyl terminal of the constituent amino acid of bFGF.

**Claims for the following Contracting State : ES**

**1.** A method for producing a mutein of basic fibroblast growth factor (bFGF) which is a replacement type mutein or a combination of a replacement type and a deletion type mutein, wherein

a replacement type mutein is a mutein in which at least one of the four cystein residues in the constituent amino acid sequence of the natural type bFGF is replaced with one amino acid other than cysteine, and

a deletion type mutein is a mutein in which one to 41 amino acids are lacking from the amino-terminal of the constituent amino acid sequence of bFGF and/or one to 61 amino acids are lacking from the carboxyl-terminal of the constitutent amino acid sequence of bFGF, which comprises cultivating a transformant harboring a vector containing a recominant DNA having a base sequence which codes for said mutein of bFGF in a culture medium to produce and accumulate the mutein in the cultured broth.

**2.** A method as claimed in Claim 1, wherein the natural type bFGF includes the amino acid sequence:

Phe-Phe-Leu-Arg-Ile-His-Pro-Asp-Gly-Arg-Val-Asp-
Gly-Val-Arg-Glu-Lys-Ser-Asp-Pro

in its molecule.

**3.** A method as claimed in Claims 1 and 2, wherein the newly introduced amino acid is a neutral amino acid.

**4.** A method as claimed in Claim 3, wherein the neutral amino acid is selected from the group consisting of glycine, valine, alanine, leucine, isoleucine, tyrosine, phenylalanine, histidine, tryptophan, serine, threonine and methionine.

**5.** A method as claimed in Claim 4, wherein the neutral amino acid is serine or threonine.

**6.** A method as claimed in Claim 5, wherein the neutral amino acid is serine.

**7.** A method as claimed in claim 6, wherein the produced mutein is a mutein in which the cystein residue at position 70 of human bFGF is replaced by serin.

**8.** A method as claimed in claim 6, wherein the produced mutein is a mutein in which the cystein residue at position 88 of human bFGF is replaced by serin.

**9.** A method as claimed in claim 6, wherein the produced mutein is a mutein in which the cystein residues at position 70 and at position 88 of human bFGF are replaced by serin.

**10.** A method as claimed in Claim 1, wherein at least one amino acid lacks from the amino terminal of the constituent amino acids of bFGF.

11. A method as claimed in Claim 1, wherein at least one amino acid lacks from the carboxyl terminal of the constituent amino acids of bFGF.

**Patentansprüche**

**Patentansprüche für folgende Vertragsstaaten : AT, BE, CH, DE, FR, GB, GR, IT, LI, LU, NL, SE**

1. Mutein des basischen Fibroblasten-Wachstumsfaktors (bFGF), das ein Ersatztyp-Mutein oder eine Kombination eines Ersatztyp-Muteins und eines Auslassungstyp-Muteins ist, wobei
ein Ersatztyp-Mutein ein Mutein ist, in welchem wenigstens einer der vier Cysteinreste in der Sequenz der den Naturtyp-bFGF bildenden Aminosäuren durch eine andere Aminosäure als Cystein ersetzt ist, und
ein Auslassungstyp-Mutein ein Mutein ist, in welchem dem Aminoende der Sequenz der den Naturtyp-bFGF bildenden Aminosäuren eine bis 41 Aminosäuren fehlen und/oder dem Carboxyende der Sequenz der den Naturtyp-bFGF bildenden Aminosäuren eine bis 61 Aminosäuren fehlen.

2. Mutein wie in Anspruch 1 beansprucht, in welchem der Naturtyp-bGFG in seinem Molekül die Aminosäuresequenz:

Phe-Phe-Leu-Arg-Ile-His-Pro-Asp-Gly-Arg-Val-Asp-Gly-Val-
Arg-Glu-Lys-Ser-Asp-Pro

enthält.

3. Mutein wie in Anspruch 1 und 2 beansprucht, in welchem die neu eingeführte Aminosäure eine neutrale Aminosäure ist.

4. Mutein wie in Anspruch 3 beansprucht, in welchem die neutrale Aminosäure aus der Gruppe ausgewählt ist, die aus Glycin, Valin, Alanin, Leucin, Isoleucin, Tyrosin, Phenylalanin, Histidin, Tryptophan, Serin, Threonin und Methionin besteht.

5. Mutein wie in Anspruch 4 beansprucht, in welchem die natürliche Aminosäure Serin oder Threonin ist.

6. Mutein wie in Anspruch 5 beansprucht, in welchem die neutrale Aminosäure Serin ist.

7. Mutein wie in Anspruch 6 beansprucht, in welchem der Cysteinrest in Position 70 des Human-bFGF durch Serin ersetzt ist.

8. Mutein wie in Anspruch 6 beansprucht, in welchem der Cysteinrest in Position 88 des Human-bFGF durch Serin ersetzt ist.

9. Mutein wie in Anspruch 6 beansprucht, in welchem die Cysteinreste in Position 70 und in Position 88 des Human-bFGF durch Serin ersetzt sind.

10. Mutein wie in Anspruch 1 beansprucht, in welchem dem Aminoende der den bFGF bildenden Aminosäuren wenigstens eine Aminosäure fehlt.

11. Mutein wie in Anspruch 1 beansprucht, in welchem dem Carboxylende der den bFGF bildenden Aminosäuren wenigstens eine Aminosäure fehlt.

12. Rekombinante DNA mit einer Basensequenz, die für das in einem der Ansprüche 1-11 beanspruchte Mutein aus dem basischen Fibroblasten-Wachstumsfaktor (bFGF) kodiert.

13. Rekombinante DNA wie in Anspruch 12 beansprucht, in welcher die neu eingeführte Aminosäure eine neutrale Aminosäure ist, die aus der Gruppe ausgewählt ist, die aus Glycin, Valin, Alanin, Leucin, Isoleucin, Tyrosin, Phenylalanin, Histidin, Tryptophan, Serin, Threonin und Methionin besteht.

**14.** Rekombinante DNA wie in Anspruch 13 beansprucht, in welcher die neu eingeführte Aminosäure Serin ist.

**15.** Rekombinante DNA wie in Anspruch 12 beansprucht, in welcher dem Aminoende oder dem Carboxylende der den bFGF bildenden Aminosäuren wenigstens eine Aminosäure fehlt.

**16.** Transformante, die einen Vektor beherbergt, der eine rekombinante DNA mit einer Basensequenz enthält, welche für das in einem der Ansprüche 1-11 definierte Mutein des basischen Fibroblasten-Wachstumsfaktor (bFGF) kodiert.

**17.** Transformante wie in Anspruch 16 beansprucht, in welcher die neu eingeführte Aminosäure eine neutrale Aminosäure ist, die aus der Gruppe ausgewählt ist, die aus Glycin, Valin, Alanin, Leucin, Isoleucin, Tyrosin, Phenylalanin, Histidin, Tryptophan, Serin, Threonin und Methionin besteht.

**18.** Transformante wie in Anspruch 17 beansprucht, in welcher die neu eingeführte Aminosäure Serin ist.

**19.** Transformante wie in Anspruch 16 beansprucht, in welcher dem Aminoende oder dem Carboxylende der den bFGF bildenden Aminosäuren wenigstens eine Aminosäure fehlt.

**20.** Verfahren zum Herstellen eines Muteins des basischen Fibroblasten-Wachstumsfaktors (bFGF), welches das Kultivieren einer Transformanten, die einen Vektor beherbergt, der eine rekombinante DNA mit einer Basensequenz enthält, welche für das in einem der Ansprüche 1-11 definierte Mutein des basischen Fibroblasten-Wachstumsfaktors (bFGF) kodiert, in einem Kulturmedium unter Erzeugen und Anreichern des Muteins in der Kulturbrühe umfaßt.

**21.** Verfahren wie in Anspruch 20 beansprucht, wobei die neu eingeführte Aminosäure eine neutrale Aminosäure ist, die aus der Gruppe ausgewählt ist, die aus Glycin, Valin, Alanin, Leucin, Isoleucin, Tyrosin, Phenylalanin, Histidin, Tryptophan, Serin, Threonin und Methionin besteht.

**22.** Verfahren wie in Anspruch 21 beansprucht, wobei die neu eingeführte Aminosäure Serin ist.

**23.** Verfahren wie in Anspruch 20 beansprucht, wobei dem Aminoende oder dem Carboxylende der den Naturtyp-bFGF bildenden Aminosäuren wenigstens eine Aminosäure fehlt.

**Patentansprüche für folgenden Vertragsstaat : ES**

**1.** Verfahren zum Herstellen eines Muteins des basischen Fibroblasten-Wachstumsfaktors (bFGF), das ein Ersatztyp-Mutein oder eine Kombination eines Ersatztyp-Muteins und eines Auslassungstyp-Muteins ist, wobei
ein Ersatztyp-Mutein ein Mutein ist, in welchem wenigstens einer der vier Cysteinreste in der Sequenz der den Naturtyp-bFGF bildenden Aminosäuren durch eine andere Aminosäure als Cystein ersetzt ist, und
ein Auslassungstyp-Mutein ein Mutein ist, in welchem dem Aminoende der Sequenz der den Naturtyp-bFGF bildenden Aminosäuren eine bis 41 Aminosäuren fehlen und/oder dem Carboxyende der Sequenz der den Naturtyp-bFGF bildenden Aminosäuren eine bis 61 Aminosäuren fehlen,
welches das Kultivieren einer Transformanten, die einen Vektor beherbergt, der eine rekombinante DNA mit einer Basensequenz enthält, welche für das Mutein des basischen Fibroblasten-Wachstumsfaktors (bFGF) kodiert, in einem Kulturmedium unter Erzeugen und Anreichern des Muteins in der Kulturbrühe umfaßt.

**2.** Verfahren wie in Anspruch 1 beansprucht, bei welchem der Naturtyp-bGFG in seinem Molekül die Aminosäuresequenz:

Phe-Phe-Leu-Arg-Ile-His-Pro-Asp-Gly-Arg-Val-Asp-Gly-Val-
Arg-Glu-Lys-Ser-Asp-Pro

enthält.

3. Verfahren wie in Anspruch 1 und 2 beansprucht, bei welchem die neu eingeführte Aminosäure eine neutrale Aminosäure ist.

4. Verfahren wie in Anspruch 3 beansprucht, bei welchem die neutrale Aminosäure aus der Gruppe ausgewählt ist, die aus Glycin, Valin, Alanin, Leucin, Isoleucin, Tyrosin, Phenylalanin, Histidin, Tryptophan, Serin, Threonin und Methionin besteht.

5. Verfahren wie in Anspruch 4 beansprucht, bei welchem die natürliche Aminosäure Serin oder Threonin ist.

6. Verfahren wie in Anspruch 5 beansprucht, bei welchem die neutrale Aminosäure Serin ist.

7. Verfahren wie in Anspruch 6 beansprucht, bei welchem das hergestellte Mutein ein Mutein ist, in welchem der Cysteinrest in Position 70 des Human-bFGF durch Serin ersetzt ist.

8. Verfahren wie in Anspruch 6 beansprucht, bei welchem das hergestellte Mutein ein Mutein ist, in welchem der Cysteinrest in Position 88 des Human-bFGF durch Serin ersetzt ist.

9. Verfahren wie in Anspruch 6 beansprucht, bei welchem das hergestellte Mutein ein Mutein ist, bei welchem die Cysteinreste in Position 70 und in Position 88 des Human-bFGF durch Serin ersetzt sind.

10. Verfahren wie in Anspruch 1 beansprucht, bei welchem dem Aminoende der den bFGF bildenden Aminosäuren wenigstens eine Aminosäure fehlt.

11. Verfahren wie in Anspruch 1 beansprucht, bei welchem dem Carboxylende der den bFGF bildenden Aminosäuren wenigstens eine Aminosäure fehlt.

**Revendications**
**Revendications pour les Etats contractants suivants : AT, BE, CH, DE, FR, GB, GR, IT, LI, LU, NL, SE**

1. Protéine mutante dérivée du facteur basique de croissance des fibroblastes (bFCF), qui est une protéine mutante par substitution ou une protéine mutante par substitution et délétion combinées,
une protéine mutante par substitution étant une protéine mutante dans laquelle au moins l'un des quatre résidus de cystéine présents dans la séquence d'acides aminés constitutifs du bFCF naturel est remplacé par un résidu d'un acide aminé autre que la cystéine, et
une protéine mutante par délétion est une protéine mutante dans laquelle il manque de 1 à 41 résidus d'acide aminé de l'extrémité amino-terminale de la séquence d'acides aminés constitutifs du bFCF et/ou de 1 à 61 résidus d'acide aminé de l'extrémité carboxy-terminale de la séquence d'acides aminés constitutifs du bFCF.

2. Protéine mutante conforme à la revendication 1, dans laquelle la molécule de bFCF naturel contient la séquence d'acides aminés suivante :

Phe-Phe-Leu-Arg-Ile-His-Pro-Asp-Gly-Arg-Val-Asp-
Gly-Val-Arg-Glu-Lys-Ser-Asp-Pro

3. Protéine mutante conforme à l'une des revendications 1 et 2, dans laquelle le nouveau résidu d'acide aminé introduit est un résidu d'acide aminé neutre.

4. Protéine mutante conforme à la revendication 3, dans laquelle l'acide aminé neutre est choisi dans l'ensemble constitué par les glycine, valine, alanine, leucine, isoleucine, tyrosine, phénylalanine, histidine, tryptophane, sérine, thréonine et méthionine.

5. Protéine mutante conforme à la revendication 4, dans laquelle l'acide aminé neutre est la sérine ou la thréonine.

6. Protéine mutante conforme à la revendication 5, dans laquelle l'acide aminé neutre est la sérine.

7. Protéine mutante conforme à la revendication 6, dans laquelle le résidu de cystéine situé en position 70 dans le bFCF humain est remplacé par un résidu de sérine.

8. Protéine mutante conforme à la revendication 6, dans laquelle le résidu de cystéine situé en position 88 dans le bFCF humain est remplacé par un résidu de sérine.

9. Protéine mutante conforme à la revendication 6, dans laquelle les résidus de cystéine situés en positions 70 et 88 dans le bFCF humain sont remplacés par des résidus de sérine.

10. Protéine mutante conforme à la revendication 1, dans laquelle il manque au moins 1 résidu d'acide aminé de l'extrémité amino-terminale de la séquence d'acides aminés constitutifs du bFCF.

11. Protéine mutante conforme à la revendication 1, dans laquelle il manque au moins 1 résidu d'acide aminé de l'extrémité carboxy-terminale de la séquence d'acides aminés constitutifs du bFCF.

12. ADN recombinant présentant une séquence de bases qui code une protéine mutante dérivée du facteur basique de croissance des fibroblastes (bFCF), définie dans l'une des revendications 1 à 11.

13. ADN recombinant conforme à la revendication 12, dans lequel le nouveau résidu d'acide aminé introduit est un résidu d'un acide aminé neutre choisi dans l'ensemble constitué par les glycine, valine, alanine, leucine, isoleucine, tyrosine, phénylalanine, histidine, tryptophane, sérine, thréonine et méthionine.

14. ADN recombinant conforme à la revendication 13, dans lequel le nouveau résidu d'acide aminé introduit est un résidu de sérine.

15. ADN recombinant conforme à la revendication 12, dans lequel il manque au moins 1 résidu d'acide aminé de l'extrémité amino-terminale ou de l'extrémité carboxy-terminale de la séquence d'acides aminés constitutifs du bFCF.

16. Transformant hébergeant un vecteur contenant un ADN recombinant présentant une séquence de bases qui code une protéine mutante dérivée du facteur basique de croissance des fibroblastes (bFCF), définie dans l'une des revendications 1 à 11.

17. Transformant conforme à la revendication 16, dans lequel le nouveau résidu d'acide aminé introduit est un résidu d'un acide aminé neutre choisi dans l'ensemble constitué par les glycine, valine, alanine, leucine, isoleucine, tyrosine, phénylalanine, histidine, tryptophane, sérine, thréonine et méthionine.

18. Transformant conforme à la revendication 17, dans lequel le nouveau résidu d'acide aminé introduit est un résidu de sérine.

19. Transformant conforme à la revendication 16, dans lequel il manque au moins 1 résidu d'acide aminé de l'extrémité amino-terminale ou de l'extrémité carboxy-terminale de la séquence d'acides aminés constitutifs du bFCF.

20. Procédé de production d'une protéine mutante dérivée du facteur basique de croissance des fibroblastes (bFCF), qui comporte le fait de cultiver, dans un milieu de culture, un transformant hébergeant un vecteur contenant un ADN recombinant présentant une séquence de bases qui code la protéine mutante dérivée du bFCF, définie dans l'une des revendications 1 à 11, de façon que la protéine mutante soit produite et s'accumule dans le bouillon de culture.

21. Procédé conforme à la revendication 20, dans lequel le nouveau résidu d'acide aminé introduit est un résidu d'un acide aminé neutre choisi dans l'ensemble constitué par les glycine, valine, alanine, leucine, isoleucine, tyrosine, phénylalanine, histidine, tryptophane, sérine, thréonine et méthionine.

**22.** Procédé conforme à la revendication 22, dans lequel le nouveau résidu d'acide aminé introduit est un résidu de sérine.

**23.** Procédé conforme à la revendication 20, dans lequel il manque au moins 1 résidu d'acide aminé de l'extrémité amino-terminale ou de l'extrémité carboxy-terminale de la séquence d'acides aminés constitutifs du bFCF.

**Revendications pour l'Etat contractant suivant : ES**

**1.** Procédé de production d'une protéine mutante dérivée du facteur basique de croissance des fibroblastes (bFCF), qui est une protéine mutante par substitution ou une protéine mutante par substitution et délétion combinées,
une protéine mutante par substitution étant une protéine mutante dans laquelle au moins l'un des quatre résidus de cystéine présents dans la séquence d'acides aminés constitutifs du bFCF naturel est remplacé par un résidu d'un acide aminé autre que la cystéine, et
une protéine mutante par délétion est une protéine mutante dans laquelle il manque de 1 à 41 résidus d'acide aminé de l'extrémité amino-terminale de la séquence d'acides aminés constitutifs du bFCF et/ou de 1 à 61 résidus d'acide aminé de l'extrémité carboxy-terminale de la séquence d'acides aminés constitutifs du bFCF,
lequel procédé comporte le fait de cultiver, dans un milieu de culture, un transformant hébergeant un vecteur contenant un ADN recombinant présentant une séquence de bases qui code ladite protéine mutante dérivée du bFCF, de façon que la protéine mutante soit produite et s'accumule dans le bouillon de culture.

**2.** Procédé conforme à la revendication 1, dans lequel la molécule de bFCF naturel contient la séquence d'acides aminés suivante :

Phe-Phe-Leu-Arg-Ile-His-Pro-Asp-Gly-Arg-Val-Asp-
Gly-Val-Arg-Glu-Lys-Ser-Asp-Pro

**3.** Procédé conforme à l'une des revendications 1 et 2, dans lequel le nouveau résidu d'acide aminé introduit est un résidu d'acide aminé neutre.

**4.** Procédé conforme à la revendication 3, dans lequel l'acide aminé neutre est choisi dans l'ensemble constitué par les glycine, valine, alanine, leucine, isoleucine, tyrosine, phénylalanine, histidine, tryptophane, sérine, thréonine et méthionine.

**5.** Procédé conforme à la revendication 4, dans lequel l'acide aminé neutre est la sérine ou la thréonine.

**6.** Procédé conforme à la revendication 5, dans lequel l'acide aminé neutre est la sérine.

**7.** Procédé conforme à la revendication 6, dans lequel le résidu de cystéine situé en position 70 dans le bFCF humain est remplacé par un résidu de sérine.

**8.** Procédé conforme à la revendication 6, dans lequel le résidu de cystéine situé en position 88 dans le bFCF humain est remplacé par un résidu de sérine.

**9.** Procédé conforme à la revendication 6, dans lequel les résidus de cystéine situés en positions 70 et 88 dans le bFCF humain sont remplacés par des résidus de sérine.

**10.** Procédé conforme à la revendication 1, dans lequel il manque au moins 1 résidu d'acide aminé de l'extrémité amino-terminale de la séquence d'acides aminés constitutifs du bFCF.

11. Procédé conforme à la revendication 1, dans lequel il manque au moins 1 résidu d'acide aminé de l'extrémité carboxy-terminale de la séquence d'acides aminés constitutifs du bFCF.

Figure 1

```
MetProAlaLeuProGluAspGlyGlySerGlyAlaPheProProGlyHisPheLysAsp   20
ATGCCAGCATTGCCCGAGGATGGCGGCAGCGGCGCCTTCCCGCCCGGCCACTTCAAGGAC   60

ProLysArgLeuTyrCysLysAsnGlyGlyPhePheLeuArgIleHisProAspGlyArg   40
CCCAAGCGGCTGTACTGCAAAAACGGGGGCTTCTTCCTGCGCATCCACCCCGACGGCCGA  120

ValAspGlyValArgGluLysSerAspProHisIleLysLeuGlnLeuGlnAlaGluGlu   60
GTTGACGGGGTCCGGGAGAAGAGCGACCCTCACATCAAGCTACAACTTCAAGCAGAAGAG  180

ArgGlyValValSerIleLysGlyValCysAlaAsnArgTyrLeuAlaMetLysGluAsp   80
AGAGGAGTTGTGTCTATCAAAGGAGTGTGTGCTAACCGTTACCTGGCTATGAAGGAAGAT  240

GlyArgLeuLeuAlaSerLysCysValThrAspGluCysPhePhePheGluArgLeuGlu  100
GGAAGATTACTGGCTTCTAAATGTGTTACGGATGAGTGTTTCTTTTTTGAACGATTGGAA  300

SerAsnAsnTyrAsnThrTyrArgSerArgLysTyrThrSerTrpTyrValAlaLeuLys  120
TCTAATAACTACAATACTTACCGGTCAAGGAAATACACCAGTTGGTATGTGGCACTGAAA  360

ArgThrGlyGlnTyrLysLeuGlySerLysThrGlyProGlyGlnLysAlaIleLeuPhe  140
CGAACTGGGCAGTATAAACTTGGATCCAAAACAGGACCTGGGCAGAAAGCTATACTTTTT  420

LeuProMetSerAlaLysSertrm                                      147
CTTCCAATGTCTGCTAAGAGCTGA                                      444
```

Figure 2

```
                  Ser
(1)   5'-CGTTCTTGCTGTAGAGCCGCT-3'
                     (RsaI)

                     Ser
(2)   5'-AACGATTAGCGCTCACTCC-3'
                   HaeII

                Ser
(3)   5'-GTAACAGACTTAGAAGCTAGT-3'
                         AluI

                      Ser
(4)   5'-TCGAAGAAGAAAGACTCATCC-3'
                       HinfI
```

Figure 3

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrSerLysAsnGlyGlyPhePheLeuArgIleHisProAspGlyArg     40
CCCAAGCGGCTCTACAGCAAGAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValCysAlaAsnArgTyrLeuAlaMetLysGluAsp     80
AGAGGAGTTGTGTCTATCAAAGGAGTGTGTGCTAACCGTTACCTGGCTATGAAGGAAGAT    240

GlyArgLeuLeuAlaSerLysCysValThrAspGluCysPhePhePheGluArgLeuGlu    100
GGAAGATTACTGGCTTCTAAATGTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 4

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrCysLysAsnGlyGlyPhePheLeuArgIleHisProAspGlyArg     40
CCCAAGCGGCTGTACTGCAAAAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValSerAlaAsnArgTyrLeuAlaMetLysGluAsp     80
AGAGGAGTTGTGTCTATCAAAGGAGTGAGCGCTAATCGTTACCTGGCTATGAAGGAAGAT    240

GlyArgLeuLeuAlaSerLysCysValThrAspGluCysPhePhePheGluArgLeuGlu    100
GGAAGATTACTGGCTTCTAAATGTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 5

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrCysLysAsnGlyGlyPhePheLeuArgIleHisProAspGlyArg     40
CCCAAGCGGCTGTACTGCAAAAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValCysAlaAsnArgTyrLeuAlaMetLysGluAsp     80
AGAGGAGTTGTGTCTATCAAAGGAGTGTGTGCTAACCGTTACCTGGCTATGAAGGAAGAT    240

GlyArgLeuLeuAlaSerLysSerValThrAspGluCysPhePhePheGluArgLeuGlu    100
GGAAGATTACTAGCTTCTAAGTCTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 6

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluSerPhePhePheGluArgLeuGlu   100
GGAAGATTACTGGCTTCTAAATGTGTTACGGATGAGTCTTTCTTCTTCGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 7

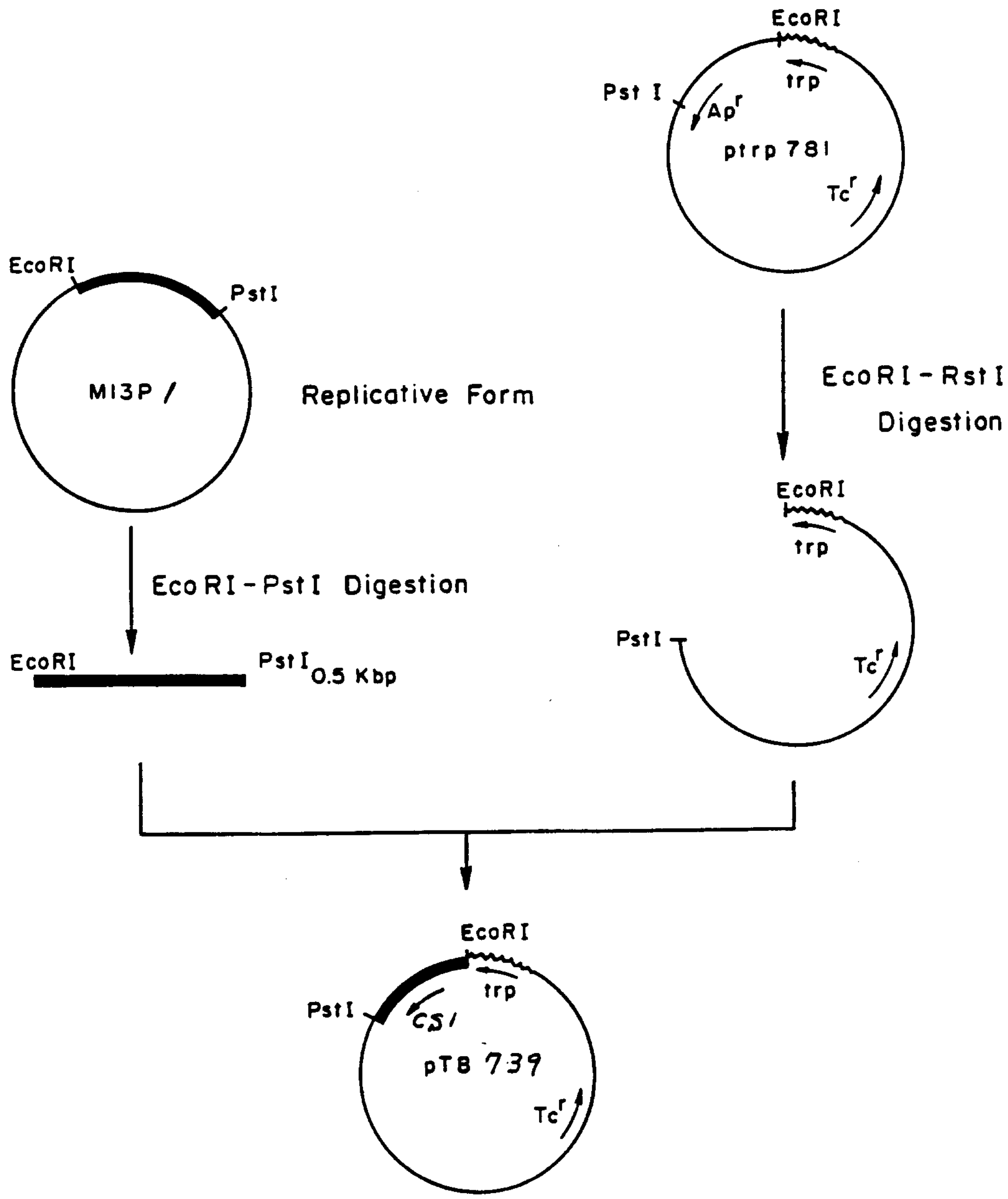
EcoRI
trp
Pst I
Apr
ptrp 781
Tcr
EcoRI
PstI
M13P /
Replicative Form
EcoRI-RstI
Digestion
EcoRI-PstI Digestion
EcoRI
PstI
0.5 Kbp
EcoRI
trp
PstI
Tcr
EcoRI
trp
PstI
CS1
pTB 739
Tcr

Figure 8

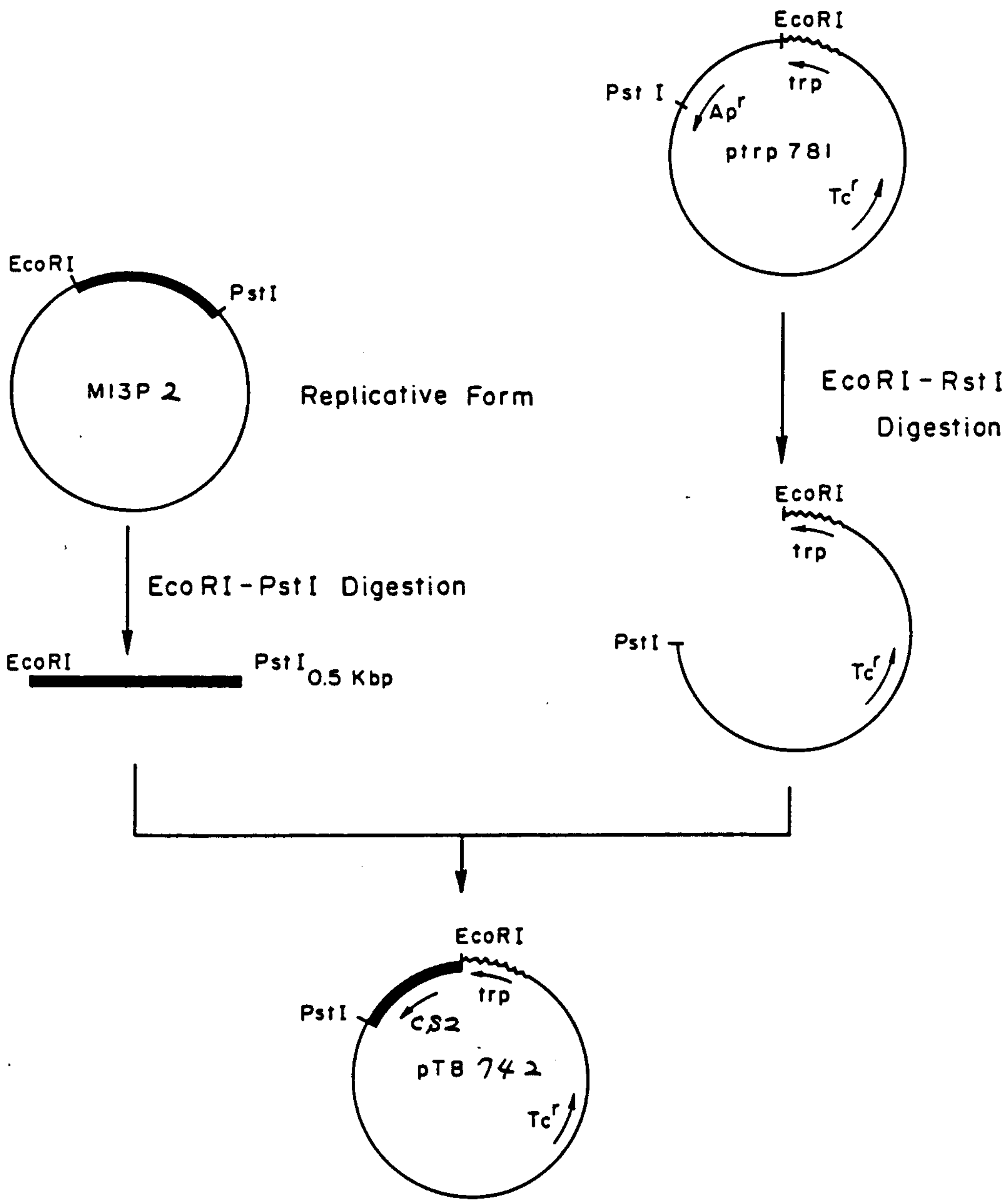
EcoRI
Pst I
Apr
trp
ptrp 781
Tcr
EcoRI
PstI
M13P 2
Replicative Form
EcoRI-RstI
Digestion
EcoRI
trp
EcoRI-PstI Digestion
EcoRI
PstI
0.5 Kbp
PstI
Tcr
EcoRI
trp
PstI
CS2
pTB 742
Tcr

Figure 9

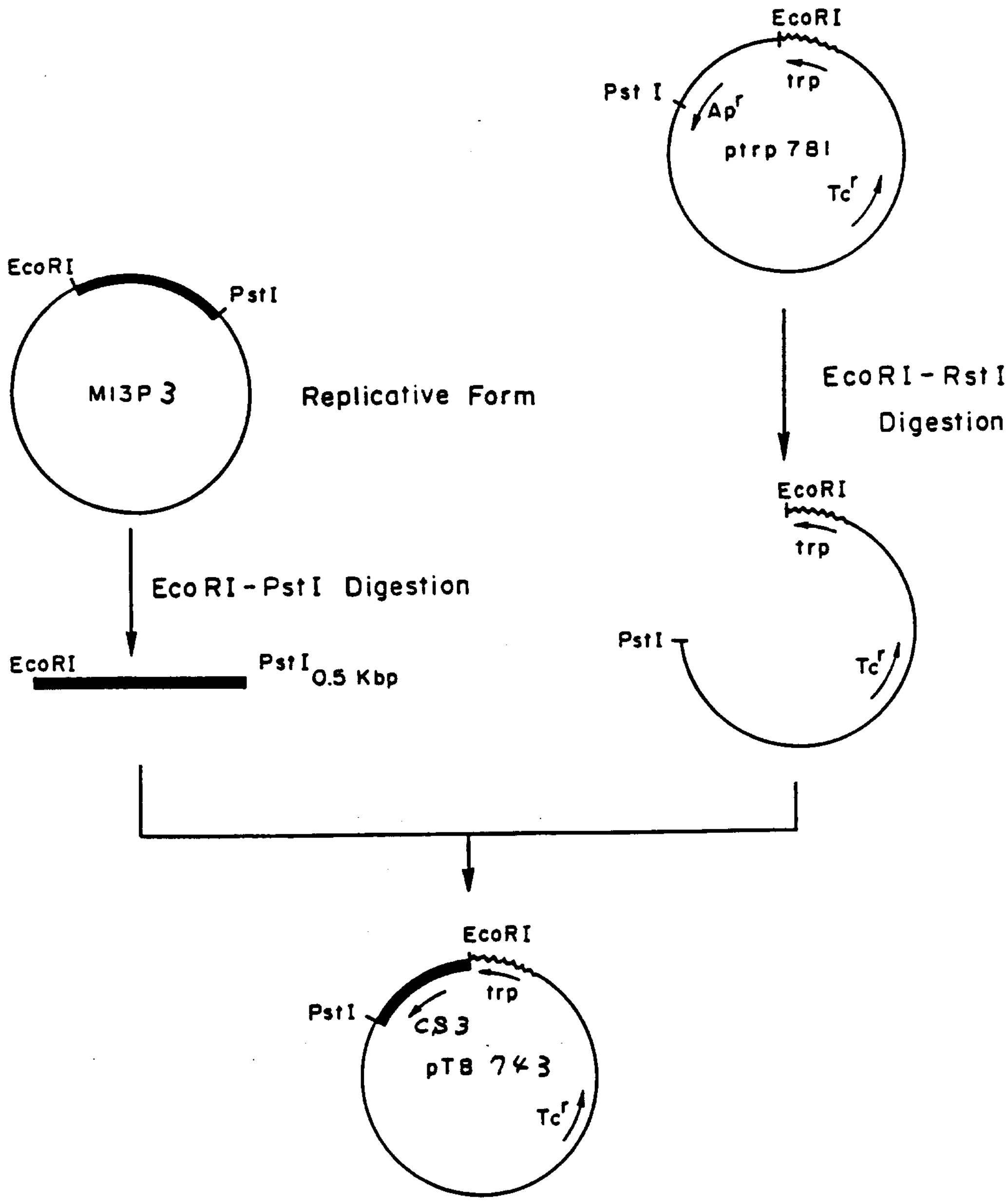
EcoRI
Pst I
Apr
trp
ptrp 781
Tcr
EcoRI
PstI
M13P 3
Replicative Form
EcoRI - RstI
Digestion
EcoRI
trp
PstI
Tcr
EcoRI - PstI Digestion
EcoRI
PstI 0.5 Kbp
EcoRI
trp
PstI
CS3
pTB 743
Tcr

Figure 10

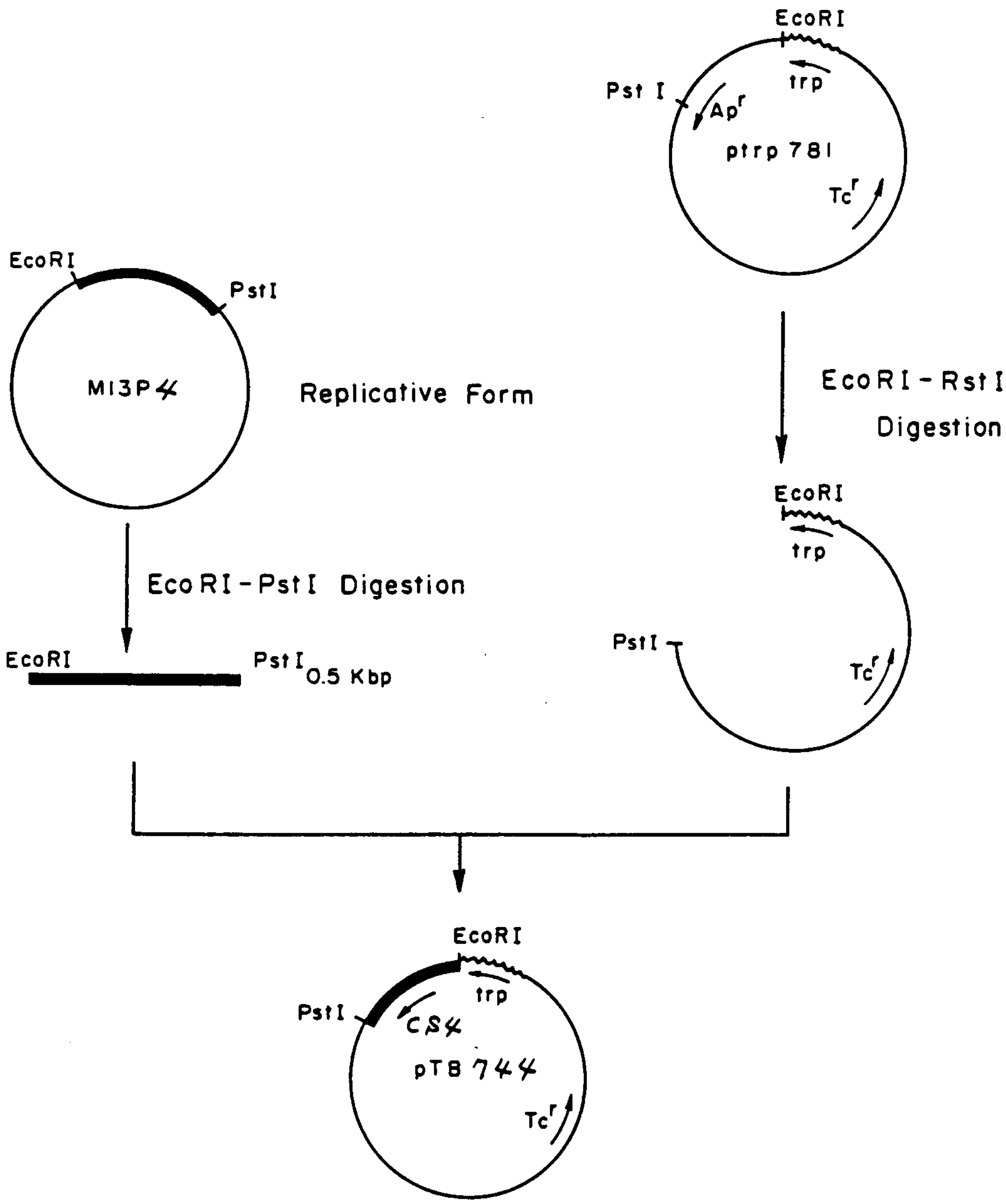
EcoRI
Pst I
trp
Ap^r
ptrp 781
Tc^r
EcoRI
PstI
M13P4
Replicative Form
EcoRI-RstI
Digestion
EcoRI
trp
EcoRI-PstI Digestion
EcoRI
PstI
0.5 Kbp
PstI
Tc^r
EcoRI
PstI
trp
CS4
pTB 744
Tc^r

Figure 11

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValSerAlaAsnArgTyrLeuAlaMetLysGluAsp    80
AGAGGAGTTGTGTCTATCAAAGGAGTGAGCGCTAATCGTTACCTGGCTATGAAGGAAGAT   240

GlyArgLeuLeuAlaSerLysSerValThrAspGluCysPhePhePheGluArgLeuGlu   100
GGAAGATTACTAGCTTCTAAGTCTGTTACGGATGAGTGTTTCTTTTTTGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 12

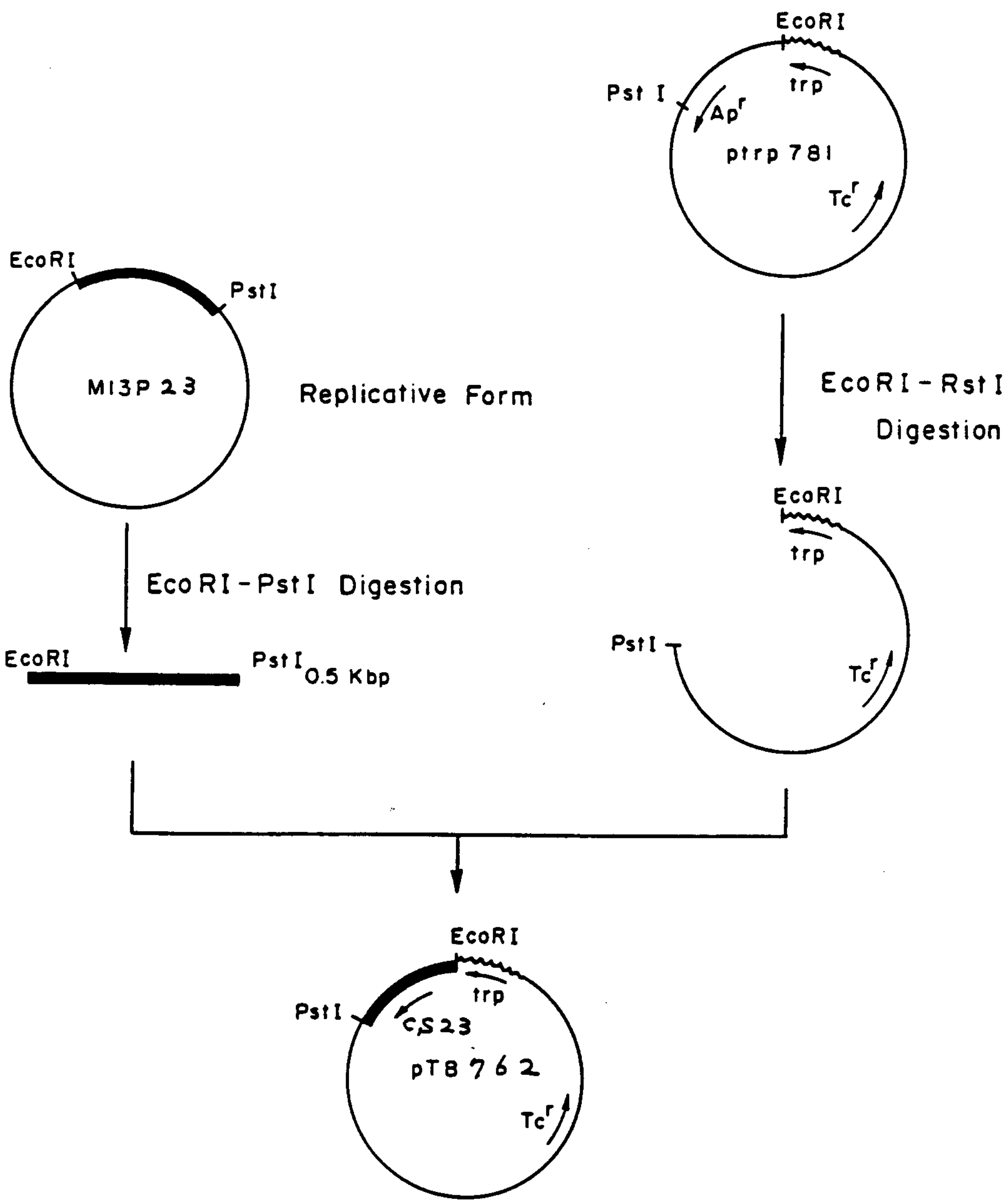
EcoRI
Pst I
trp
$Ap^r$
ptrp 781
$Tc^r$
EcoRI
PstI
M13P 23
Replicative Form
EcoRI-RstI
Digestion
EcoRI
trp
EcoRI-PstI Digestion
EcoRI
PstI
0.5 Kbp
PstI
$Tc^r$
EcoRI
trp
PstI
cS23
pT8762
$Tc^r$

Figure 13

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrSerLysAsnGlyGlyPhePheLeuArgIleHisProAspGlyArg     40
CCCAAGCGGCTCTACAGCAAGAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValSerAlaAsnArgTyrLeuAlaMetLysGluAsp     80
AGAGGAGTTGTGTCTATCAAAGGAGTGAGCGCTAATCGTTACCTGGCTATGAAGGAAGAT    240

GlyArgLeuLeuAlaSerLysSerValThrAspGluCysPhePhePheGluArgLeuGlu    100
GGAAGATTACTAGCTTCTAAGTCTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 14

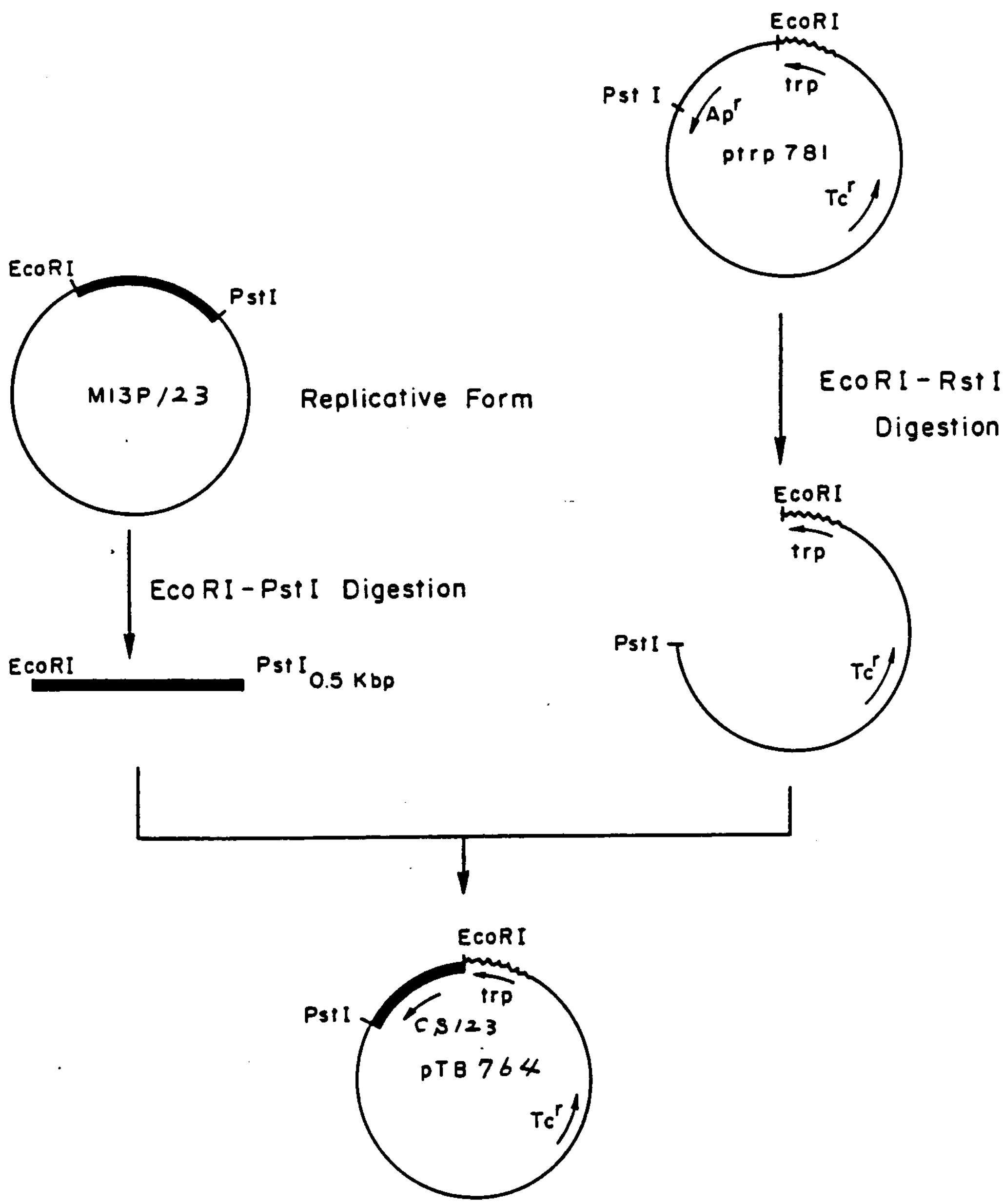
EcoRI
Pst I
trp
Apr
ptrp 781
Tcr
EcoRI
PstI
M13P/23
Replicative Form
EcoRI-RstI
Digestion
EcoRI
trp
PstI
Tcr
EcoRI-PstI Digestion
EcoRI
PstI
0.5 Kbp
EcoRI
trp
PstI
CS/23
pTB 764
Tcr

Figure 15

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrSerLysAsnGlyGlyPhePheLeuArgIleHisProAspGlyArg     40
CCCAAGCGGCTCTACAGCAAGAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValSerAlaAsnArgTyrLeuAlaMetLysGluAsp     80
AGAGGAGTTGTGTCTATCAAAGGAGTGAGCGCTAATCGTTACCTGGCTATGAAGGAAGAT    240

GlyArgLeuLeuAlaSerLysSerValThrAspGluSerPhePhePheGluArgLeuGlu    100
GGAAGATTACTAGCTTCTAAGTCTGTTACGGATGAGTCTTTCTTCTTCGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 16

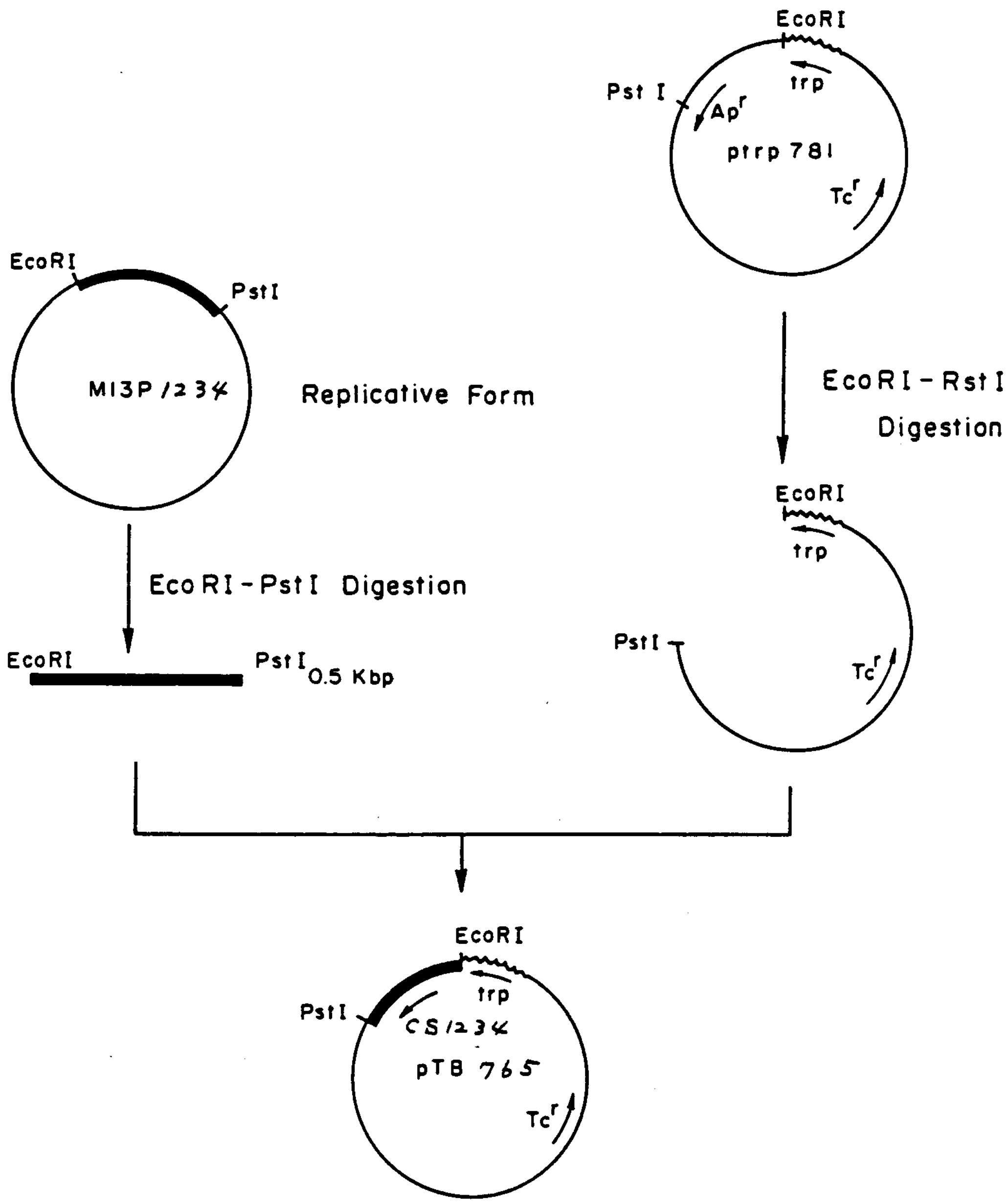
EcoRI
Pst I
trp
Apr
ptrp 781
Tcr
EcoRI
PstI
M13P/234
Replicative Form
EcoRI-RstI
Digestion
EcoRI-PstI Digestion
EcoRI
PstI
0.5 Kbp
EcoRI
trp
PstI
Tcr
EcoRI
PstI
trp
CS/234
pTB 765
Tcr

Figure 17

```
MetProAlaLeuProGluAspGlyGlySerGlyAlaPheProProGlyHisPheLysAsp      20
ATGCCAGCATTGCCCGAGGATGGCGGCAGCGGCGCCTTCCCGCCCGGCCACTTCAAGGAC      60

ProLysArgLeuTyrSerLysAsnGlyGlyPhePheLeuArgIleHisProAspGlyArg      40
CCCAAGCGGCTCTACAGCAAGAACGGGGGCTTCTTCCTGCGCATCCACCCCGACGGCCGA     120

ValAspGlyValArgGluLysSerAspProHisIleLysLeuGlnLeuGlnAlaGluGlu      60
GTTGACGGGGTCCGGGAGAAGAGCGACCCTCACATCAAGCTACAACTTCAAGCAGAAGAG     180

ArgGlyValValSerIleLysGlyValSerAlaAsnArgTyrLeuAlaMetLysGluAsp      80
AGAGGAGTTGTGTCTATCAAAGGAGTGAGCGCTAATCGTTACCTGGCTATGAAGGAAGAT     240

GlyArgLeuLeuAlaSerLysCysValThrAspGluCysPhePhePheGluArgLeuGlu     100
GGAAGATTACTGGCTTCTAAATGTGTTACGGATGAGTGTTTCTTTTTTGAACGATTGGAA     300

SerAsnAsnTyrAsnThrTyrArgSerArgLysTyrThrSerTrpTyrValAlaLeuLys     120
TCTAATAACTACAATACTTACCGGTCAAGGAAATACACCAGTTGGTATGTGGCACTGAAA     360

ArgThrGlyGlnTyrLysLeuGlySerLysThrGlyProGlyGlnLysAlaIleLeuPhe     140
CGAACTGGGCAGTATAAACTTGGATCCAAAACAGGACCTGGGCAGAAAGCTATACTTTTT     420

LeuProMetSerAlaLysSertrm                                         147
CTTCCAATGTCTGCTAAGAGCTGA                                         444.
```

Figure 18

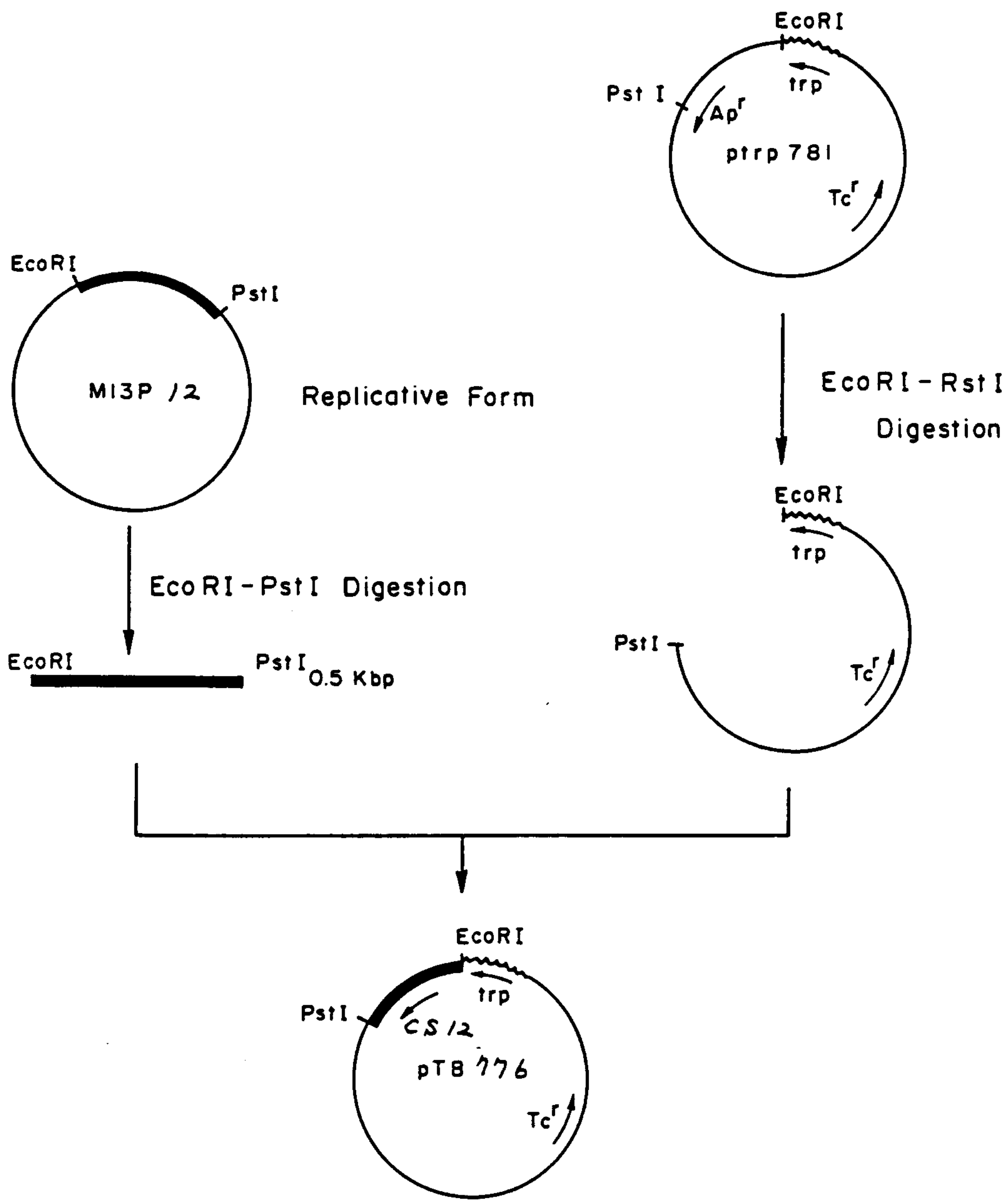
EcoRI
Pst I
trp
Apr
ptrp 781
Tcr
EcoRI
PstI
M13P 12
Replicative Form
EcoRI-RstI
Digestion
EcoRI
trp
EcoRI-PstI Digestion
EcoRI
PstI
0.5 Kbp
PstI
Tcr
EcoRI
trp
PstI
CS12
pTB 776
Tcr

Figure 19

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValSerAlaAsnArgTyrLeuAlaMetLysGluAsp    80
AGAGGAGTTGTGTCTATCAAAGGAGTGAGCGCTAATCGTTACCTGGCTATGAAGGAAGAT   240

GlyArgLeuLeuAlaSerLysCysValThrAspGluSerPhePhePheGluArgLeuGlu   100
GGAAGATTACTGGCTTCTAAATGTGTTACGGATGAGTCTTTCTTCTTCGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 20

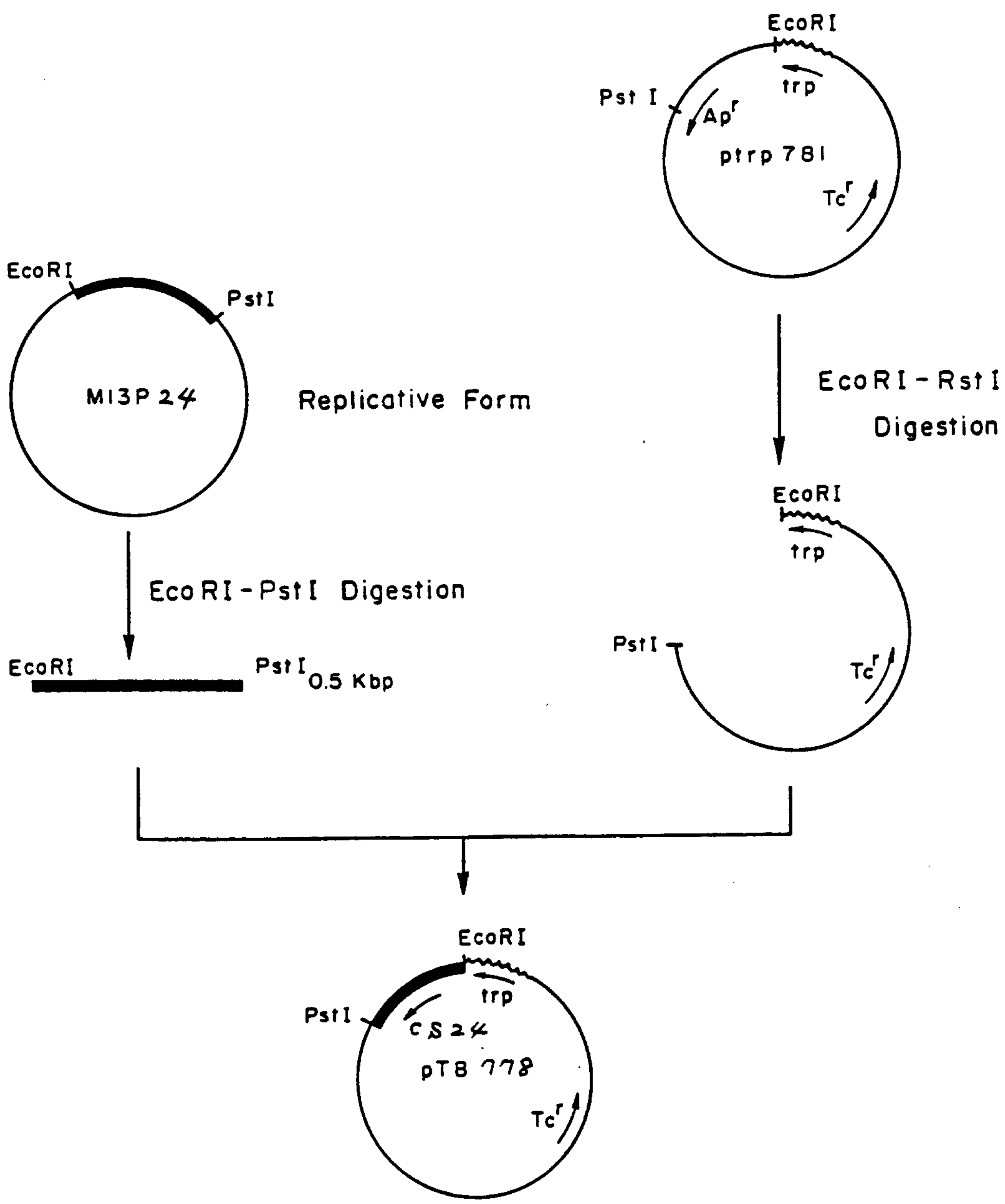
EcoRI
Pst I
trp
Ap^r
ptrp 781
Tc^r
EcoRI
PstI
M13P 24
Replicative Form
EcoRI - RstI
Digestion
EcoRI - PstI Digestion
EcoRI
PstI
0.5 Kbp
EcoRI
trp
PstI
Tc^r
EcoRI
PstI
trp
cS 24
pTB 778
Tc^r

Figure 21

MetProAlaLeuProGluAspGlyGlySerGlyAlaPheProProGlyHisPheLysAsp 20

ATGCCAGCATTGCCCGAGGATGGCGGCAGCGGCGCCTTCCCGCCCGGCCACTTCAAGGAC 60

ProLysArgLeuTyrSerLysAsnGlyGlyPhePheLeuArgIleHisProAspGlyArg 40

CCCAAGCGGCTCTACAGCAAGAACGGGGGCTTCTTCCTGCGCATCCACCCCGACGGCCGA 120

ValAspGlyValArgGluLysSerAspProHisIleLysLeuGlnLeuGlnAlaGluGlu 60

GTTGACGGGGTCCGGGAGAAGAGCGACCCTCACATCAAGCTACAACTTCAAGCAGAAGAG 180

ArgGlyValValSerIleLysGlyValCysAlaAsnArgTyrLeuAlaMetLysGluAsp 80

AGAGGAGTTGTGTCTATCAAAGGAGTGTGTGCTAACCGTTACCTGGCTATGAAGGAAGAT 240

GlyArgLeuLeuAlaSerLysSerValThrAspGluCysPhePhePheGluArgLeuGlu 100

GGAAGATTACTAGCTTCTAAGTCTGTTACGGATGAGTGTTTCTTTTTTGAACGATTGGAA 300

SerAsnAsnTyrAsnThrTyrArgSerArgLysTyrThrSerTrpTyrValAlaLeuLys 120

TCTAATAACTACAATACTTACCGGTCAAGGAAATACACCAGTTGGTATGTGGCACTGAAA 360

ArgThrGlyGlnTyrLysLeuGlySerLysThrGlyProGlyGlnLysAlaIleLeuPhe 140

CGAACTGGGCAGTATAAACTTGGATCCAAAACAGGACCTGGGCAGAAAGCTATACTTTTT 420

LeuProMetSerAlaLysSertrm 147

CTTCCAATGTCTGCTAAGAGCTGA 444.

Figure 22

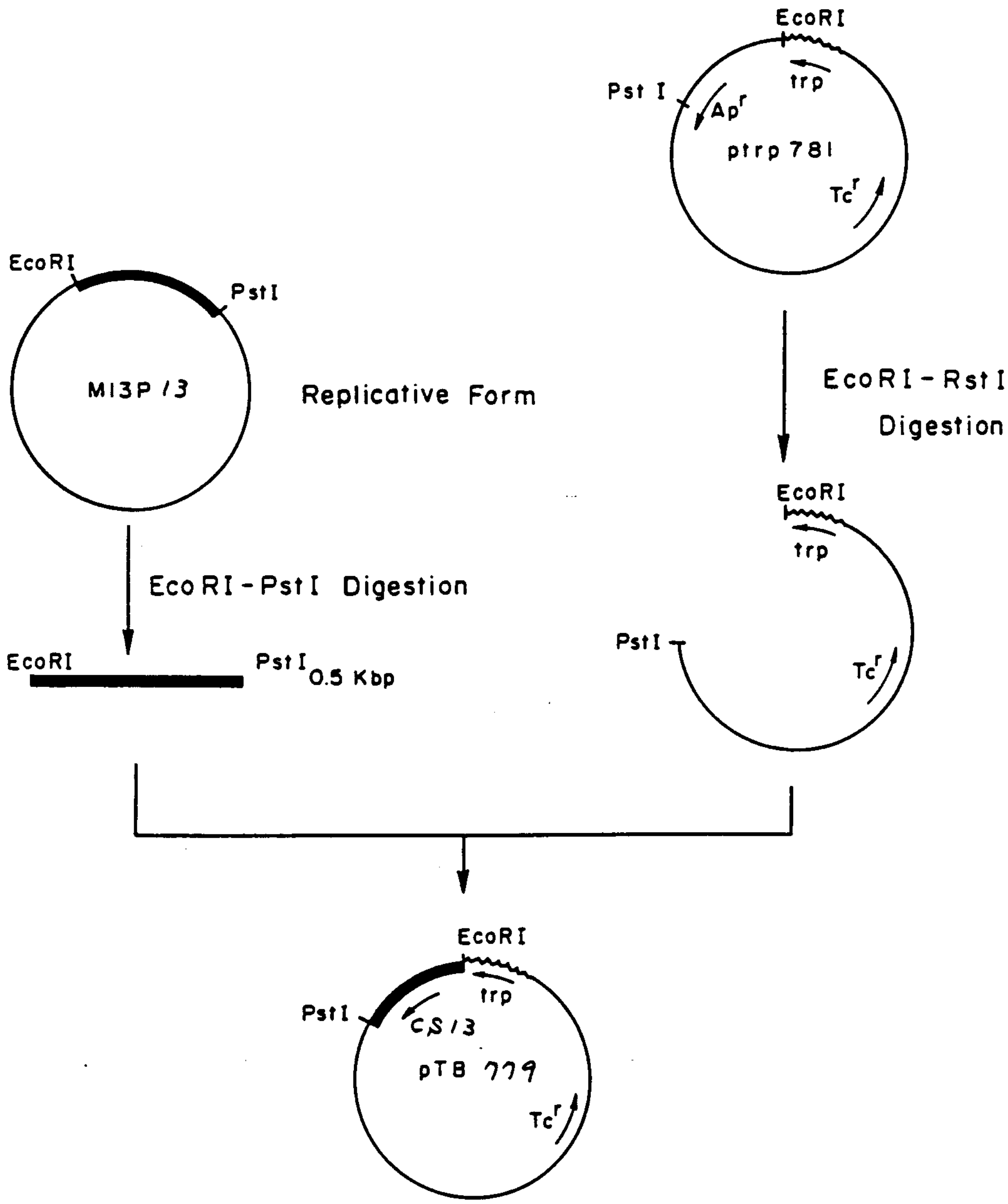

EcoRI
Pst I
trp
Apr
ptrp 781
Tcr
EcoRI
PstI
M13P13
Replicative Form
EcoRI-RstI
Digestion
EcoRI
trp
PstI
Tcr
EcoRI-PstI Digestion
EcoRI
PstI
0.5 Kbp
EcoRI
trp
PstI
CS13
pTB 779
Tcr

Figure 23

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrSerLysAsnGlyGlyPhePheLeuArgIleHisProAspGlyArg    40
CCCAAGCGGCTCTACAGCAAGAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluSerPhePhePheGluArgLeuGlu   100
GGAAGATTACTGGCTTCTAAATGTGTTACGGATGAGTCTTTCTTCTTCGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                        147
CTTCCAATGTCTGCTAAGAGCTGA                                        444.
```

Figure 24

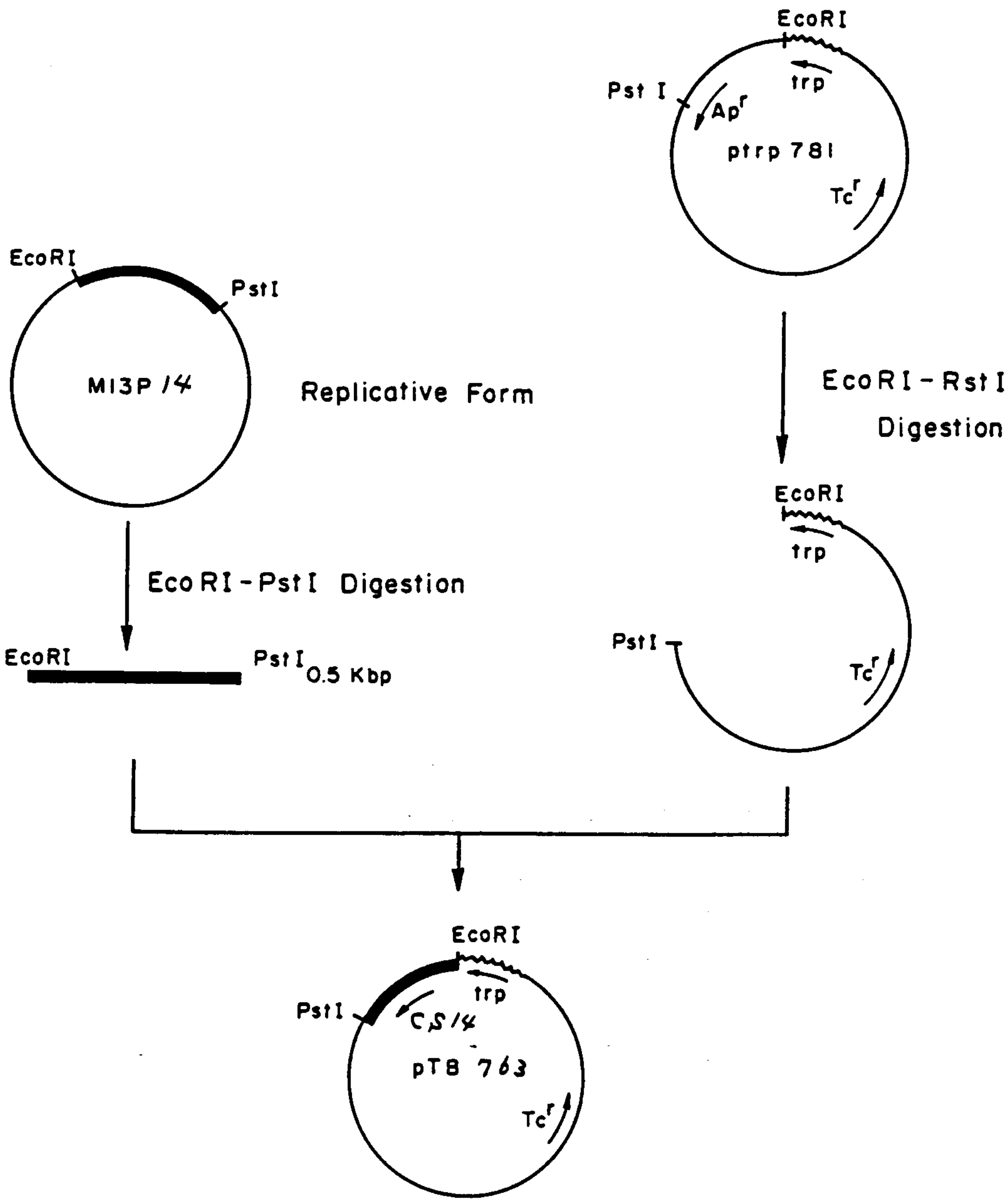
EcoRI
Pst I
trp
Apr
ptrp 781
Tcr
EcoRI
PstI
M13P 14
Replicative Form
EcoRI-RstI
Digestion
EcoRI
trp
PstI
Tcr
EcoRI-PstI Digestion
EcoRI
PstI 0.5 Kbp
EcoRI
trp
PstI
CS14
pTB 763
Tcr

Figure 25

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysSerValThrAspGluSerPhePhePheGluArgLeuGlu   100
GGAAGATTACTAGCTTCTAAGTCTGTTACGGATGAGTCTTTCTTCTTCGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444.
```

Figure 26

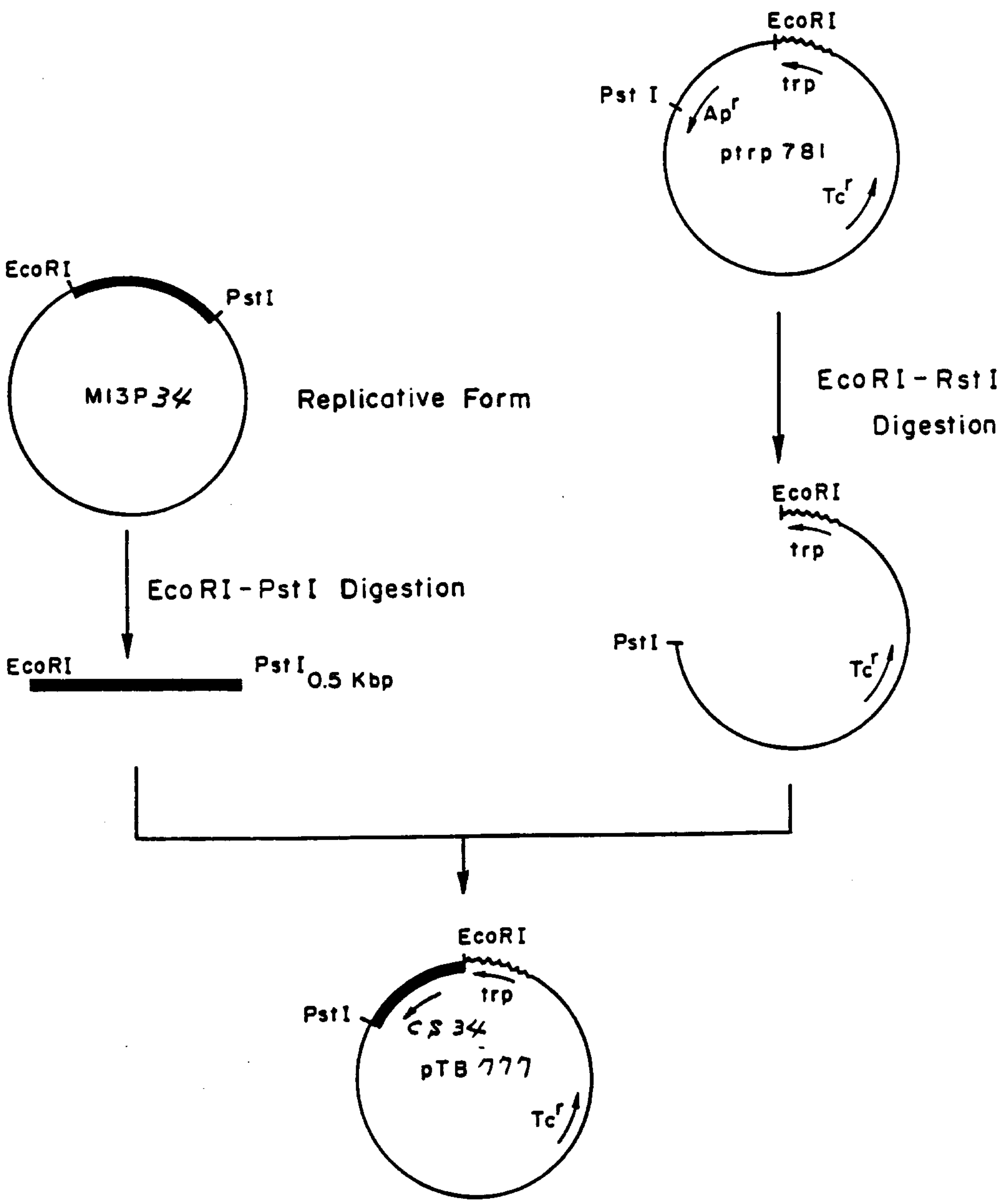
EcoRI
Pst I
Apr
trp
ptrp 781
Tcr
EcoRI
PstI
M13P34
Replicative Form
EcoRI - RstI
Digestion
EcoRI - PstI Digestion
EcoRI
PstI 0.5 Kbp
EcoRI
trp
PstI
Tcr
EcoRI
trp
PstI
CS 34
pTB 777
Tcr

Figure 27

```
MetProAlaLeuProGluAspGlyGlySerGlyAlaPheProProGlyHisPheLysAsp   20
ATGCCAGCATTGCCCGAGGATGGCGGCAGCGGCGCCTTCCCGCCCGGCCACTTCAAGGAC   60

ProLysArgLeuTyrCysLysAsnGlyGlyPhePheLeuArgIleHisProAspGlyArg   40
CCCAAGCGGCTGTACTGCAAAAACGGGGGCTTCTTCCTGCGCATCCACCCCGACGGCCGA  120

ValAspGlyValArgGluLysSerAspProHisIleLysLeuGlnLeuGlnAlaGluGlu   60
GTTGACGGGGTCCGGGAGAAGAGCGACCCTCACATCAAGCTACAACTTCAAGCAGAAGAG  180

ArgGlyValValSerIleLysGlyValSerAlaAsnArgTyrLeuAlaMetLysGluAsp   80
AGAGGAGTTGTGTCTATCAAAGGAGTGAGCGCTAATCGTTACCTGGCTATGAAGGAAGAT  240

GlyArgLeuLeuAlaSerLysSerValThrAspGluSerPhePhePheGluArgLeuGlu  100
GGAAGATTACTAGCTTCTAAGTCTGTTACGGATGAGTCTTTCTTCTTCGAACGATTGGAA  300

SerAsnAsnTyrAsnThrTyrArgSerArgLysTyrThrSerTrpTyrValAlaLeuLys  120
TCTAATAACTACAATACTTACCGGTCAAGGAAATACACCAGTTGGTATGTGGCACTGAAA  360

ArgThrGlyGlnTyrLysLeuGlySerLysThrGlyProGlyGlnLysAlaIleLeuPhe  140
CGAACTGGGCAGTATAAACTTGGATCCAAAACAGGACCTGGGCAGAAAGCTATACTTTTT  420

LeuProMetSerAlaLysSertrm                                      147
CTTCCAATGTCTGCTAAGAGCTGA                                      444.
```

Figure 28

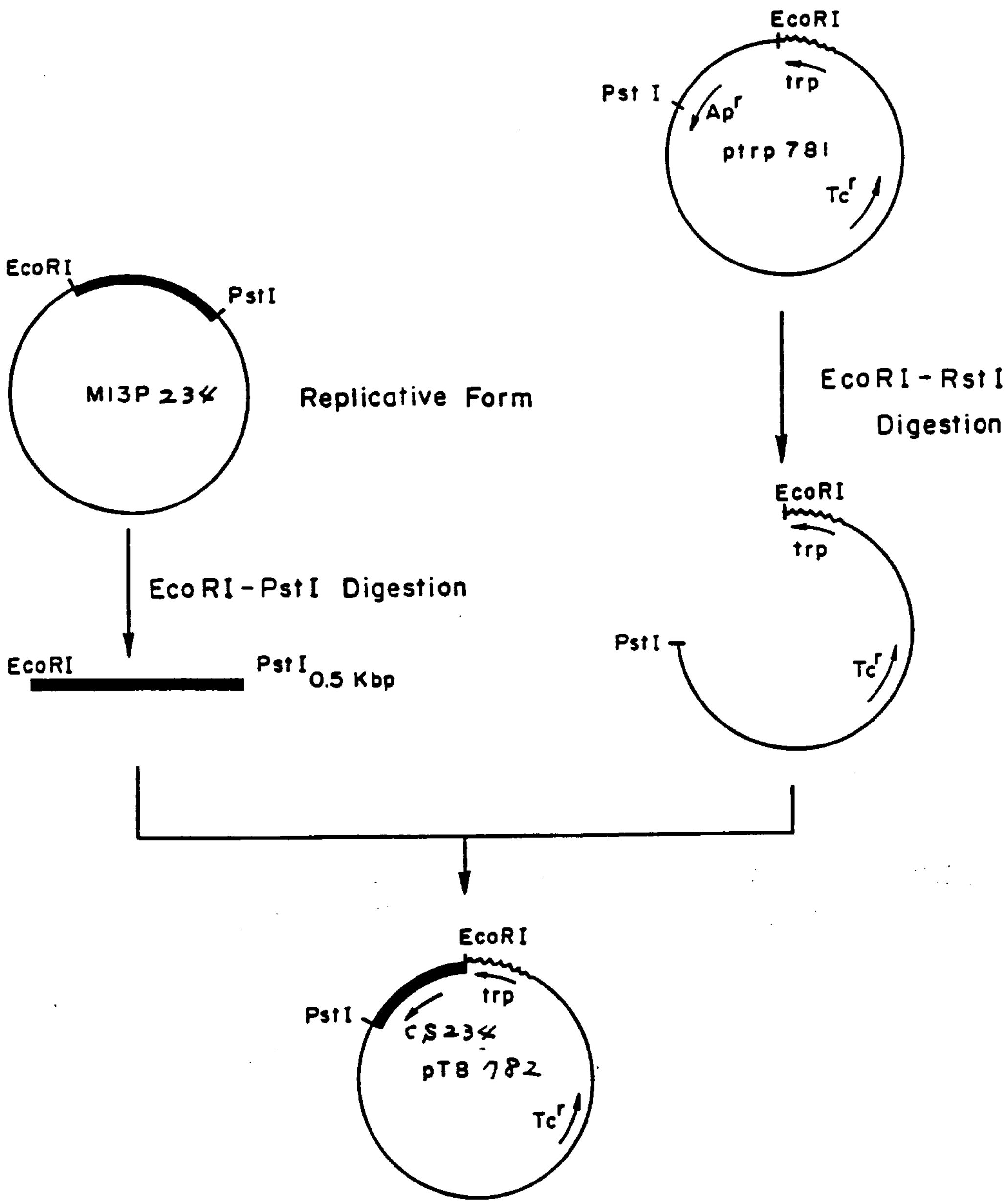
EcoRI
trp
Pst I
Ap$^r$
ptrp 781
Tc$^r$
EcoRI
PstI
M13P 234
Replicative Form
EcoRI-RstI
Digestion
EcoRI
trp
PstI
Tc$^r$
EcoRI-PstI Digestion
EcoRI
PstI
0.5 Kbp
EcoRI
trp
PstI
CS234
pTB 782
Tc$^r$

Figure 29

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrSerLysAsnGlyGlyPhePheLeuArgIleHisProAspGlyArg     40
CCCAAGCGGCTCTACAGCAAGAACGGGGGCTTCTTCCTGCGCATCCACCCCGACGGCCGA    120

ValAspGlyValArgGluLysSerAspProHisIleLysLeuGlnLeuGlnAlaGluGlu     60
GTTGACGGGGTCCGGGAGAAGAGCGACCCTCACATCAAGCTACAACTTCAAGCAGAAGAG    180

ArgGlyValValSerIleLysGlyValSerAlaAsnArgTyrLeuAlaMetLysGluAsp     80
AGAGGAGTTGTGTCTATCAAAGGAGTGAGCGCTAATCGTTACCTGGCTATGAAGGAAGAT    240

GlyArgLeuLeuAlaSerLysCysValThrAspGluSerPhePhePheGluArgLeuGlu    100
GGAAGATTACTGGCTTCTAAATGTGTTACGGATGAGTCTTTCTTCTTCGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444.
```

Figure 30

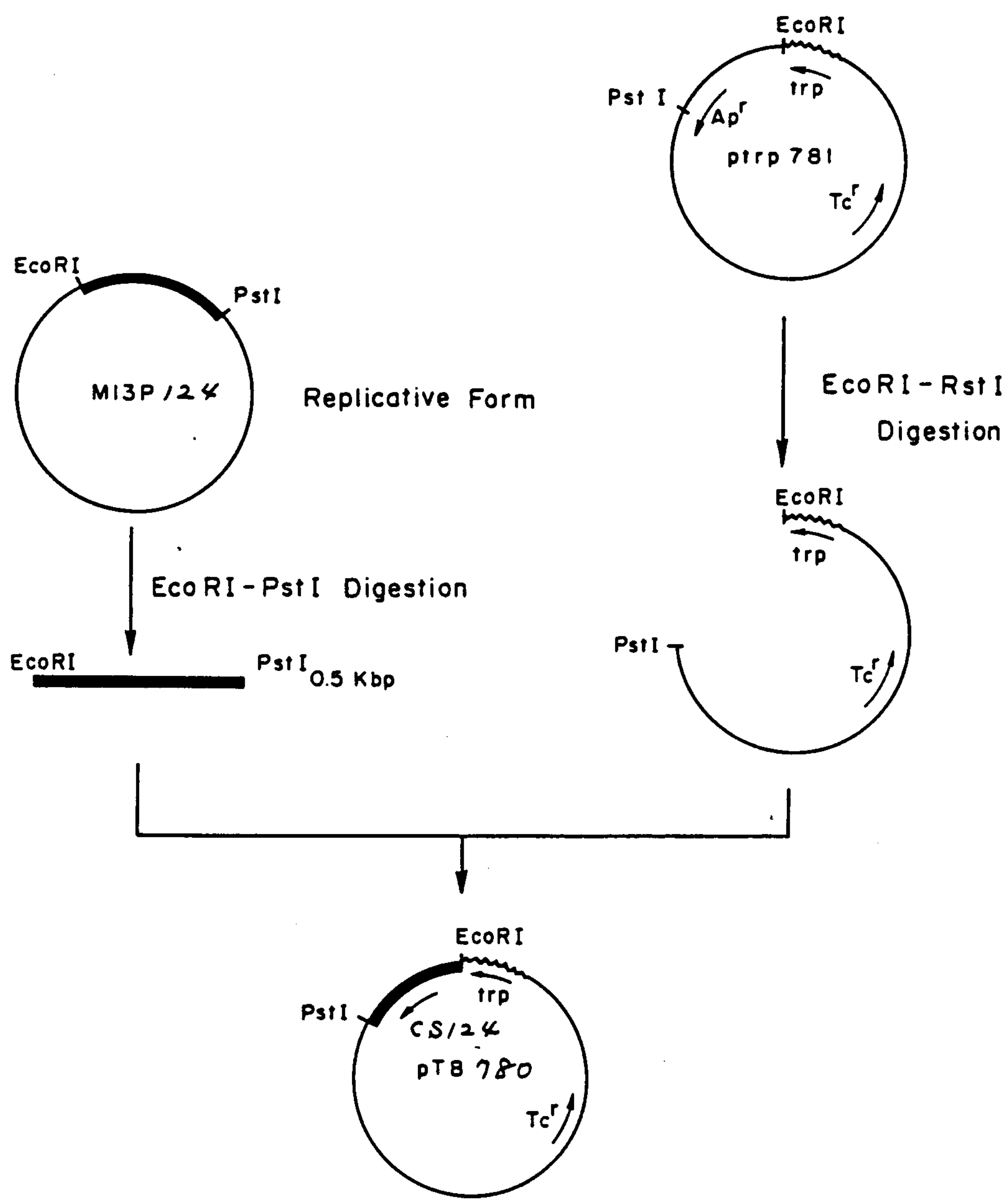
EcoRI
trp
Pst I
Apr
ptrp 781
Tcr
EcoRI
PstI
M13P/24
Replicative Form
EcoRI-RstI
Digestion
EcoRI
trp
PstI
Tcr
EcoRI-PstI Digestion
EcoRI
PstI
0.5 Kbp
EcoRI
trp
PstI
CS/24
pTB 780
Tcr

Figure 31

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrSerLysAsnGlyGlyPhePheLeuArgIleHisProAspGlyArg    40
CCCAAGCGGCTCTACAGCAAGAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysSerValThrAspGluSerPhePhePheGluArgLeuGlu   100
GGAAGATTACTAGCTTCTAAGTCTGTTACGGATGAGTCTTTCTTCTTCGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444.
```

Figure 32

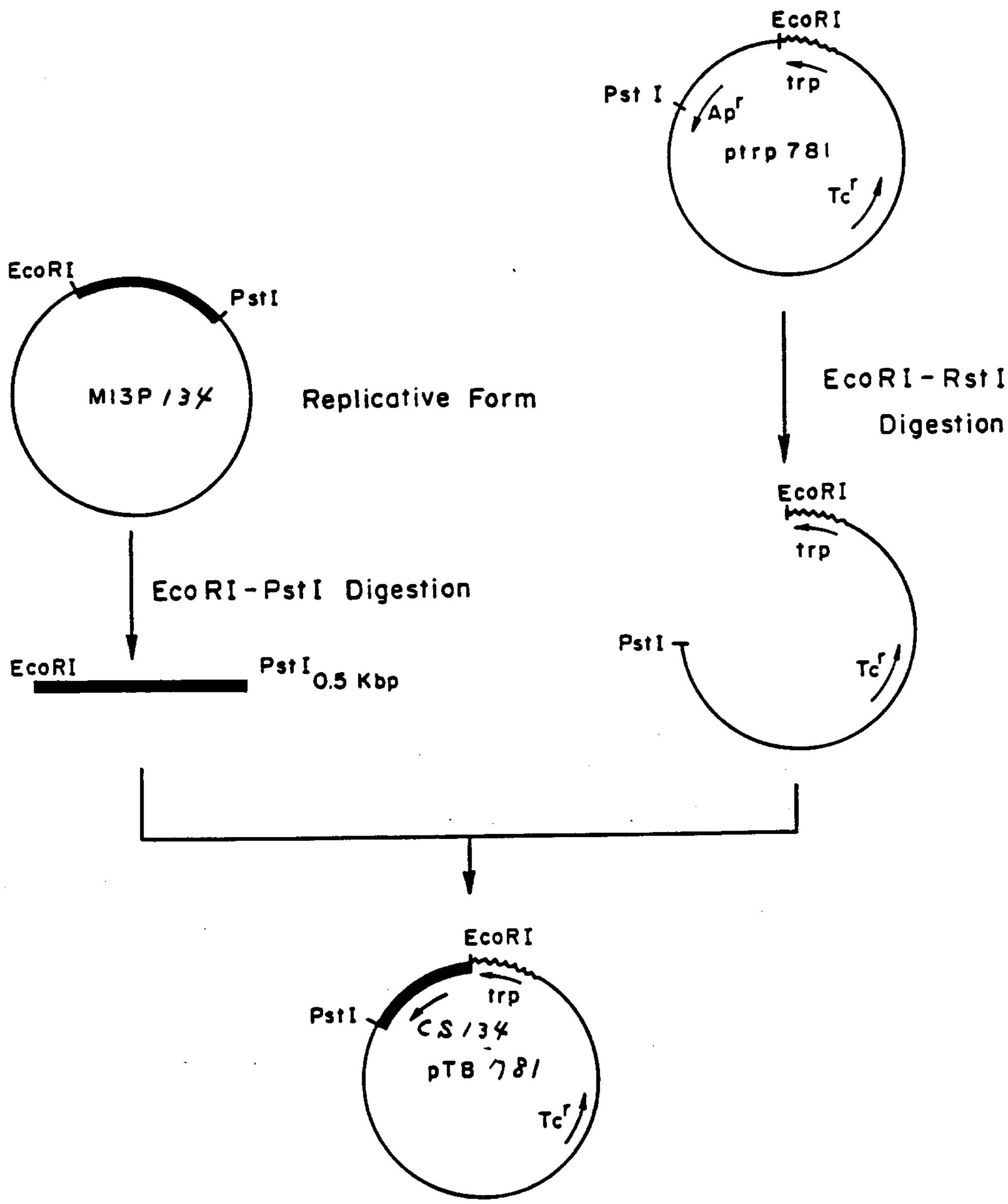
EcoRI
Pst I
trp
Ap^r
ptrp 781
Tc^r
EcoRI
PstI
M13P/34
Replicative Form
EcoRI-RstI
Digestion
EcoRI-PstI Digestion
EcoRI
PstI
0.5 Kbp
EcoRI
trp
PstI
Tc^r
EcoRI
trp
PstI
CS/34
pTB 781
Tc^r

Figure 33

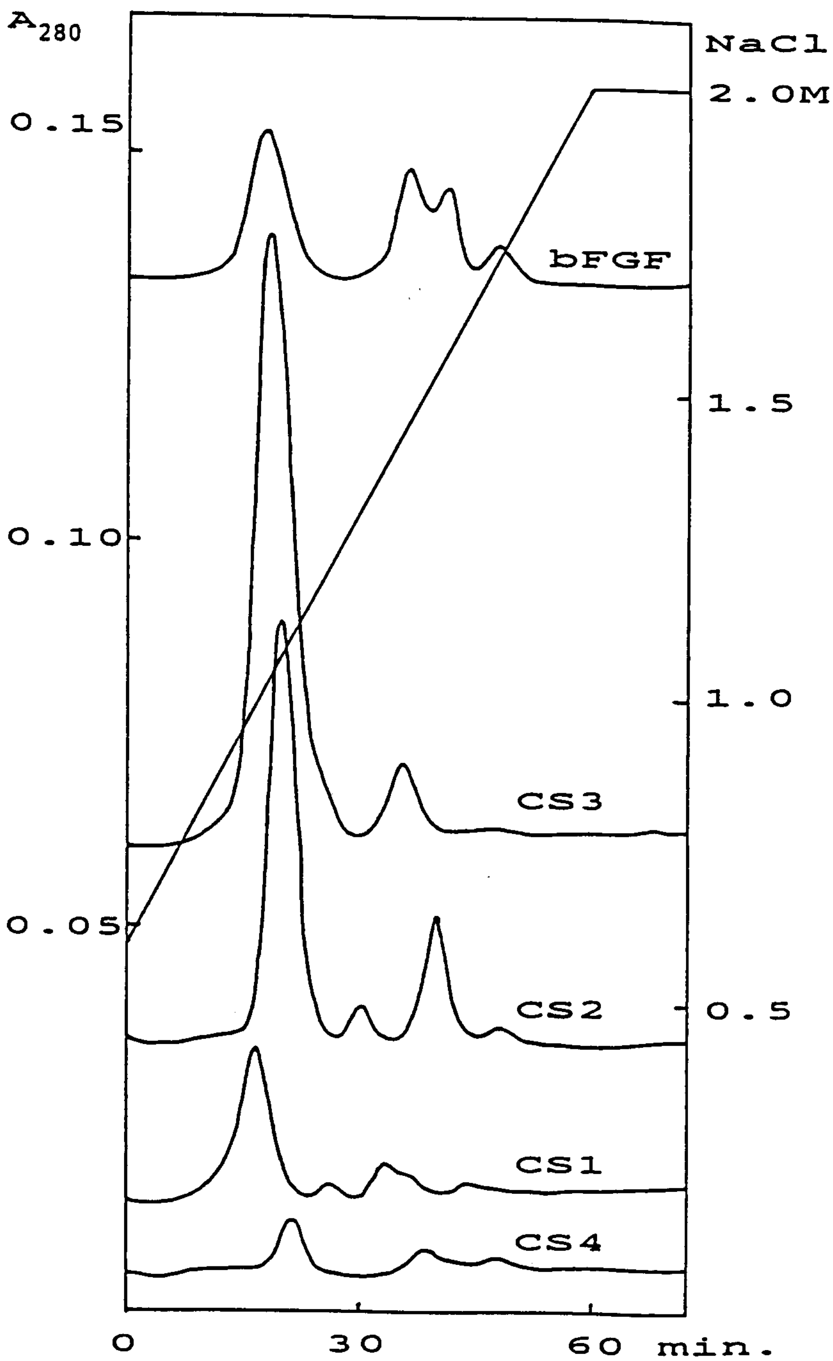
A280
0.15
0.10
0.05
NaCl
2.0M
1.5
1.0
0.5
bFGF
CS3
CS2
CS1
CS4
0
30
60
min.

Figure 34

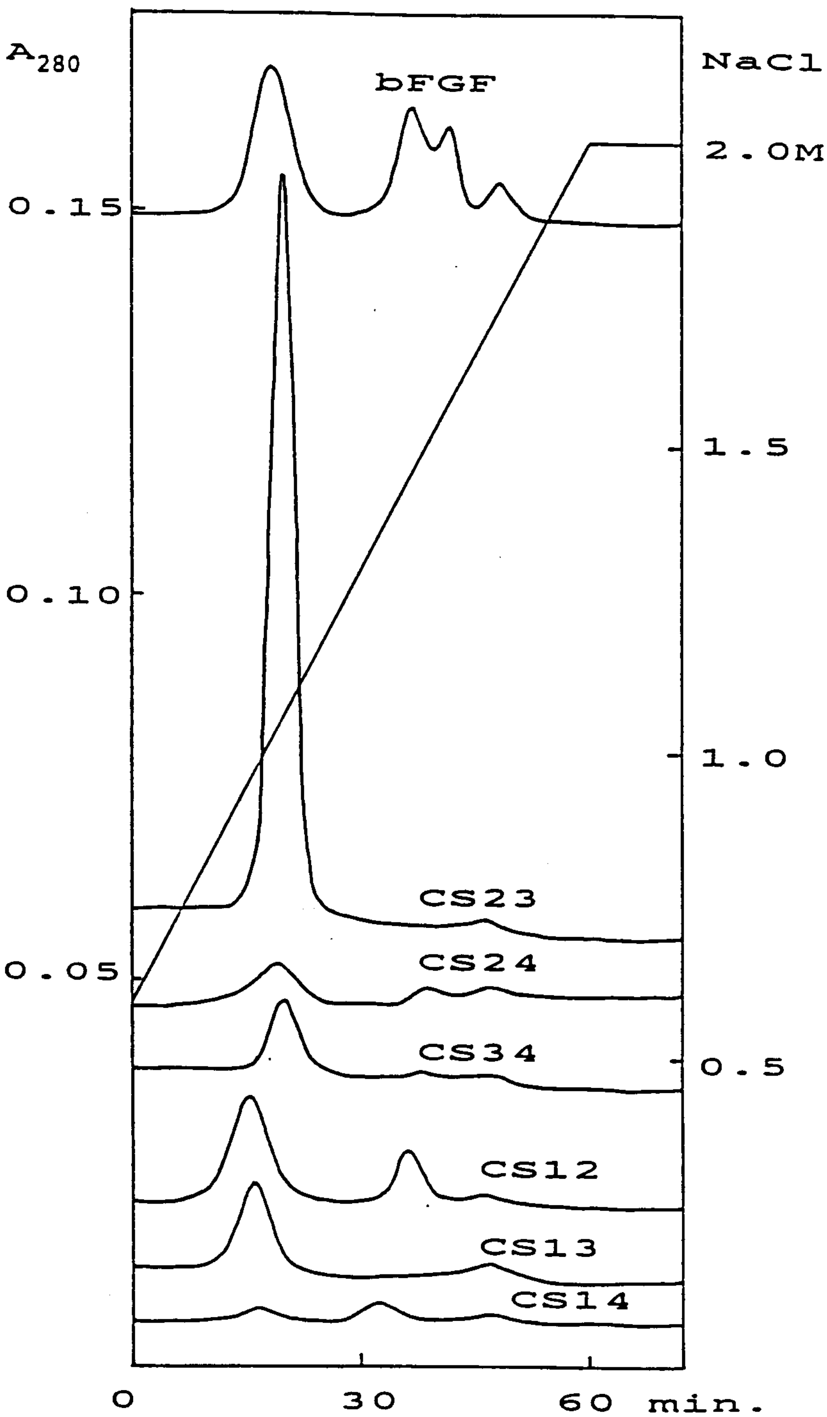
A280
NaCl
bFGF
2.0M
0.15
1.5
0.10
1.0
CS23
0.05
CS24
CS34
0.5
CS12
CS13
CS14
0
30
60
min.

Figure 35

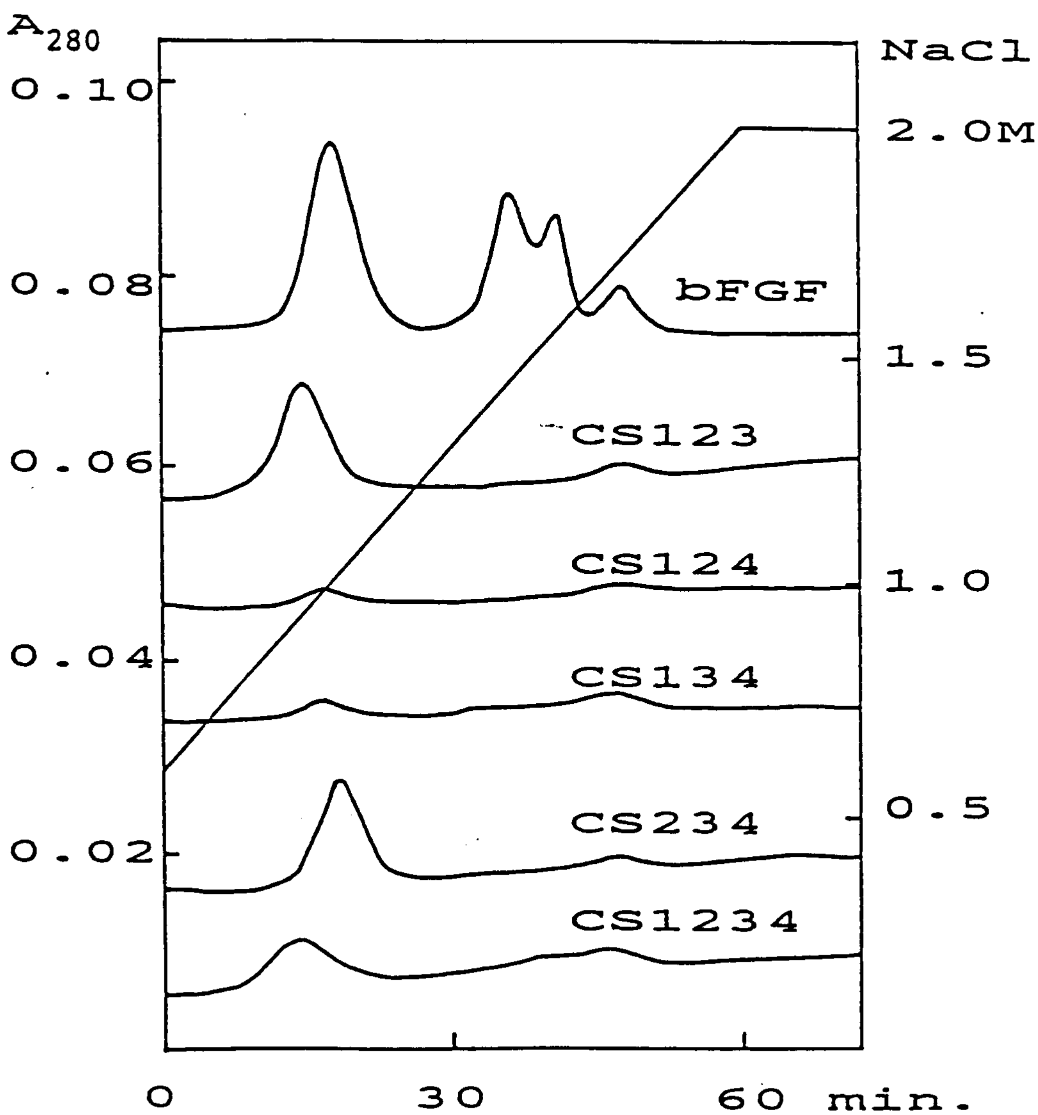
A280
0.10
0.08
0.06
0.04
0.02
NaCl
2.0M
1.5
1.0
0.5
bFGF
CS123
CS124
CS134
CS234
CS1234
0
30
60
min.

Figure 36

$+H_2O_2$

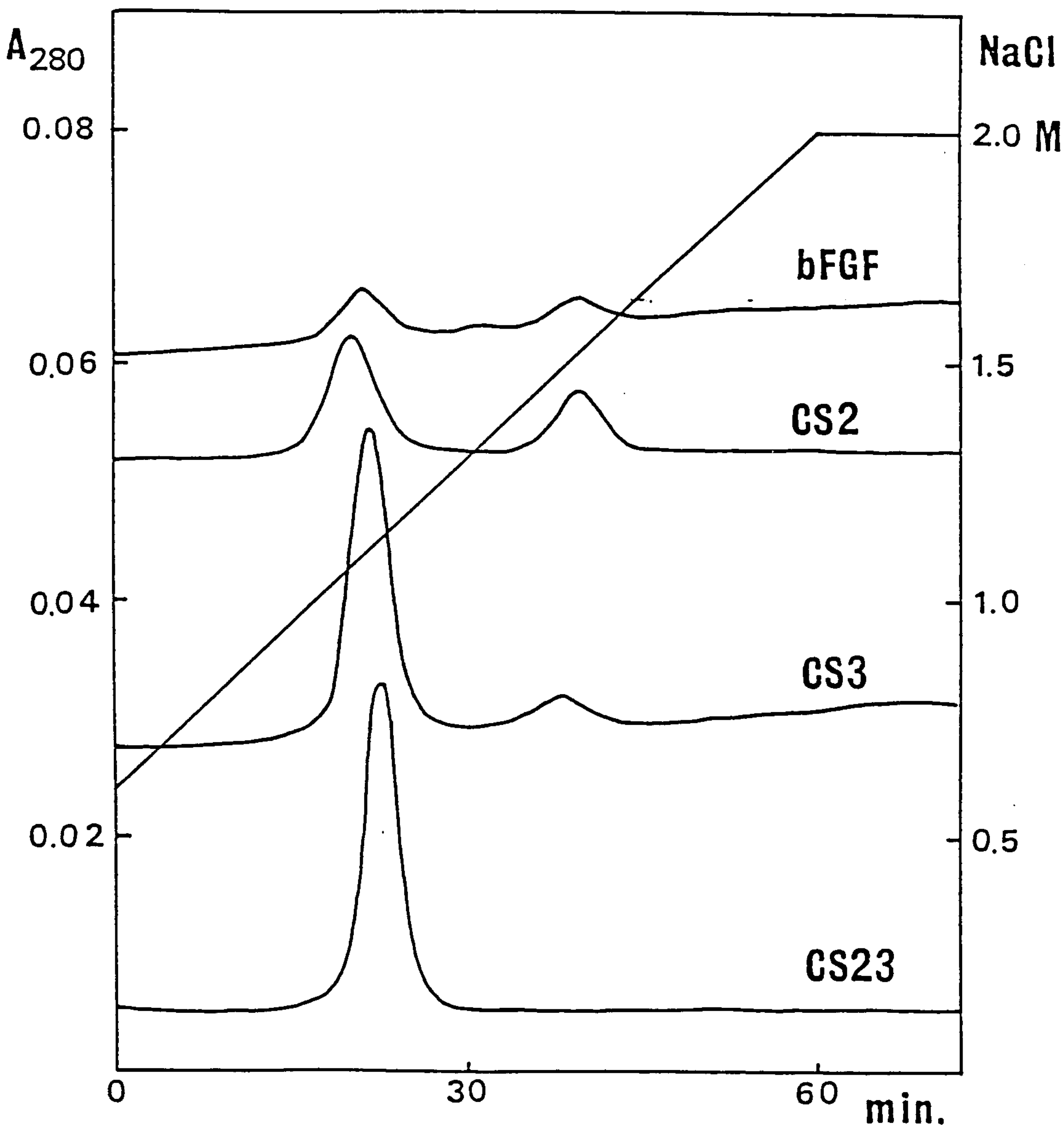

Figure 37

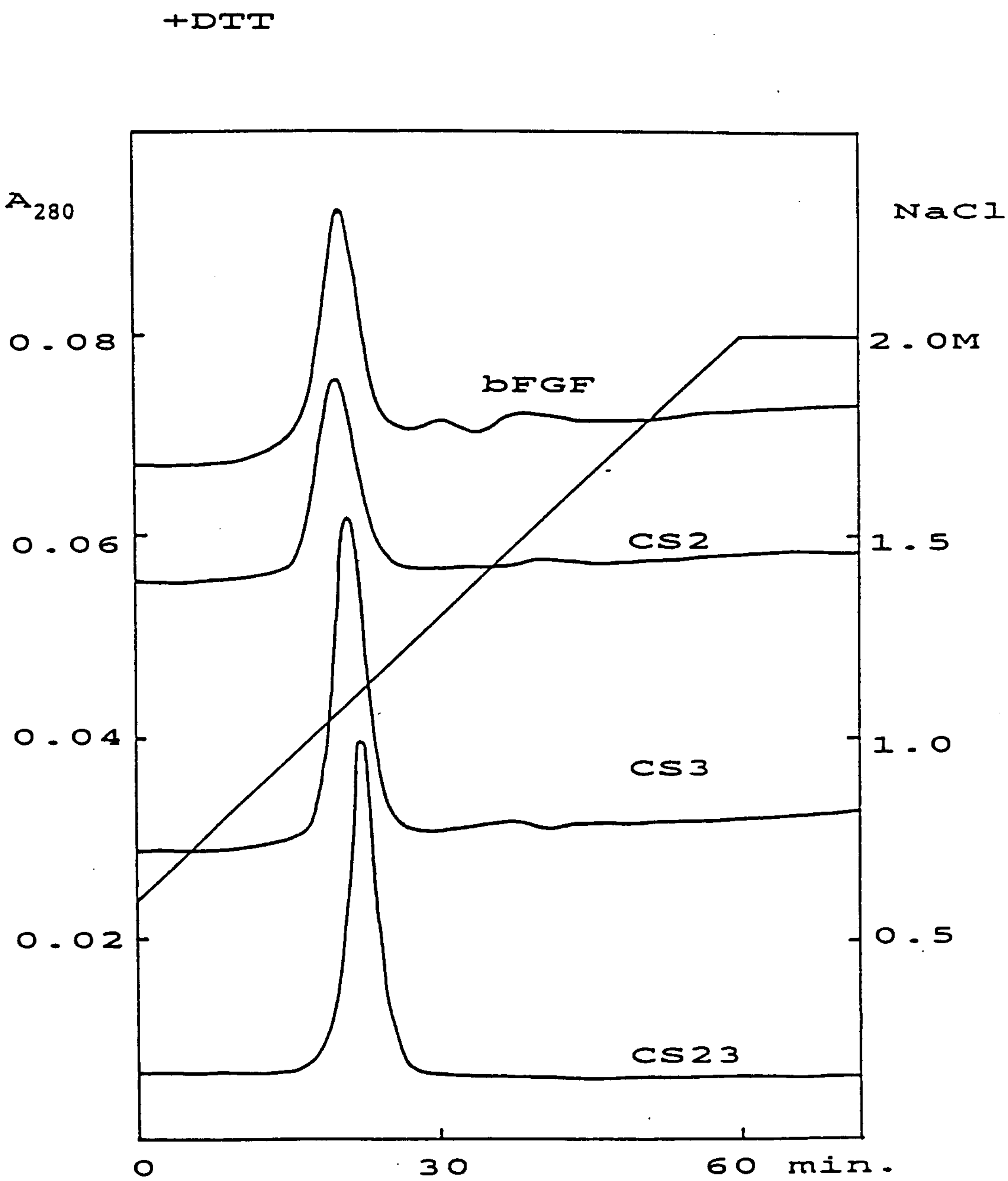
+DTT
A280
NaCl
0.08
0.06
0.04
0.02
2.0M
1.5
1.0
0.5
bFGF
CS2
CS3
CS23
0
30
60 min.

Figure 38

(1) MetThr
5'-CGCCCATGGTGCCATCCTC-3'
NcoI

(2) MeT
5'-CGGGCATGAATTCGCCGCT-3'
EcoRI

(3) trm
5'-TAACACCTTAAGAAGCCAG-3'
AflII

(4) Asn
5'-CCGGACTCCGTTAACTCGG-3'
HpaI

(5) Gln
5'-CTTCTCCTGGACTCCGTCAAC-3'
(HpaII)

(6) Arg
5'-CGGACTCCTCTAACTCGGC-3'
(HpaI)

(7) Leu Ser
5'-TCTTCTC CAG GGA TCC GTT -3'
BamHI

Figure 39

```
MetProAlaLeuProGluAspGlyThrMetGlyAlaPheProProGlyHisPheLysAsp    20
ATGCCAGCATTGCCCGAGGATGGCACCATGGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluCysPhePhePheGluArgLeuGlu   100
GGAAGATTACTGGCTTCTAAATGTGTTACGGATGAGTGTTTCTTTTTTGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 40

```
            MetProGlyHisPheLysAsp                        7
      GAATTCATGCCCGGCCACTTCAAGGAC                       27

ProLysArgLeuTyrCysLysAsnGlyGlyPhePheLeuArgIleHisProAspGlyArg    27
CCCAAGCGGCTGTACTGCAAAAACGGGGGCTTCTTCCTGCGCATCCACCCCGACGGCCGA    87

ValAspGlyValArgGluLysSerAspProHisIleLysLeuGlnLeuGlnAlaGluGlu    47
GTTGACGGGGTCCGGGAGAAGAGCGACCCTCACATCAAGCTACAACTTCAAGCAGAAGAG   147

ArgGlyValValSerIleLysGlyValCysAlaAsnArgTyrLeuAlaMetLysGluAsp    67
AGAGGAGTTGTGTCTATCAAAGGAGTGTGTGCTAACCGTTACCTGGCTATGAAGGAAGAT   207

GlyArgLeuLeuAlaSerLysCysValThrAspGluCysPhePhePheGluArgLeuGlu    87
GGAAGATTACTGGCTTCTAAATGTGTTACGGATGAGTGTTTCTTTTTTGAACGATTGGAA   267

SerAsnAsnTyrAsnThrTyrArgSerArgLysTyrThrSerTrpTyrValAlaLeuLys   107
TCTAATAACTACAATACTTACCGGTCAAGGAAATACACCAGTTGGTATGTGGCACTGAAA   327

ArgThrGlyGlnTyrLysLeuGlySerLysThrGlyProGlyGlnLysAlaIleLeuPhe   127
CGAACTGGGCAGTATAAACTTGGATCCAAAACAGGACCTGGGCAGAAAGCTATACTTTTT   387

LeuProMetSerAlaLysSertrm                                       134
CTTCCAATGTCTGCTAAGAGCTGA                                       411
```

Figure 41

```
MetProAlaLeuProGluAspGlyGlySerGlyAlaPheProProGlyHisPheLysAsp      20
ATGCCAGCATTGCCCGAGGATGGCGGCAGCGGCGCCTTCCCGCCCGGCCACTTCAAGGAC      60

ProLysArgLeuTyrCysLysAsnGlyGlyPhePheLeuArgIleHisProAspGlyArg      40
CCCAAGCGGCTGTACTGCAAAAACGGGGGCTTCTTCCTGCGCATCCACCCCGACGGCCGA     120

ValAspGlyValArgGluLysSerAspProHisIleLysLeuGlnLeuGlnAlaGluGlu      60
GTTGACGGGGTCCGGGAGAAGAGCGACCCTCACATCAAGCTACAACTTCAAGCAGAAGAG     180

ArgGlyValValSerIleLysGlyValCysAlaAsnArgTyrLeuAlaMetLysGluAsp      80
AGAGGAGTTGTGTCTATCAAAGGAGTGTGTGCTAACCGTTACCTGGCTATGAAGGAAGAT     240

GlyArgLeuLeuAlaSertrm                                              86
GGAAGATTACTGGCTTCTTAAGGTGTTACGGATGAGTGTTTCTTTTTGAACGATTGGAA     300

TCTAATAACTACAATACTTACCGGTCAAGGAAATACACCAGTTGGTATGTGGCACTGAAA     360

CGAACTGGGCAGTATAAACTTGGATCCAAAACAGGACCTGGGCAGAAAGCTATACTTTTT     420

CTTCCAATGTCTGCTAAGAGCTGA                                         444
```

Figure 42

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrCysLysAsnGlyGlyPhePheLeuArgIleHisProAspGlyArg     40
CCCAAGCGGCTGTACTGCAAAAACGGGGGCTTCTTCCTGCGCATCCACCCCGACGGCCGA    120

ValAsnGlyValArgGluLysSerAspProHisIleLysLeuGlnLeuGlnAlaGluGlu     60
GTTAACGGAGTCCGGGAGAAGAGCGACCCTCACATCAAGCTACAACTTCAAGCAGAAGAG    180

ArgGlyValValSerIleLysGlyValCysAlaAsnArgTyrLeuAlaMetLysGluAsp     80
AGAGGAGTTGTGTCTATCAAAGGAGTGTGTGCTAACCGTTACCTGGCTATGAAGGAAGAT    240

GlyArgLeuLeuAlaSerLysCysValThrAspGluCysPhePhePheGluArgLeuGlu    100
GGAAGATTACTGGCTTCTAAATGTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 43

```
MetProAlaLeuProGluAspGlyGlySerGlyAlaPheProProGlyHisPheLysAsp     20
ATGCCAGCATTGCCCGAGGATGGCGGCAGCGGCGCCTTCCCGCCCGGCCACTTCAAGGAC     60

ProLysArgLeuTyrCysLysAsnGlyGlyPhePheLeuArgIleHisProAspGlyArg     40
CCCAAGCGGCTGTACTGCAAAAACGGGGGCTTCTTCCTGCGCATCCACCCCGACGGCCGA    120

ValAspGlyValGlnGluLysSerAspProHisIleLysLeuGlnLeuGlnAlaGluGlu     60
GTTGACGGAGTCCAGGAGAAGAGCGACCCTCACATCAAGCTACAACTTCAAGCAGAAGAG    180

ArgGlyValValSerIleLysGlyValCysAlaAsnArgTyrLeuAlaMetLysGluAsp     80
AGAGGAGTTGTGTCTATCAAAGGAGTGTGTGCTAACCGTTACCTGGCTATGAAGGAAGAT    240

GlyArgLeuLeuAlaSerLysCysValThrAspGluCysPhePhePheGluArgLeuGlu    100
GGAAGATTACTGGCTTCTAAATGTGTTACGGATGAGTGTTTCTTTTTTGAACGATTGGAA    300

SerAsnAsnTyrAsnThrTyrArgSerArgLysTyrThrSerTrpTyrValAlaLeuLys    120
TCTAATAACTACAATACTTACCGGTCAAGGAAATACACCAGTTGGTATGTGGCACTGAAA    360

ArgThrGlyGlnTyrLysLeuGlySerLysThrGlyProGlyGlnLysAlaIleLeuPhe    140
CGAACTGGGCAGTATAAACTTGGATCCAAAACAGGACCTGGGCAGAAAGCTATACTTTTT    420

LeuProMetSerAlaLysSertrm                                        147
CTTCCAATGTCTGCTAAGAGCTGA                                        444
```

Figure 44

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValArgGlyValArgGluLysSerAspProHisIleLysLeuGlnLeuGlnAlaGluGlu    60
GTTAGAGGAGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluCysPhePhePheGluArgLeuGlu   100
GGAAGATTACTGGCTTCTAAATGTGTTACGGATGAGTGTTTCTTTTTTGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 45

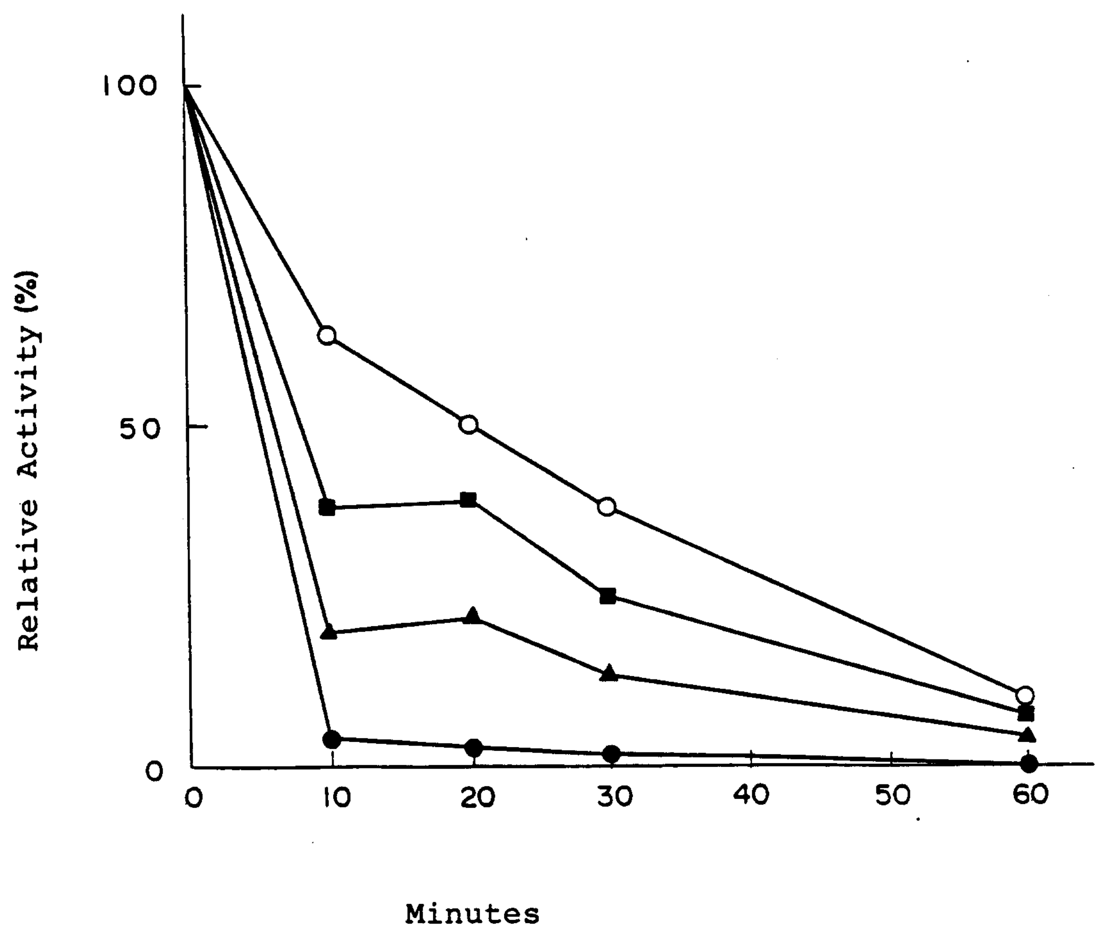

Figure 46

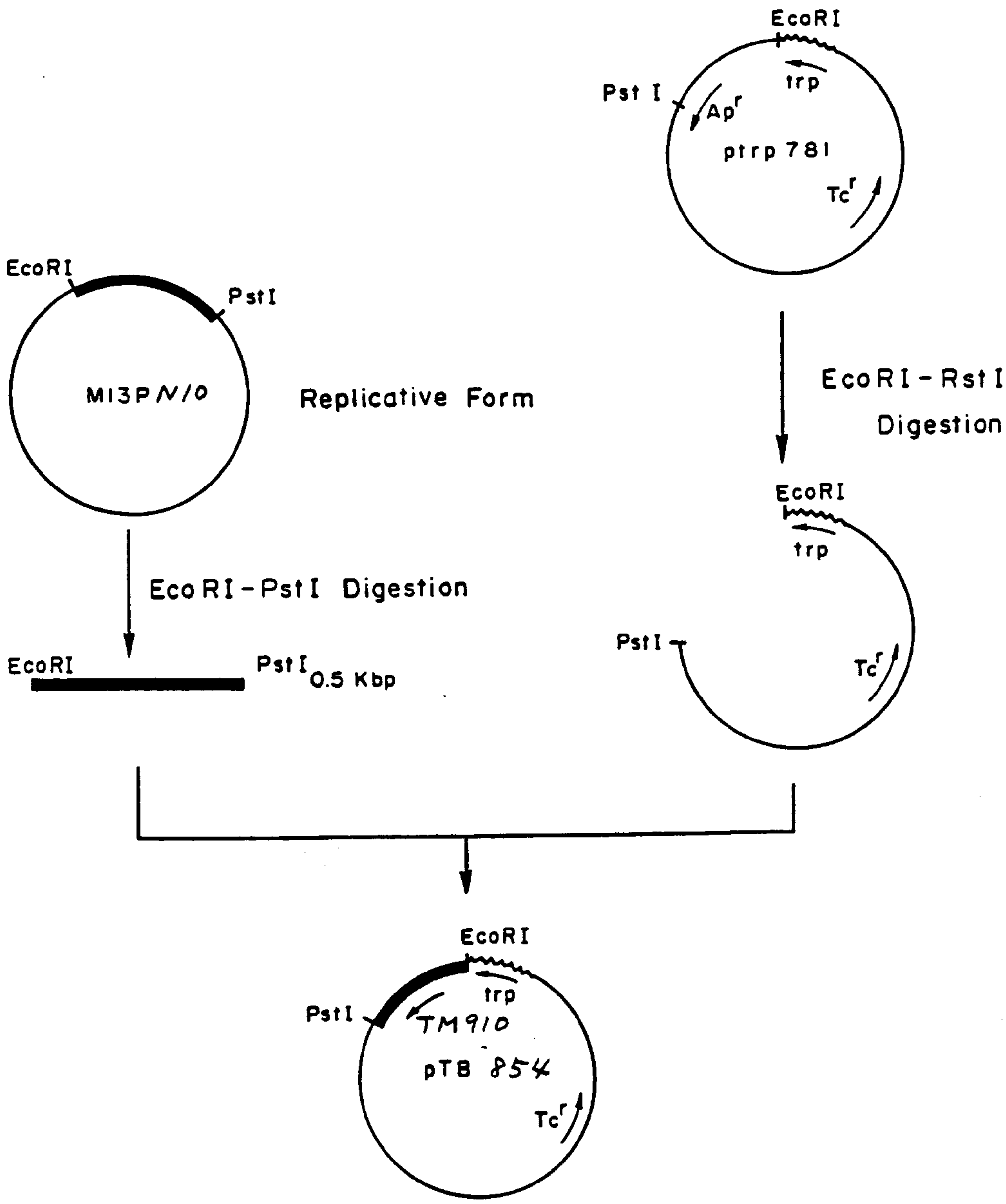
EcoRI
Pst I
trp
Ap$^r$
ptrp 781
Tc$^r$
EcoRI
PstI
M13PN10
Replicative Form
EcoRI-RstI
Digestion
EcoRI
trp
PstI
Tc$^r$
EcoRI-PstI Digestion
EcoRI
PstI 0.5 Kbp
EcoRI
trp
PstI
TM910
pTB 854
Tc$^r$

Figure 47

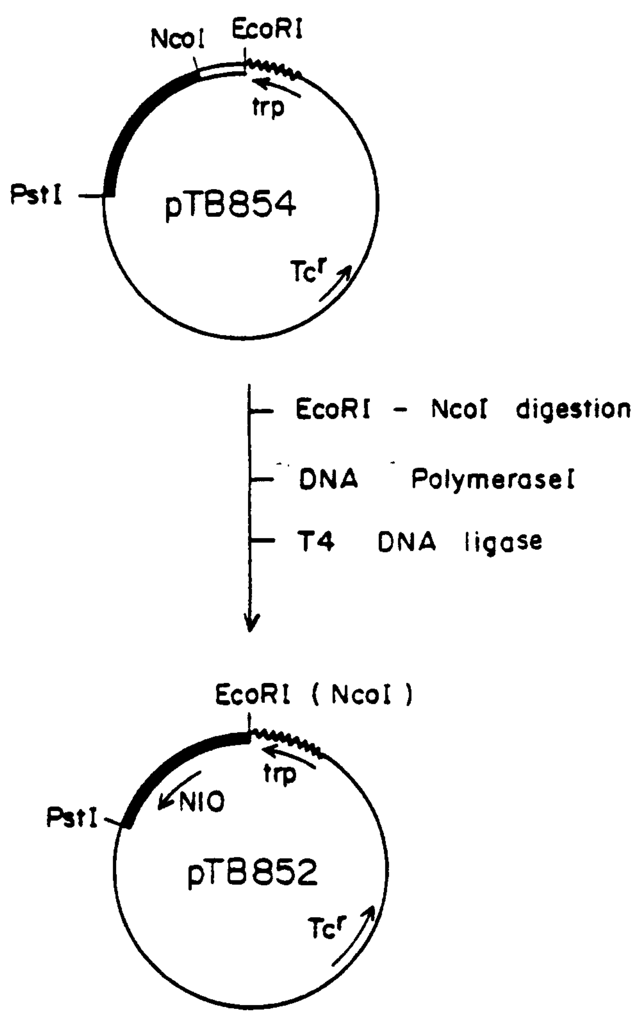
NcoI
EcoRI
trp
PstI
pTB854
Tc^r
EcoRI - NcoI digestion
DNA Polymerase I
T4 DNA ligase
EcoRI ( NcoI )
trp
NIO
PstI
pTB852
Tc^r

Figure 48

```
         MetGlyAlaPheProProGlyHisPheLysAsp
GAATTCATGGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

SerAsnAsnTyrAsnThrTyrArgSerArgLysTyrThrSerTrpTyrValAlaLeuLys
TCTAATAACTACAATACTTACCGGTCAAGGAAATACACCAGTTGGTATGTGGCACTGAAA

ArgThrGlyGlnTyrLysLeuGlySerLysThrGlyProGlyGlnLysAlaIleLeuPhe
CGAACTGGGCAGTATAAACTTGGATCCAAAACAGGACCTGGGCAGAAAGCTATACTTTTT

LeuProMetSerAlaLysSertrm
CTTCCAATGTCTGCTAAGAGCTGA
```

Figure 49

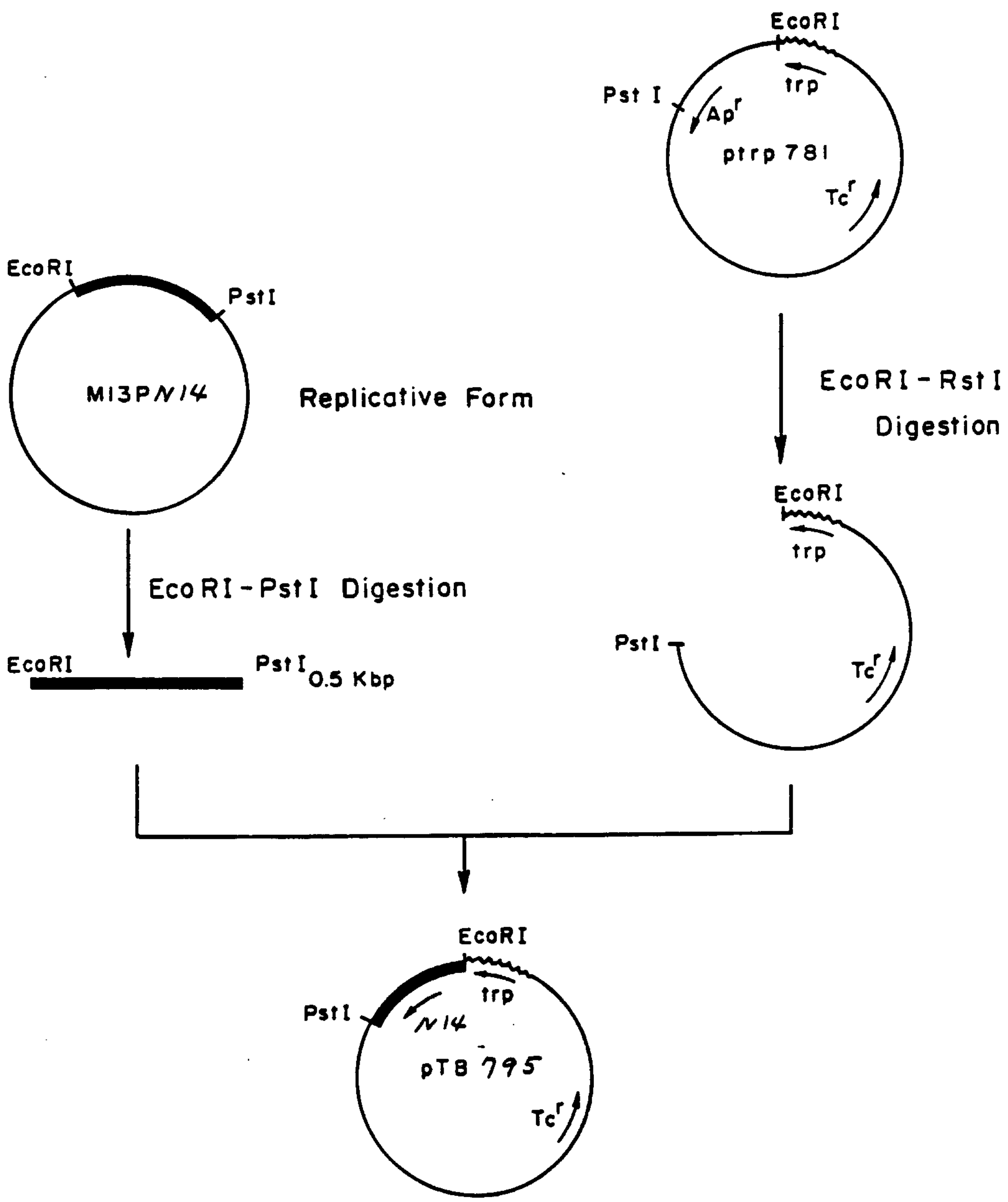
EcoRI
Pst I
trp
Apr
ptrp 781
Tcr
EcoRI
PstI
M13PN14
Replicative Form
EcoRI-RstI
Digestion
EcoRI
trp
EcoRI-PstI Digestion
EcoRI
PstI
0.5 Kbp
PstI
Tcr
EcoRI
PstI
trp
N14
pTB 795
Tcr

Figure 50

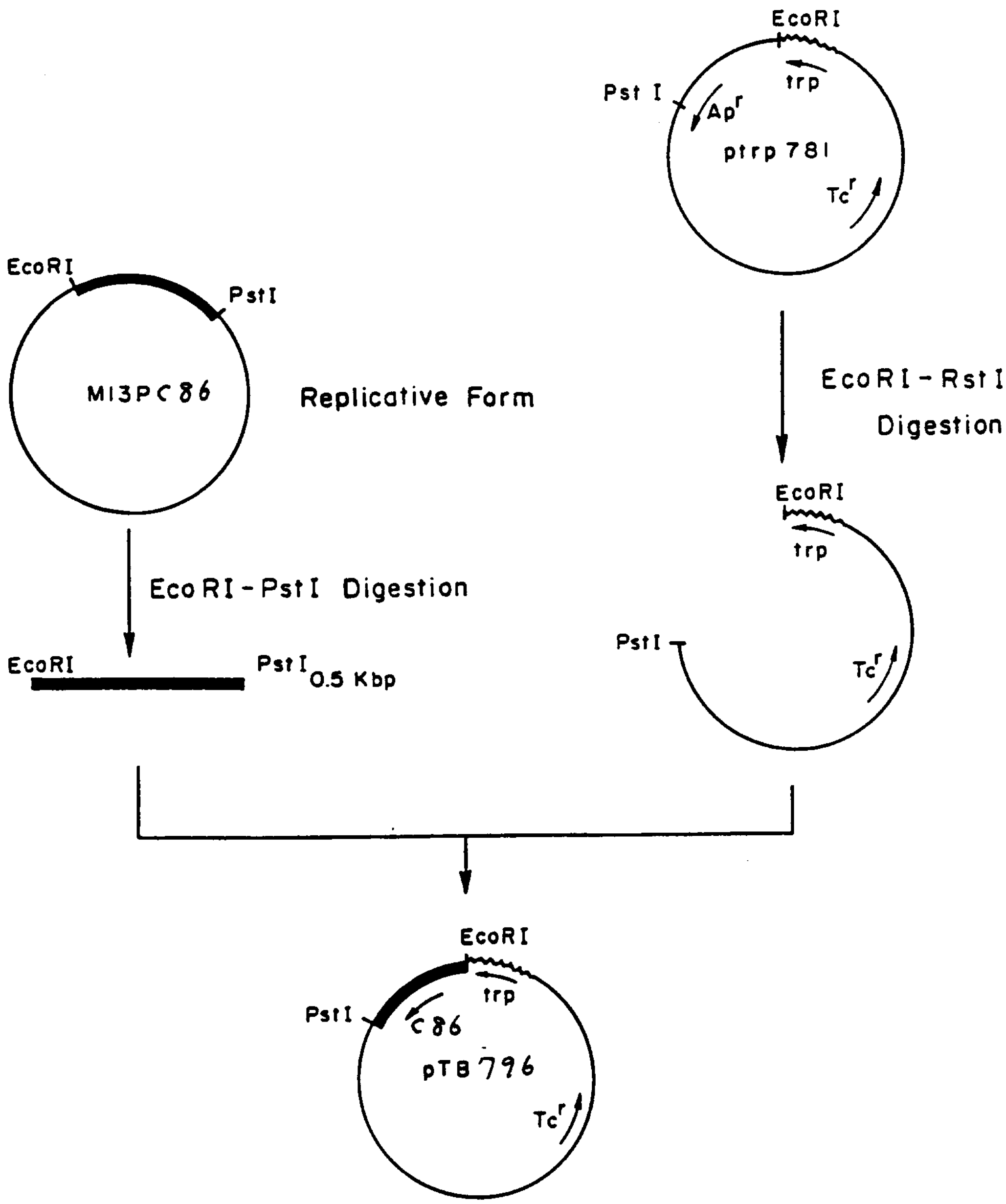
EcoRI
Pst I
trp
Ap^r
ptrp 781
Tc^r
EcoRI-RstI
Digestion
EcoRI
PstI
M13PC86
Replicative Form
EcoRI-PstI Digestion
EcoRI
PstI
0.5 Kbp
EcoRI
trp
PstI
Tc^r
EcoRI
trp
PstI
C86
pTB796
Tc^r

Figure 51

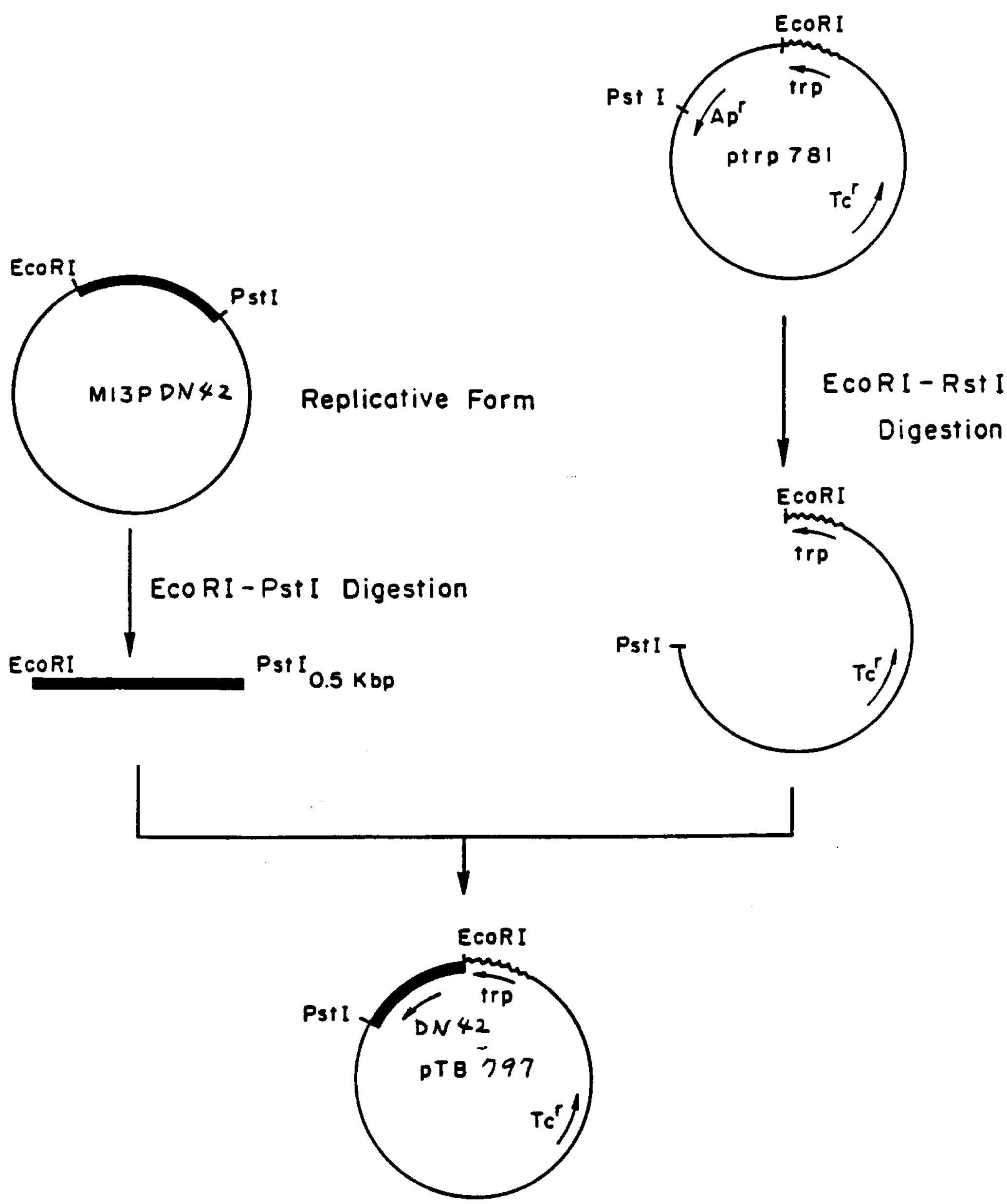
EcoRI
Pst I
trp
Apr
ptrp 781
Tcr
EcoRI
PstI
M13P DN42
Replicative Form
EcoRI-RstI
Digestion
EcoRI-PstI Digestion
EcoRI
PstI
0.5 Kbp
EcoRI
trp
PstI
Tcr
EcoRI
PstI
trp
DN42
pTB 797
Tcr

Figure 52

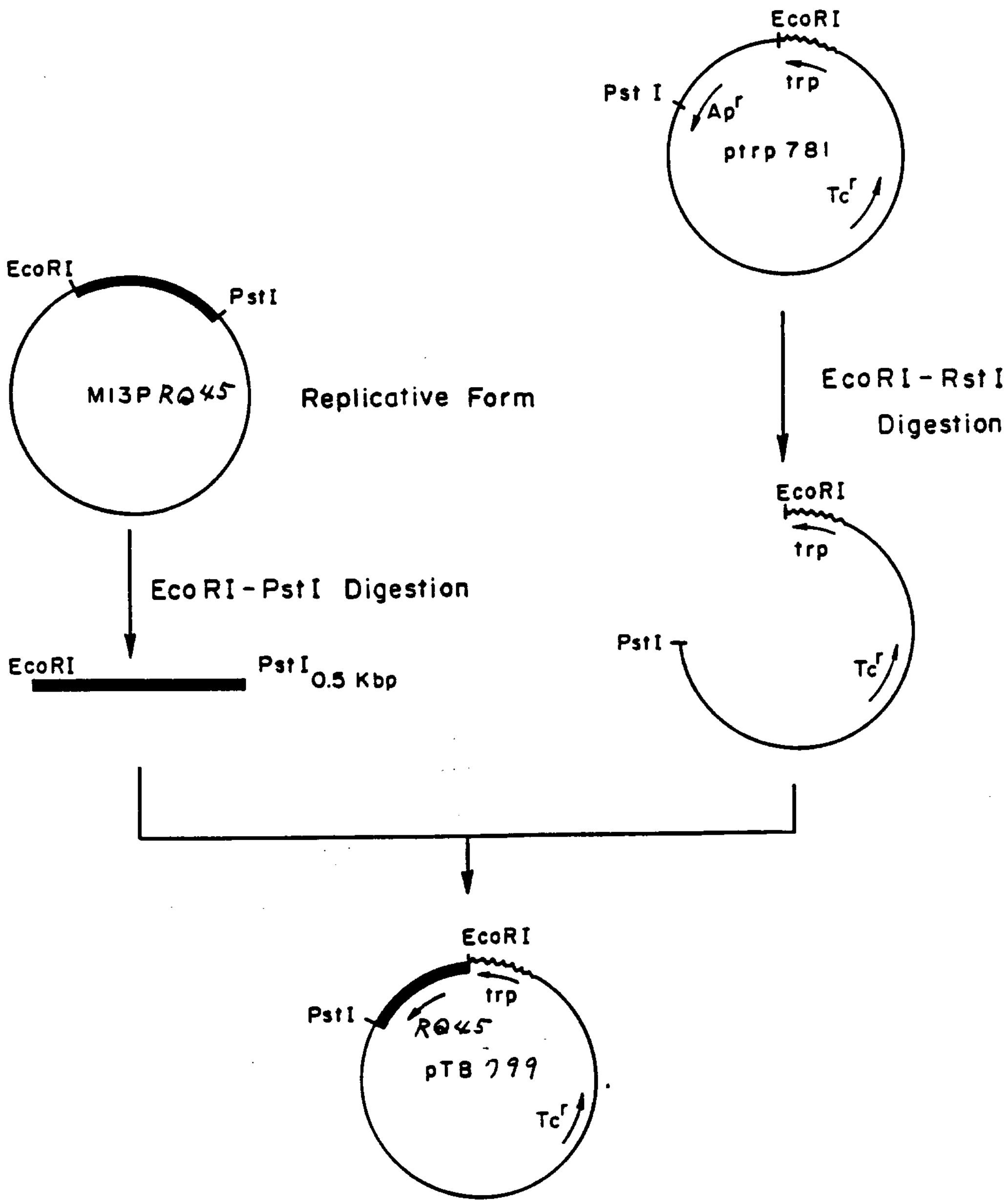
EcoRI
trp
Pst I
Ap^r
ptrp 781
Tc^r
EcoRI
PstI
M13P RQ45
Replicative Form
EcoRI - RstI
Digestion
EcoRI
trp
PstI
Tc^r
EcoRI - PstI Digestion
EcoRI
PstI
0.5 Kbp
EcoRI
trp
PstI
RQ45
pTB 799
Tc^r

Figure 53

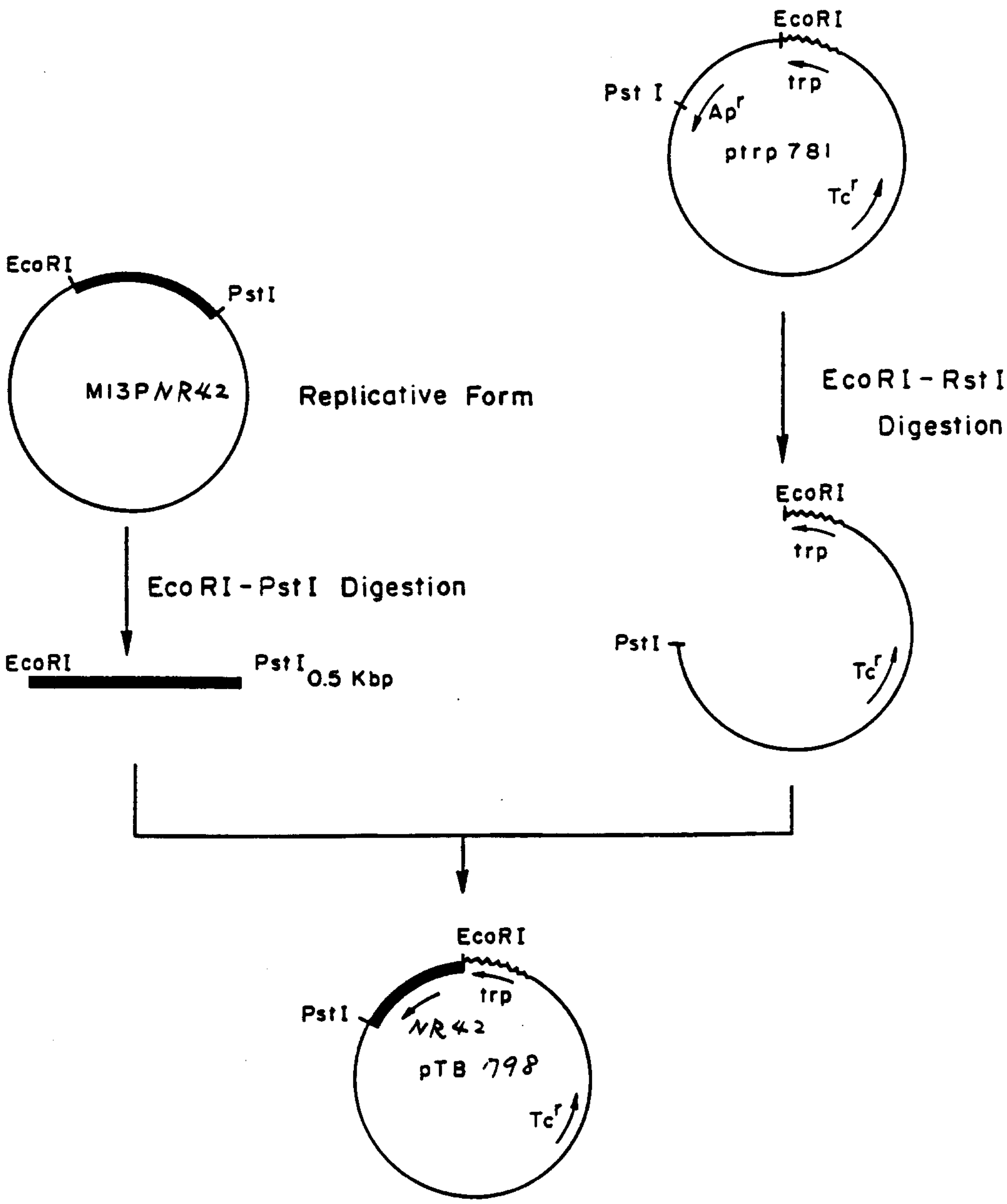
EcoRI
Pst I
Apr
trp
ptrp 781
Tcr
EcoRI
PstI
M13PNR42
Replicative Form
EcoRI-RstI
Digestion
EcoRI
trp
PstI
Tcr
EcoRI-PstI Digestion
EcoRI
PstI
0.5 Kbp
EcoRI
trp
PstI
NR42
pTB 798
Tcr

Figure 54

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAsnGlySerLeuGluLysSerAspProHisIleLysLeuGlnLeuGlnAlaGluGlu    60
GTTAACGGATCCCTGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluCysPhePhePheGluArgLeuGlu   100
GGAAGATTACTGGCTTCTAAATGTGTTACGGATGAGTGTTTCTTTTTTGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySerLysThrGlyProGlyGlnLysAlaIleLeuPhe   140
CGAACTGGGCAGTATAAACTTGGATCCAAAACAGGACCTGGGCAGAAAGCTATACTTTTT   420

LeuProMetSerAlaLysSertrm                                       147
CTTCCAATGTCTGCTAAGAGCTGA                                       444
```

Figure 55

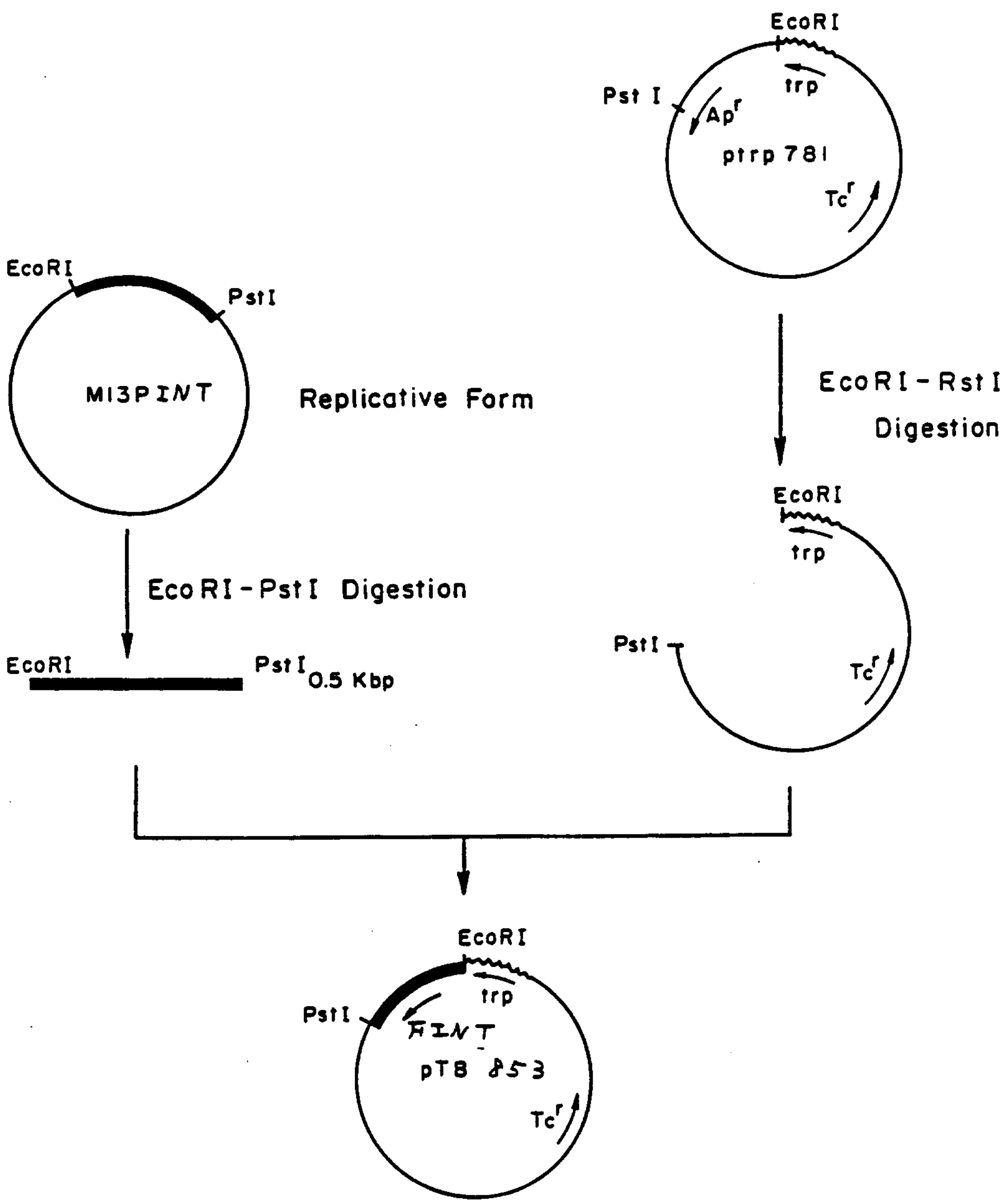
EcoRI
Pst I
trp
Apr
ptrp 781
Tcr
EcoRI
PstI
M13PINT
Replicative Form
EcoRI-RstI
Digestion
EcoRI
trp
PstI
Tcr
EcoRI-PstI Digestion
EcoRI
PstI 0.5 Kbp
EcoRI
trp
PstI
PINT
pTB 853
Tcr

Figure 56

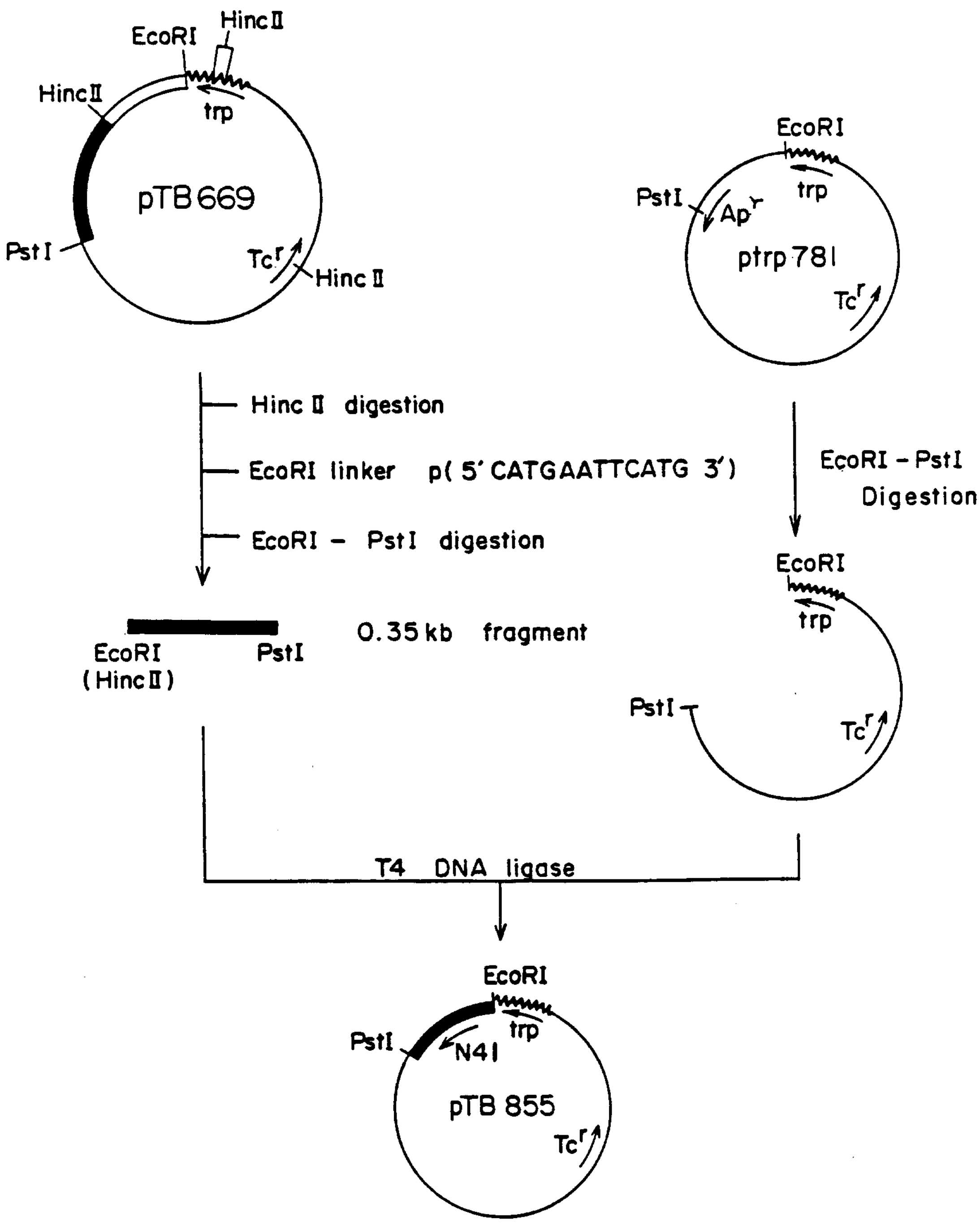
EcoRI
HincII
HincII
trp
pTB669
PstI
Tc$^r$
HincII
EcoRI
trp
PstI
Ap$^r$
ptrp781
Tc$^r$
HincII digestion
EcoRI linker p( 5' CATGAATTCATG 3')
EcoRI - PstI digestion
EcoRI - PstI
Digestion
EcoRI
trp
0.35 kb fragment
EcoRI
(HincII)
PstI
PstI
Tc$^r$
T4 DNA ligase
EcoRI
trp
PstI
N41
pTB 855
Tc$^r$

Figure 57

GAATTC

MetAspGlyValArgGluLysSerAspProHisIleLysLeuGlnLeuGlnAlaGluGlu
ATGGACGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

SerAsnAsnTyrAsnThrTyrArgSerArgLysTyrThrSerTrpTyrValAlaLeuLys
TCTAATAACTACAATACTTACCGGTCAAGGAAATACACCAGTTGGTATGTGGCACTGAAA

ArgThrGlyGlnTyrLysLeuGlySerLysThrGlyProGlyGlnLysAlaIleLeuPhe
CGAACTGGGCAGTATAAACTTGGATCCAAAACAGGACCTGGGCAGAAAGCTATACTTTTT

LeuProMetSerAlaLysSertrm
CTTCCAATGTCTGCTAAGAGCTGA

Figure 58

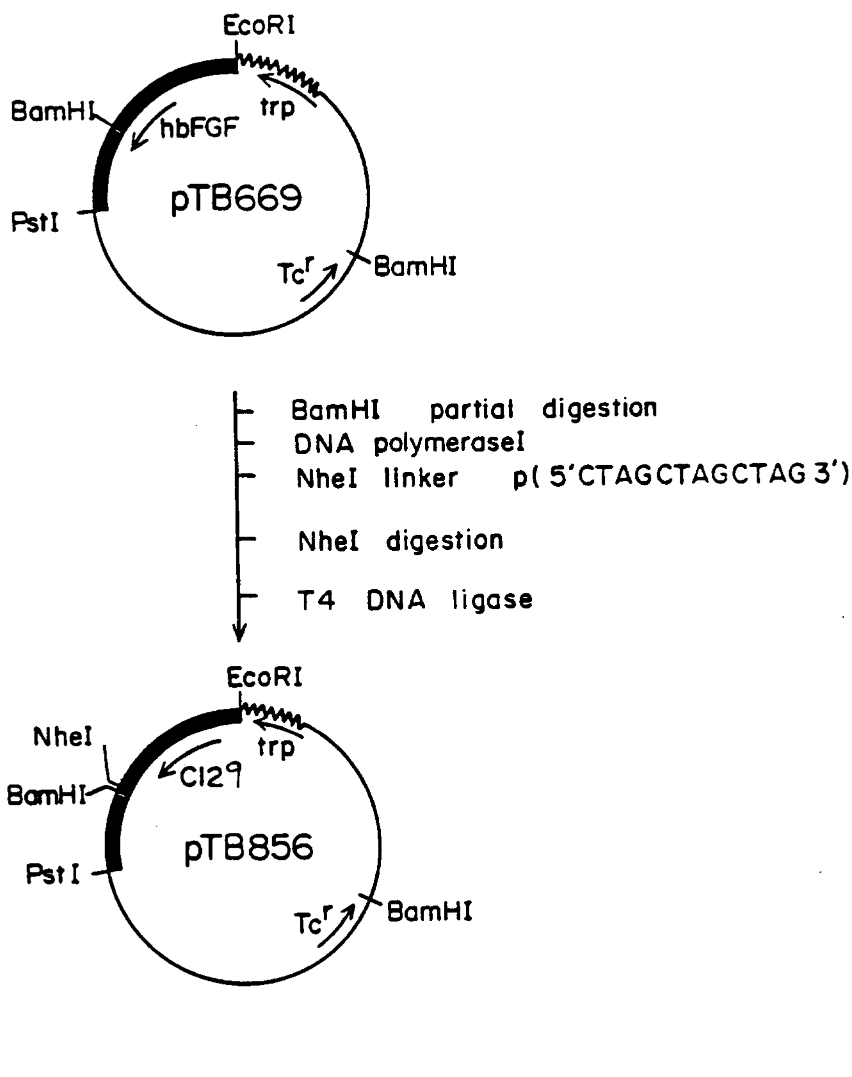
EcoRI
BamHI
hbFGF
trp
pTB669
PstI
Tc^r
BamHI
BamHI partial digestion
DNA polymeraseI
NheI linker p( 5'CTAGCTAGCTAG 3')
NheI digestion
T4 DNA ligase
EcoRI
NheI
trp
C129
BamHI
pTB856
PstI
Tc^r
BamHI

Figure 59

```
MetProAlaLeuProGluAspGlyGlySerGlyAlaPheProProGlyHisPheLysAsp    20
ATGCCAGCATTGCCCGAGGATGGCGGCAGCGGCGCCTTCCCGCCCGGCCACTTCAAGGAC    60

ProLysArgLeuTyrCysLysAsnGlyGlyPhePheLeuArgIleHisProAspGlyArg    40
CCCAAGCGGCTGTACTGCAAAAACGGGGGCTTCTTCCTGCGCATCCACCCCGACGGCCGA   120

ValAspGlyValArgGluLysSerAspProHisIleLysLeuGlnLeuGlnAlaGluGlu    60
GTTGACGGGGTCCGGGAGAAGAGCGACCCTCACATCAAGCTACAACTTCAAGCAGAAGAG   180

ArgGlyValValSerIleLysGlyValCysAlaAsnArgTyrLeuAlaMetLysGluAsp    80
AGAGGAGTTGTGTCTATCAAAGGAGTGTGTGCTAACCGTTACCTGGCTATGAAGGAAGAT   240

GlyArgLeuLeuAlaSerLysCysValThrAspGluCysPhePhePheGluArgLeuGlu   100
GGAAGATTACTGGCTTCTAAATGTGTTACGGATGAGTGTTTCTTTTTTGAACGATTGGAA   300

SerAsnAsnTyrAsnThrTyrArgSerArgLysTyrThrSerTrpTyrValAlaLeuLys   120
TCTAATAACTACAATACTTACCGGTCAAGGAAATACACCAGTTGGTATGTGGCACTGAAA   360

ArgThrGlyGlnTyrLysLeuGlySer trm                                129
CGAACTGGGCAGTATAAACTTGGATCC TAG                                390
```

Figure 60

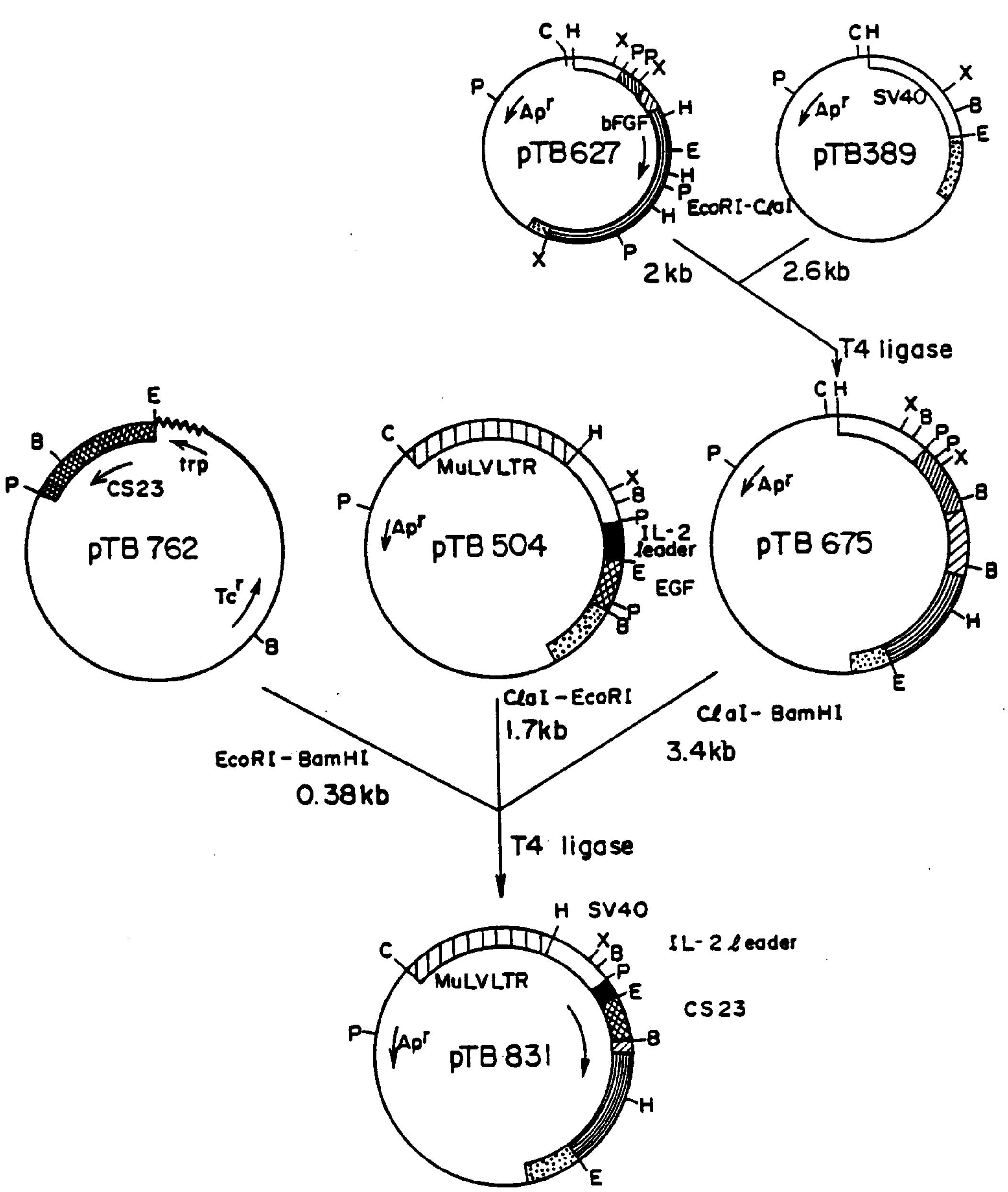
pTB627
bFGF
Apr
EcoRI-ClaI
2 kb
pTB389
SV40
2.6kb
T4 ligase
pTB 762
CS23
trp
Tcr
pTB 504
MuLVLTR
IL-2 leader
EGF
pTB 675
ClaI - EcoRI
1.7kb
ClaI - BamHI
3.4kb
EcoRI - BamHI
0.38kb
T4 ligase
pTB 831
SV40
IL-2 leader
CS23